# EUROPEAN PATENT SPECIFICATION

(11) **EP 2 753 613 B1**
(45) Date of publication and mention of the grant of the patent: **04.03.2020**
(21) Application number: 12829376.8
(22) Date of filing: 10.09.2012
(51) Int. Cl.: C07D 311/86, C07D 311/78, C07D 405/10, C07D 405/14, C07D 413/10, C07D 417/10, A61K 31/352, A61K 31/4025, A61K 31/404, A61K 31/4155, A61K 31/4178, A61K 31/4196, A61K 31/4545, A61K 31/5377, A61K 31/541, A61K 38/05, C07K 5/06, A61P 31/04

(54) **DERIVATIVES OF XANTHONE COMPOUNDS**
DERIVATE AUS XANTHONVERBINDUNGEN
DÉRIVÉS DE COMPOSÉS DE XANTHONE

(30) Priority: 08.09.2011 SG 201106479
(43) Date of publication of application: 16.07.2014
(73) Proprietor: Agency for Science, Technology and Research, Singapore 138632 (SG); Singapore Health Services Pte Ltd, Singapore 168753 (SG)
(72) Inventor: ZOU, Hanxun, Singapore 168751 (SG); RAJANMANI, Lakshminiarayan, Singapore 168751 (SG); ZHOU, Lei, Singapore 168751 (SG); TANG, Chang Chui Charles, Singapore 169608 (SG); KOH, Jun Jie, Singapore 168751 (SG); TAN, Tiang Hwee Donald, Singapore 168751 (SG); VERMA, Chandra, Singapore 138671 (SG); BEUERMAN, Roger W., Singapore 168751 (SG); LIU, Shouping, Singapore 168751 (SG); PADMANABHAN, Saraswathi, Singapore 168751 (SG)
(74) Representative: Brann AB
(86) International application number: PCT/SG2012/000328
(87) International publication number: WO 2013/036207

(56) References cited:
- WO-A1-97/34482
- WO-A1-2009/137014
- MAHABUSARAKAM WILAWAN ET AL: "Prenylated xanthones as potential antiplasmodial substances", PLANTA MEDICA, THIEME VERLAG, DE, vol. 72, no. 10, 1 August 2006 (2006-08-01), pages 912-916, XP002464730, ISSN: 0032-0943, DOI: 10.1055/S-2006-947190
- ZHE XIONG LU ET AL: "Inhibition of eukaryote protein kinases and of a cyclic nucleotide-binding phosphatase by prenylated xanthones", CHEMICO-BIOLOGICAL INTERACTIONS., vol. 114, no. 1-2, 1 July 1998 (1998-07-01), pages 121-140, XP055158289, IR ISSN: 0009-2797, DOI: 10.1016/S0009-2797(98)00049-0
- NGOUPAYO J ET AL: "Antimicrobial and immunomodulatory properties of prenylated xanthones from twigs of Garcinia staudtii", BIOORGANIC & MEDICINAL CHEMISTRY, PERGAMON, GB, vol. 17, no. 15, 1 August 2009 (2009-08-01), pages 5688-5695, XP026336581, ISSN: 0968-0896, DOI: 10.1016/J.BMC.2009.06.009 [retrieved on 2009-06-13]
- SAKAGAMI Y ET AL: "Antibacterial activity of alpha-mangostin against vancomycin resistant Enterococci (VRE) and synergism with antibiotics", PHYTOMEDICINE, GUSTAV FISCHER VERLAG, STUTTGART, DE, vol. 12, no. 3, 22 March 2005 (2005-03-22) , pages 203-208, XP027805541, ISSN: 0944-7113 [retrieved on 2005-03-22]
- SUKSAMRARN S ET AL: "Antimycobacterial activity of prenylated xanthoones from the fruits of Garcinia mangostana", CHEMICAL AND PHARMACEUTICAL BULLETIN, PHARMACEUTICAL SOCIETY OF JAPAN, JP, vol. 51, no. 7, 1 July 2003 (2003-07-01), pages 857-859, XP002345097, ISSN: 0009-2363, DOI: 10.1248/CPB.51.857
- IINUMA M ET AL: "ANTIBACTERIAL ACTIVITY OF XANTHONES FROM GUTTIFERAEOUS PLANTS AGAINST METHICILLIN-RESISTANT STAPHYLOCOCCUS AUREUS", JOURNAL OF PHARMACY AND PHARMACOLOGY, JOHN WILEY & SONS LTD, LONDON; GB, vol. 48, no. 8, 1 January 1996 (1996-01-01), pages 861-865, XP008052626, ISSN: 0022-3573
- CHOMNAWANG M T ET AL: "Antibacterial Activity of Thai Medicinal Plants against Methicillin-resistant Staphylococcus aureus", FITOTERAPIA, IDB HOLDING, MILAN, IT, vol. 80, no. 2, 1 March 2009 (2009-03-01), pages 102-104, XP025958654, ISSN: 0367-326X, DOI: 10.1016/J.FITOTE.2008.10.007 [retrieved on 2008-11-05]
- DATABASE CAPLUS [Online] XP003033816 Retrieved from STN Database accession no. 1996:694176 & JP H08 217 672 A (TERUMO CORP) 27 August 1996
- DATABASE CAPLUS [Online] XP003033817 Retrieved from STN Database accession no. 1997:385270 & JP H09 110 688 A (MEIJI MILK) 28 April 1997
- DATABASE CAPLUS [Online] XP003033818 Retrieved from STN Database accession no. 1996:707533 & JP H08 231 396 A (TERUMO CORP) 10 September 1996
- DATABASE CAPLUS [Online] XP003033819 Retrieved from STN Database accession no. 2005:253646 & JP 2005 075791 A (SAKAGAMI) 24 March 2005
- DATABASE CAPLUS [Online] XP003033820 Retrieved from STN Database accession no. 2010:1476452 & CN 101 889 998 A (JINAN UNIV) 24 November 2010
- DATABASE CAPLUS [Online] XP003033821 Retrieved from STN Database accession no. 2009:487770 & JP 2009 084169 A (NIMURA) 23 April 2009

## Description

### Technical Field

The present disclosure generally relates to derivatives of xanthone compounds. The present disclosure also relates to use of derivatives of xanthone compounds in the treatment of microbial disease.

### Background

Typically, antibiotic medications are used to treat microbial infections in patients. Many diseases that once killed people can now be treated effectively with antibiotics. However, some microbial organisms have become resistant to commonly used antibiotics.

Antibiotic resistant bacteria are bacteria that are extremely difficult to kill by most antibiotics and often require very high concentrations or combinations of several antibiotics. They are able to survive and even multiply in the presence of one or more antibiotic agents. Most infection-causing bacteria can become resistant to at least some antibiotics. Bacteria that are resistant to many antibiotics are known as multi-resistant organisms (MROs) or multi-drug resistant MDRs.

Antibiotic resistance can cause serious, prolonged disease and/or death and is an important public health problem world-wide. It can be minimised by avoiding unnecessary prescribing and overprescribing of antibiotics, the correct use of prescribed antibiotics, and good hygiene and infection control.

Some bacteria are naturally resistant to some antibiotics. For example, benzyl penicillin has very little effect on most organisms found in the human digestive system (gut). Some bacteria have developed resistance to antibiotics that were once commonly used to treat them. For example, *Staphylococcus aureus* ('golden staph') and *Neisseria gonorrhoeae* (the cause of gonorrhoea) are now almost always resistant to benzyl penicillin. In the past, these infections were usually controlled by penicillin.

The most serious concern with antibiotic resistance is that the bacteria have become resistant to almost all of the easily available antibiotics. Important examples are methicillin-resistant Staphylococcus aureus (MRSA), vancomycin-resistant Enterococcus (VRE) and multi-drug-resistant Mycobacterium tuberculosis (MDR-TB).

MRSA is currently the most serious hospital-acquired infection and as it does not respond to treatments with almost all the known and most powerful antibiotics, it has been termed a "superbug" out of human control. In fact, because of the untamed nature of the "superbug", experts have been predicting that the era of antibiotics is over.

One promising approach to efficiently address MRSA infection is to develop a new generation of antibiotics by approaches of molecular mimicking of natural antimicrobial peptides (AMPs) and utilising natural compounds.

There is a need to provide an alternative antibiotic agent that overcomes, or at least ameliorates, one or more of the disadvantages described above.

### Summary

Described herein for reference is a compound of Formula I or a salt thereof: 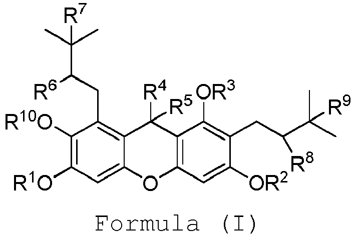 wherein
each of R¹ and R² are each independently is hydrogen, alkyl, alkenyl, alkynyl, cycloalkyl, cycloalkenyl, aralkyl, heterocycloalkyl, heterocycloalkenyl, heteroaryl, heteroaralkyl, -(C=O)R¹¹, -(C=O)OR¹¹, -(C=O)NR¹²R¹³, -SO₂R¹¹,-SO₂NR¹²R¹³, or R¹⁴, wherein R¹⁴ is 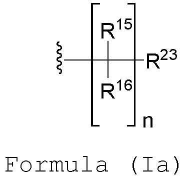 or 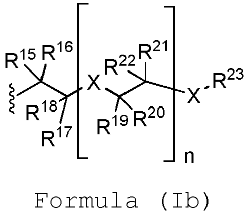 wherein
n is 0-20;
X is -O-, - (C=O)-, -O(C=O)-, -(C=O)O-, -N(R¹¹)-,-(C=O)N(R¹¹), -N(R¹¹)(C=O)-, -O(C=O)N(R¹¹)-, -N(R¹¹)(C=O)O-, -N(R¹²)(C=O)N(R¹³)-, -NR¹¹SO₂-, or -SO₂NR¹¹-;
each of R¹⁵, R¹⁶, R¹⁷, R¹⁸, R¹⁹, R²⁰, R²¹, and R²² independently for each occurrence is hydrogen, alkyl, alkenyl, alkynyl, cycloalkyl, cycloalkenyl, cycloalkenyl, aralkyl, heteroaryl, heterocycloalkyl, heterocycloalkenyl, halide, cyano, nitro, isocyanate, -R¹⁴, -OR¹¹, -SR¹¹, -(C=O)R¹¹,-(C=O)OR¹¹, -O(C=O)R¹¹, -O(C=O)OR¹¹, -(C=O)NR¹²R¹³,-O(C=O)NR¹²R¹³, -NR¹²(C=O)OR¹³, -NR¹²R¹³, -N(R¹²)₂R¹³, -N(R¹²)-NR¹²R¹³, -NR¹²(C=O)R¹³, -(S=O)R¹¹, -SO₂R¹¹, -SO₃R¹¹, -OSO₃R¹¹,-OPO₂OR¹¹, -SO₂NR¹²R¹³, -(C=NR¹²)NR¹²R¹³, -NR¹³(C=NR¹²) R¹¹, or-N(R¹³)(C=NR¹²)NR¹²R¹³; or any two instances of R¹⁵, R¹⁶, R¹⁷, R¹⁸, R¹⁹, R²⁰, R²¹, R²², or R²³ taken together with the carbon to which they are bonded form (C=O); or any two instances of R¹⁵, R¹⁶, R¹⁷, R¹⁸, R¹⁹, R²⁰, R²¹, R²², or R²³ taken together with the carbon to which they are bonded form a 3-8 membered carbocylic or heterocyclic ring; or any two instances of R¹⁵, R¹⁶, R¹⁷, R¹⁸, R¹⁹, R²⁰, R²¹, R²², or R²³ taken together form a bond;
R²³ independently for each occurrence is hydrogen, alkyl, alkenyl, alkynyl, cycloalkyl, cycloalkenyl, aralkyl, heteroaryl, heterocycloalkyl, heterocycloalkenyl, halide, cyano, nitro, isocyanate, -R¹⁴, -OR¹¹, -SR¹¹, -(C=O)R¹¹,-(C=O)OR¹¹, -O(C=O)R¹¹, -O(C=O)OR¹¹, -(C=O)NR¹²R¹³,-O(C=O)NR¹²R¹³, -NR¹²(C=O)OR¹³, -NR¹²R¹³, -N(R¹²)₂R¹³, -N(R¹²)-NR¹²R¹³, -NR¹²(C=O)R¹³, -(S=O)R¹¹, -SO₂R¹¹, -SO₃R¹¹, -OSO₃R¹¹,-OPO₂OR¹¹, -PO₂OR¹¹, -SO₂NR¹²R¹³, -(C=NR¹²)NR¹²R¹³,-NR¹³(C=NR¹²)R¹¹, -NR¹¹(C=NR¹²)R¹³, -N(R¹³)(C=NR¹²)NR¹²R¹³, 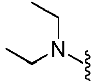 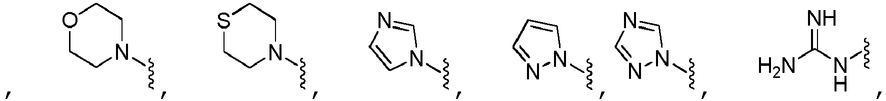 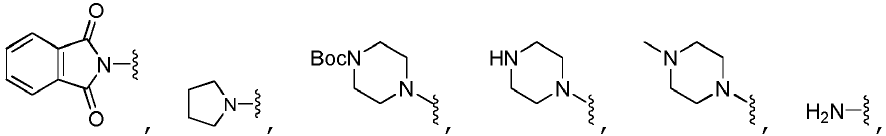 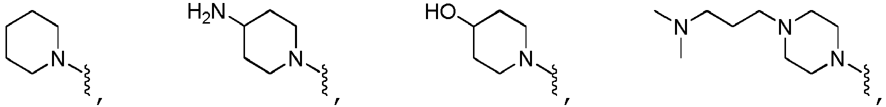 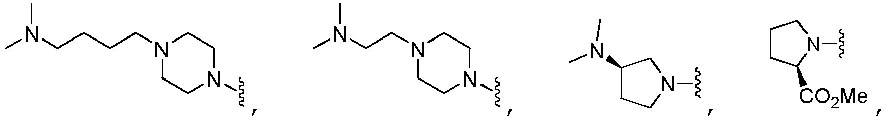 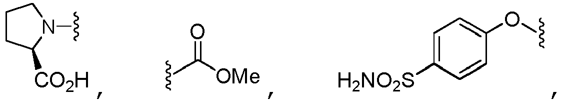 -OPO₂OH, -OSO₃H, 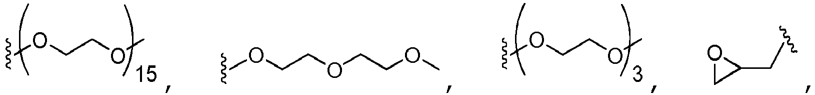 or 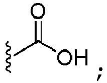
each of R³ and R¹⁰ independently for each occurrence is hydrogen, alkyl, alkenyl, alkynyl, cycloalkyl, cycloalkenyl, aralkyl, heterocycloalkyl, heterocycloalkenyl, heteroaryl, heteroaralkyl, -O(C=O)R¹¹, -O(C=O)OR¹¹, -O(C=O)NR¹²R¹³, -OSO₃R¹¹, or -OPO₂OR¹¹;
each of R⁴ and R⁵ independently is hydrogen, alkyl, alkenyl, alkynyl, cycloalkyl, cycloalkenyl, aralkyl, heterocycloalkyl, heterocycloalkenyl, heteroaryl, heteroaralkyl, -OR¹¹, -O(C=O)R¹¹, -O(C=O)OR¹¹, or-O(C=O)NR¹²R¹³; or R⁴ and R⁵ taken together with the carbon to which they are bonded form (C=O);
each of R⁶, R⁷, R⁸, and R⁹ independently for each occurrence is hydrogen; or R⁶ and R⁷ taken together form a bond; or R⁸ and R⁹ taken together form a bond;
R¹¹ independently for each occurrence is hydrogen, alkyl, alkenyl, alkynyl, cycloalkyl, cycloalkenyl, aralkyl, heterocycloalkyl, heterocycloalkenyl, heteroaryl, heteroaralkyl, or R¹⁴; and
each of R¹² and R¹³ independently for each occurrence is hydrogen, alkyl, alkenyl, alkynyl, cycloalkyl, cycloalkenyl, aralkyl, heterocycloalkyl, heterocycloalkenyl, heteroaryl, heteroaralkyl, or R¹⁴; or R¹² or R¹³ taken together form a 3-8 membered heterocyclic ring, wherein at least one of R¹ or R² is R¹⁴.

According to a first aspect of the invention, there is provided a compound of Formula II or a salt thereof: 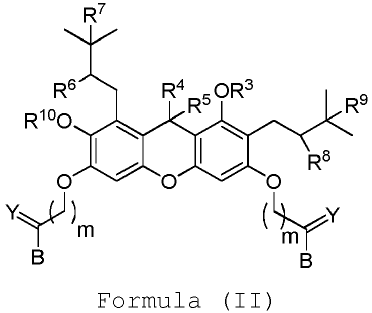 wherein
m is 1;
Y is O;
B is NHR²⁴;
each of R³ and R¹⁰ independently for each occurrence is hydrogen, alkyl, alkenyl, alkynyl, cycloalkyl, cycloalkenyl, aralkyl, heterocycloalkyl, heterocycloalkenyl, heteroaryl, heteroaralkyl, -O(C=O)R¹¹, -O(C=O)OR¹¹, -O(C=O)NR¹²R¹³, -OSO₃R¹¹, or -OPO₂OR¹¹;
R⁴ and R⁵ taken together with the carbon to which they are bonded form (C=O);
R⁶ and R⁷ taken together form a bond; R⁸ and R⁹ taken together form a bond;
R¹¹ is independently for each occurrence hydrogen, alkyl, alkenyl, alkynyl, cycloalkyl, cycloalkenyl, aralkyl, heterocycloalkyl, heterocycloalkenyl, heteroaryl, or heteroaralkyl;
each of R¹² and R¹³ independently for each occurrence is hydrogen, alkyl, alkenyl, alkynyl, cycloalkyl, cycloalkenyl, aralkyl, heterocycloalkyl, heterocycloalkenyl, heteroaryl, or heteroaralkyl; or R¹² or R¹³ taken together form a 3-8 membered heterocyclic ring;
R²⁴ is 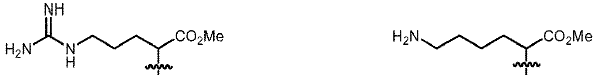 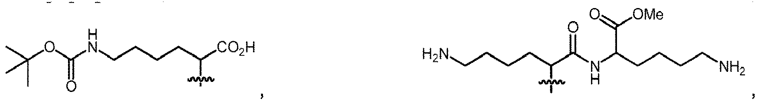 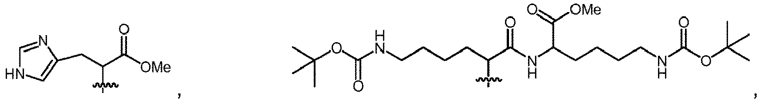 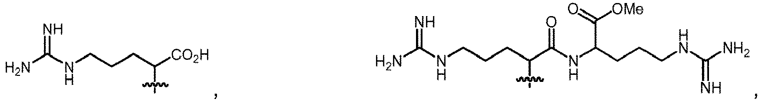 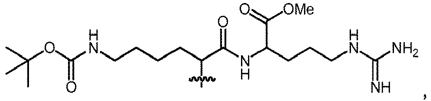 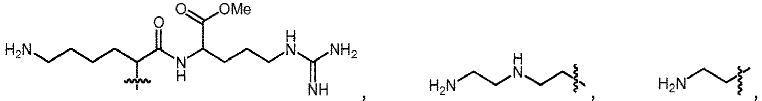 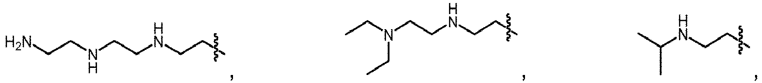 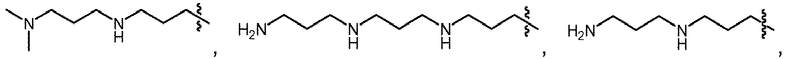 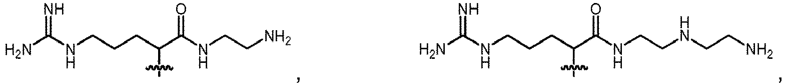 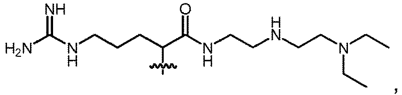 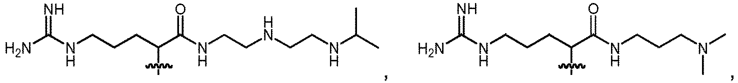 or

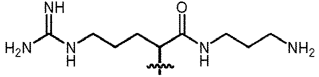

Also described herein for reference is a compound of Formula III or a salt thereof: 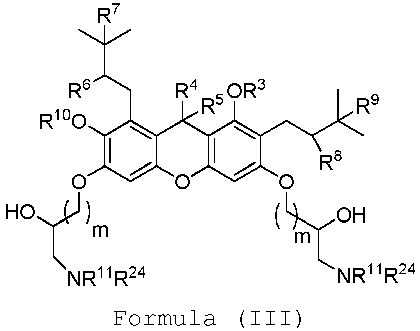 wherein
m is 1-10;
each of R³ and R¹⁰ independently for each occurrence is hydrogen, alkyl, alkenyl, alkynyl, cycloalkyl, cycloalkenyl, aralkyl, heterocycloalkyl, heterocycloalkenyl, heteroaryl, heteroaralkyl, -O(C=O)R¹¹, -O(C=O)OR¹¹, -O(C=O)NR¹²R¹³, -OSO₃R¹¹, or -OPO₂OR¹¹;
R⁴ and R⁵ are independently selected from hydrogen, alkyl, alkenyl, alkynyl, cycloalkyl, cycloalkenyl, aralkyl, heterocycloalkyl, heterocycloalkenyl, heteroaryl, heteroaralkyl, -OR¹¹, -O(C=O)R¹¹, -O(C=O)OR¹¹, and-O(C=O)NR¹²R¹³; or R⁴ and R⁵ taken together with the carbon to which they are bonded form (C=O);
each of R⁶, R⁷, R⁸, and R⁹ independently for each occurrence is hydrogen; or R⁶ and R⁷ taken together form a bond; or R⁸ and R⁹ taken together form a bond;
R¹¹ is independently for each occurrence is hydrogen, alkyl, alkenyl, alkynyl, cycloalkyl, cycloalkenyl, aralkyl, heterocycloalkyl, heterocycloalkenyl, heteroaryl, heteroaralkyl; or R¹¹ and R²⁴ taken together with the nitrogen to which they are attached form a 3-8 membered heterocyclic ring;
each of R¹² and R¹³ independently for each occurrence is hydrogen, alkyl, alkenyl, alkynyl, cycloalkyl, cycloalkenyl, aralkyl, heterocycloalkyl, heterocycloalkenyl, heteroaryl, or heteroaralkyl; or R¹² or R¹³ taken together form a 3-8 membered heterocyclic ring; R²⁴ is 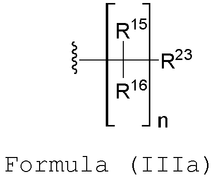 or 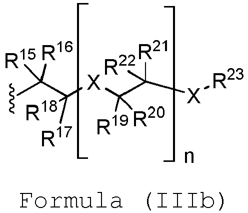
wherein
n is 0-12;
X is -O-, -(C=O)-, -O(C=O)-, -(C=O)O-, -N(R¹¹)-,-(C=O)N(R¹¹), -N(R¹¹)(C=O)-, -O(C=O)N(R¹¹)-, -N(R¹¹)(C=O)O-, -N(R¹²)(C=O)N(R¹³)-, -NR¹¹SO₂-, or -SO₂NR¹¹-;
each of R¹⁵, R¹⁶, R¹⁷, R¹⁸, R¹⁹, R²⁰, R²¹, and R²² independently for each occurrence is hydrogen, alkyl, alkenyl, alkynyl, cycloalkyl, cycloalkenyl, aralkyl, heteroaryl, heterocycloalkyl, heterocycloalkenyl, halide, cyano, nitro, isocyanate, -R²⁴, -OR¹¹, -SR¹¹, -(C=O)R¹¹, -(C=O)OR¹¹, -O(C=O)R¹¹, -O(C=O)OR¹¹, -(C=O)NR¹²R¹³, -O(C=O)NR¹²R¹³,-NR¹²(C=O)OR¹³, -NR¹²R¹³, -N(R¹²)₂R¹³, -N(R¹²)-NR¹²R¹³,-NR¹²(C=O)R¹³, -(S=O)R¹¹, -SO₂R¹¹, -SO₃R¹¹, -OSO₃R¹¹, -OPO₂OR¹¹, -PO₂OR¹¹, -SO₂NR¹²R¹³, -(C=NR¹²)NR¹²R¹³, -NR¹³(C=NR¹²)R¹¹,-NR¹¹ (C=NR¹²)R¹³, or -N(R¹³)(C=NR¹²)NR¹²R¹³; or any two instances of R¹⁵, R¹⁶, R¹⁷, R¹⁸, R¹⁹, R²⁰, R²¹, R²², or R²³ taken together with the carbon to which they are bonded form (C=O); or any two instances of R¹⁵, R¹⁶, R¹⁷, R¹⁸, R¹⁹, R²⁰, R²¹, R²², or R²³ taken together with the carbon to which they are bonded form a 3-8 membered carbocylic or heterocyclic ring; or any two instances of R¹⁵, R¹⁶, R¹⁷, R¹⁸, R¹⁹, R²⁰, R²¹, R²², or R²³ taken together form a bond;
R²³ independently for each occurrence is hydrogen, alkyl, alkenyl, alkynyl, cycloalkyl, cycloalkenyl, aralkyl, heteroaryl, heterocycloalkyl, heterocycloalkenyl, halide, cyano, nitro, isocyanate, -R¹⁴, -OR¹¹, -SR¹¹, -(C=O)R¹¹,-(C=O)OR¹¹, -O(C=O)R¹¹, -O(C=O)OR¹¹, -(C=O)NR¹²R¹³,-O(C=O)NR¹²R¹³, -NR¹²(C=O)OR¹³, -NR¹²R¹³, -N(R¹²)₂R¹³, -N(R¹²)-NR¹²R¹³, -NR¹²(C=O)R¹³, -(S=O)R¹¹, -SO₂R¹¹, -SO₃R¹¹, -OSO₃R¹¹,-OPO₂OR¹¹, -SO₂NR¹²R¹³, -(C=NR¹²)NR¹²R¹³, -NR¹³(C=NR¹²)R¹¹,

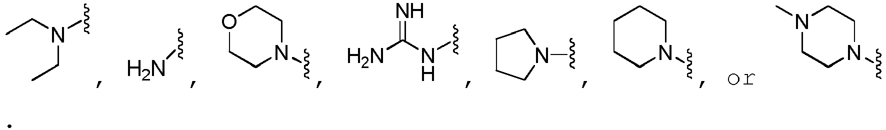

Also described herein for reference is a compound of formula:

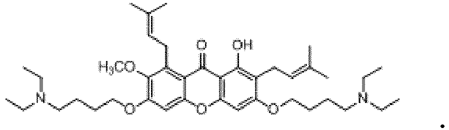

According to a second aspect, there is provided a compound of formula:

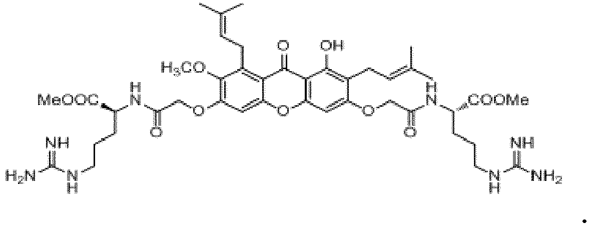

According to a third aspect, there is provided a composition comprising a compound as defined herein, together with a carrier.

According to a fourth aspect, there is provided a pharmaceutical composition comprising a compound as defined herein, together with a pharmaceutically acceptable carrier.

According to a fifth aspect, there is provided a compound as defined herein, for use as a medicament.

According to a sixth aspect, there is provided use of a compound as defined herein, in the preparation or manufacture of a medicament for the treatment of a microbial infection.

According to a seventh aspect, there is provided a pharmaceutical dosage form comprising a compound as defined herein.

According to an eighth aspect, there is provided a pharmaceutical dosage form comprising a composition as defined herein.

According to a ninth aspect, there is provided a kit comprising a compound as defined herein and directions for use.

According to a tenth aspect, there is provided a kit comprising a composition as defined herein and directions for use.

According to a eleventh aspect, there is provided a kit comprising the pharmaceutical dosage form as defined herein and directions for use.

According to a twelfth aspect, there is provided a compound as defined herein for use in a method for treating a microbial infection comprising the step of administering an effective amount of a compound as defined herein or a composition as defined herein to a patient in need of such treatment.

### Definitions

The following words and terms used herein shall have the meaning indicated:
The following are some definitions that may be helpful in understanding the description of the present invention. These are intended as general definitions and should in no way limit the scope of the present invention to those terms alone, but are put forth for a better understanding of the following description.
Unless the context requires otherwise or specifically stated to the contrary, integers, steps, or elements of the invention recited herein as singular integers, steps or elements clearly encompass both singular and plural forms of the recited integers, steps or elements.

Throughout this specification, unless the context requires otherwise, the word "comprise", or variations such as "comprises" or "comprising", will be understood to imply the inclusion of a stated step or element or integer or group of steps or elements or integers, but not the exclusion of any other step or element or integer or group of elements or integers. Thus, in the context of this specification, the term "comprising" means "including principally, but not necessarily solely".

Those skilled in the art will appreciate that the invention described herein is susceptible to variations and modifications other than those specifically described. It is to be understood that the invention includes all such variations and modifications, but that the scope of the invention is defined by the appended set of claims. The invention also includes all of the steps, features, compositions and compounds referred to or indicated in this specification, individually or collectively, and any and all combinations or any two or more of said steps or features.
In the context of this specification, the term "amino acid" is defined as having at least one primary, secondary, tertiary or quaternary amino group, and at least one acid group, wherein the acid group may be a carboxylic, sulfonic, or phosphonic acid, or mixtures thereof. The amino groups may be "alpha", "beta", "gamma" ... to "omega" with respect to the acid group(s). The backbone of the "amino acid" may be substituted with one or more groups selected from halogen, hydroxy, guanido, heterocyclic groups. Thus term "amino acids" also includes within its scope glycine, alanine, valine, leucine, isoleucine, methionine, proline, phenylalanine, tryptophane, serine, threonine, cysteine, tyrosine, asparagine, glutamine, asparte, glutamine, lysine, arginine and histidine, taurine, betaine, N-methylalanine etc. (L) and (D) forms of amino acids are included in the scope of this invention. The term "amino acid" is also intended to encompass unnatural or non-natural forms of amino acids such as ornithine and derivatives thereof, alanine derivatives, alicyclic amino acids, arginine derivatives, aromatic amino acids, asparagine derivatives, aspartic acid derivatives, beta-amino acids, cysteine derivatives, DAB (2,4-diaminobutyric acid, N-methyl amino acids, D-amino acids, diamino acids, DAP (2,3-diaminopropionic acid), glutamic acid derivatives, glutamine derivatives glycine derivatives, histidine derivatives, homo-amino acids, isoleucine derivatives, leucine derivatives, linear core amino acids, lysine derivatives, methionine derivatives, N-methyl amino acids, norleucine derivatives, norvaline derivatives, penicillamine derivatives, phenylalanine derivatives, phenylglycine derivatives, proline derivatives, pyruvic acid derivatives, pyroglutamine derivatives, serine derivatives, threonine derivatives, tryptophan derivatives, tyrosine derivatives and valine derivatives.

The term "oligopeptide" refers to a peptide comprising from 2 to 20 amino acids.

As used herein, the term "alkyl group" includes within its meaning monovalent ("alkyl") and divalent ("alkylene") straight chain or branched chain saturated aliphatic groups having from 1 to 10 carbon atoms, eg, 1, 2, 3, 4, 5, 6, 7, 8, 9, or 10 carbon atoms. For example, the term alkyl includes, but is not limited to, methyl, ethyl, 1-propyl, isopropyl, 1-butyl, 2-butyl, isobutyl, tert-butyl, amyl, 1,2-dimethylpropyl, 1,1-dimethylpropyl, pentyl, isopentyl, hexyl, 4-methylpentyl, 1-methylpentyl, 2-methylpentyl, 3-methylpentyl, 2,2-dimethylbutyl, 3,3-dimethylbutyl, 1,2-dimethylbutyl, 1,3-dimethylbutyl, 1,2,2-trimethylpropyl, 1,1,2-trimethylpropyl, 2-ethylpentyl, 3-ethylpentyl, heptyl, 1-methylhexyl, 2,2-dimethylpentyl, 3,3-dimethylpentyl, 4,4-dimethylpentyl, 1,2-dimethylpentyl, 1,3-dimethylpentyl, 1,4-dimethylpentyl, 1,2,3-trimethylbutyl, 1,1,2-trimethylbutyl, 1,1,3-trimethylbutyl, 5-methylheptyl, 1-methylheptyl, octyl, nonyl, decyl, and the like.

The term "alkenyl group" includes within its meaning monovalent ("alkenyl") and divalent ("alkenylene") straight or branched chain unsaturated aliphatic hydrocarbon groups having from 2 to 10 carbon atoms, eg, 2, 3, 4, 5, 6, 7, 8, 9, or 10 carbon atoms and having at least one double bond, of either *E, Z, cis* or *trans* stereochemistry where applicable, anywhere in the alkyl chain. Examples of alkenyl groups include but are not limited to ethenyl, vinyl, allyl, 1-methylvinyl, 1-propenyl, 2-propenyl, 2-methyl-1-propenyl, 2-methyl-1-propenyl, 1-butenyl, 2-butenyl, 3-butentyl, 1,3-butadienyl, 1-pentenyl, 2-pententyl, 3-pentenyl, 4-pentenyl, 1,3-pentadienyl, 2,4-pentadienyl, 1,4-pentadienyl, 3-methyl-2-butenyl, 1-hexenyl, 2-hexenyl, 3-hexenyl, 1,3-hexadienyl, 1,4-hexadienyl, 2-methylpentenyl, 1-heptenyl, 2-heptentyl, 3-heptenyl, 1-octenyl, 1-nonenyl, 1-decenyl, and the like.

The term "alkynyl group" as used herein includes within its meaning monovalent ("alkynyl") and divalent ("alkynylene") straight or branched chain unsaturated aliphatic hydrocarbon groups having from 2 to 10 carbon atoms and having at least one triple bond anywhere in the carbon chain. Examples of alkynyl groups include but are not limited to ethynyl, 1-propynyl, 1-butynyl, 2-butynyl, 1-methyl-2-butynyl, 3-methyl-1-butynyl, 1-pentynyl, 1-hexynyl, methylpentynyl, 1-heptynyl, 2-heptynyl, 1-octynyl, 2-octynyl, 1-nonyl, 1-decynyl, and the like.

The term "cycloalkyl" as used herein refers to cyclic saturated aliphatic groups and includes within its meaning monovalent ("cycloalkyl"), and divalent ("cycloalkylene"), saturated, monocyclic, bicyclic, polycyclic or fused polycyclic hydrocarbon radicals having from 3 to 10 carbon atoms, eg, 3, 4, 5, 6, 7, 8, 9, or 10 carbon atoms. Examples of cycloalkyl groups include but are not limited to cyclopropyl, 2-methylcyclopropyl, cyclobutyl, cyclopentyl, 2-methylcyclopentyl, 3-methylcyclopentyl, cyclohexyl, and the like.

The term "heteroalkyl" refers to a straight-or branched-chain alkyl group having from 2 to 12 atoms in the chain, one or more of which is a heteroatom selected from S, O, and N. Exemplary heteroalkyls include alkyl ethers, secondary and tertiary alkyl amines, alkyl sulfides, and the like.

The term "cycloalkenyl" as used herein, refers to cyclic unsaturated aliphatic groups and includes within its meaning monovalent ("cycloalkenyl") and divalent ("cycloalkenylene"), monocyclic, bicyclic, polycyclic or fused polycyclic hydrocarbon radicals having from 3 to 10 carbon atoms and having at least one double bond, of either *E, Z, cis* or *trans* stereochemistry where applicable, anywhere in the alkyl chain. Examples of cycloalkenyl groups include but are not limited to cyclopropenyl, cyclopentenyl, cyclohexenyl, and the like.

The term "heterocycloalkyl" as used herein, includes within its meaning monovalent ("heterocycloalkyl") and divalent ("heterocycloalkylene"), saturated, monocyclic, bicyclic, polycyclic or fused hydrocarbon radicals having from 3 to 10 ring atoms wherein 1 to 5 ring atoms are heteroatoms selected from O, N, NH, or S. Examples include azetidinyl, oxiranyl, cyclohexylimino, imdazolidinyl, imidazolinyl, morpholinyl, piperazinyl, piperidinyl, pyridyl, pyrazolidinyl, pyrazolinyl, pyrrolidinyl, pyrrolinyl, quinuclidinyl, tetrahydrofuranyl, tetrahydrothiophenyl, tetrahydropyranyl, and the like.

The term "heterocycloalkenyl" as used herein, includes within its meaning monovalent ("heterocycloalkenyl") and divalent ("heterocycloalkenylene"), saturated, monocyclic, bicyclic, polycyclic or fused polycyclic hydrocarbon radicals having from 3 to 10 ring atoms and having at least 1 double bond, wherein from 1 to 5 ring atoms are heteroatoms selected from O, N, NH or S.

The term "heteroaromatic group" and variants such as "heteroaryl" or "heteroarylene" as used herein, includes within its meaning monovalent ("heteroaryl") and divalent ("heteroarylene"), single, polynuclear, conjugated and fused aromatic radicals having 6 to 20 atoms wherein 1 to 6 atoms are heteroatoms selected from O, N, NH and S. Examples of such groups include benzimidazolyl, benzisoxazolyl, benzofuranyl, benzopyrazolyl, benzothiadiazolyl, benzothiazolyl, benzothienyl, benzotriazolyl, benzoxazolyl, furanyl, furazanyl, furyl, imidazolyl, indazolyl, indolizinyl, indolinyl, indolyl, isobenzofuranyl, isoindolyl, isothiazolyl, isoxazolyl, oxazolyl, phenanthrolinyl, purinyl, pyrazinyl, pyrazolyl, pyridazinyl, pyridinyl, 2,2'-pyridinyl, pyrimidinyl, pyrrolyl, quinolinyl, quinolyl, thiadiazolyl, thiazolyl, thiophenyl, triazolyl, and the like.

The term "halogen" or variants such as "halide" or "halo" as used herein refers to fluorine, chlorine, bromine and iodine.

The term "heteroatom" or variants such as "hetero-" as used herein refers to O, N, NH and S.

The term "alkoxy" as used herein refers to straight chain or branched alkyloxy groups. Examples include methoxy, ethoxy, n-propoxy, isopropoxy, tert-butoxy, and the like.

The term "amino" as used herein refers to groups of the form -NRₐR_{b} wherein Rₐ and R_{b} are individually selected from the group including but not limited to hydrogen, optionally substituted alkyl, optionally substituted alkenyl, optionally substituted alkynyl, and optionally substituted aryl groups.

The term "aromatic group", or variants such as "aryl" or "arylene" as used herein refers to monovalent ("aryl") and divalent ("arylene") single, polynuclear, conjugated and fused residues of aromatic hydrocarbons having from 6 to 10 carbon atoms. Examples of such groups include phenyl, biphenyl, naphthyl, phenanthrenyl, and the like.

The term "aralkyl" as used herein, includes within its meaning monovalent ("aryl") and divalent ("arylene"), single, polynuclear, conjugated and fused aromatic hydrocarbon radicals attached to divalent, saturated, straight and branched chain alkylene radicals.

The term "heteroaralkyl" as used herein, includes within its meaning monovalent ("heteroaryl") and divalent ("heteroarylene"), single, polynuclear, conjugated and fused aromatic hydrocarbon radicals attached to divalent saturated, straight and branched chain alkylene radicals.

The term "optionally substituted" as used herein means the group to which this term refers may be unsubstituted, or may be substituted with one or more groups independently selected from alkyl, alkenyl, alkynyl, thioalkyl, cycloalkyl, cycloalkenyl, heterocycloalkyl, halo, carboxyl, haloalkyl, haloalkynyl, hydroxyl, alkoxy, thioalkoxy, alkenyloxy, haloalkoxy, haloalkenyloxy, nitro, amino, nitroalkyl, nitroalkenyl, nitroalkynyl, nitroheterocyclyl, alkylamino, dialkylamino, alkenylamine, alkynylamino, acyl, alkenoyl, alkynoyl, acylamino, diacylamino, acyloxy, alkylsulfonyloxy, heterocycloxy, heterocycloamino, haloheterocycloalkyl, alkylsulfenyl, alkylcarbonyloxy, alkylthio, acylthio, phosphorus-containing groups such as phosphono and phosphinyl, aryl, heteroaryl, alkylaryl, alkylheteroaryl, cyano, cyanate, isocyanate, -C(O)NH(alkyl), and-C(O)N(alkyl)₂.

The term "haloalkyl" refers to a straight-or branched-chain alkenyl group having from 2- 12 carbon atoms in the chain and where one or more hydrogens is substituted with a halogen. Illustrative haloalkyl groups include trifluoromethyl, 2-bromopropyl, 3-chlorohexyl, 1-iodo-isobutyl, and the like.

The present invention includes within its scope all isomeric forms of the compounds disclosed herein, including all diastereomeric isomers, racemates and enantiomers. Thus, formulae (I) and (II) should be understood to include, for example, E, Z, cis, trans, (R), (S), (L), (D), (+), and/or (-) forms of the compounds, as appropriate in each case.

In the context of this invention the term "administering" and variations of that term including "administer" and "administration", includes contacting, applying, delivering or providing a compound or composition of the invention to an organism, or a surface by any appropriate means.

In the context of this specification, the term "patient" includes humans and individuals of any species of social, economic or research importance including but not limited to members of the genus ovine, bovine, equine, porcine, feline, canine, primates (including human and non-human primates), rodents, murine, caprine, leporine, and avian.

The term "substituted" is intended to indicate that one or more (e.g., 1, 2, 3, 4, or 5; in some embodiments 1, 2, or 3; and in other embodiments 1 or 2) hydrogen atoms on the group indicated in the expression using "substituted" is replaced with a selection from the indicated organic or inorganic group(s), or with a suitable organic or inorganic group known to those of skill in the art, provided that the indicated atom's normal valency is not exceeded, and that the substitution results in a stable compound. Suitable indicated organic or inorganic groups include, e.g., alkyl, alkenyl, alkynyl, alkoxy, halo, haloalkyl, hydroxy, hydroxyalkyl, aryl, heteroaryl, heterocycle, cycloalkyl, alkanoyl, alkoxycarbonyl, amino, alkylamino, dialkylamino, trifluoromethylthio, difluoromethyl, acylamino, nitro, trifluoromethyl, trifluoromethoxy, carboxy, carboxyalkyl, keto, thioxo, alkylthio, alkylsulfinyl, alkylsulfonyl, alkylsilyl, and cyano. Additionally, the suitable indicated groups can include, e.g., -X, -R, -O - , -OR,-SR, -S-, -NR 2 , -NR 3 , =NR, -CX 3 , -CN, -OCN, -SCN,-N=C=O, -NCS, -NO, -NO 2 , =N 2 , -N 3 , NC(=O)R, -C(=O)R, -C(=O)NRR -S(=O) 2 O - , -S(=O) 2 OH, -S(=O) 2 R, -OS(=O) 2 OR, -S(=O) 2 NR, -S(=O)R, -OP(=O)O 2 RR, -P(=O)O 2 RR-P(=O)(O - ) 2 , -P(=O) (OH) 2 , -C(=O)R, -C(=O)X, -C(S)R,-C(O)OR, -C(O)O - , -C(S)OR, -C(O)SR, -C(S)SR, -C(O)NRR,-C(S)NRR, -C(NR)NRR, where each X is independently a halogen (or "halo" group) : F, Cl, Br, or I; and each R is independently H, alkyl, aryl, heterocycle, protecting group or prodrug moiety. As would be readily understood by one skilled in the art, when a substituent is keto (i.e., =O) or thioxo (i.e., =S), or the like, then two hydrogen atoms on the substituted atom are replaced.

In the context of this specification, the term "treatment", refers to any and all uses which remedy a disease state or symptoms, prevent the establishment of disease, or otherwise prevent, hinder, retard, or reverse the progression of disease or other undesirable symptoms in any way whatsoever.

In the context of this disclosure the term "administering" and variations of that term including "administer" and "administration", includes contacting, applying, delivering or providing a compound or composition of the invention to an organism, or a surface by any appropriate means.

In the context of this disclosure, the term "patient" includes humans and individuals of any species of social, economic or research importance including but not limited to members of the type ovine, bovine, equine, porcine, feline, canine, primates (including human and non-human primates), rodents, murine, caprine, leporine, and avian.
In the context of this disclosure, the term "treatment", refers to any and all uses which remedy a disease state or symptoms, prevent the establishment of disease, or otherwise prevent, hinder, retard, or reverse the progression of disease or other undesirable symptoms in any way whatsoever.

"Dosage unit form" as used herein refers to physically discrete units suited as unitary dosages for the individual to be treated; each unit containing a predetermined quantity of compound(s) is calculated to produce the desired therapeutic effect in association with the required pharmaceutical carrier. The compound(s) may be formulated for convenient and effective administration in effective amounts with a suitable pharmaceutically acceptable carrier in an acceptable dosage unit. In the case of compositions containing supplementary active ingredients, the dosages are determined by reference to the usual dose and manner of administration of the said ingredients.

In the context of this disclosure the term "therapeutically effective amount", include meaning a sufficient but non-toxic amount of a compound or composition of the invention to provide the desired therapeutic or diagnostic effect. The exact amount required will vary from subject to subject depending on factors such as the species being treated, the age and general condition of the subject, the severity of the condition being treated, the particular agent being administered, the mode of administration, and so forth. Thus, it is not possible to specify an exact "effective amount". However, for any given case, an appropriate "effective amount" may be determined by one of ordinary skill in the art using only routine experimentation.

The language "pharmaceutically acceptable carrier" is intended to include solvents, dispersion media, coatings, anti-bacterial and anti-fungal agents, isotonic and absorption delaying agents, and the like. The use of such media and agents for pharmaceutically active substances is well known in the art. Except insofar as any conventional media or agent is incompatible with the compound, use thereof in the therapeutic compositions and methods of treatment and prophylaxis is contemplated.

"Unit dose form" or "dosage form" or "dosage unit form" as used herein refers to physically discrete units suited as unitary dosages for the individual to be treated; each unit containing a predetermined quantity of compound(s) is calculated to produce the desired therapeutic effect in association with the required pharmaceutical carrier. The compound(s) may be formulated for convenient and effective administration in effective amounts with a suitable pharmaceutically acceptable carrier in an acceptable dosage unit. In the case of compositions containing supplementary active ingredients, the dosages are determined by reference to the usual dose and manner of administration of the said ingredients.

By "pharmaceutically acceptable salt" it is meant those salts which, within the scope of sound medical judgement, are suitable for use in contact with the tissues of humans and lower animals without undue toxicity, irritation, allergic response and the like, and are commensurate with a reasonable benefit/risk ratio. Pharmaceutically acceptable salts are well known in the art.

The word "substantially" does not exclude "completely" e.g. a composition which is "substantially free" from Y may be completely free from Y. Where necessary, the word "substantially" may be omitted from the definition of the invention.

Unless specified otherwise, the terms "comprising" and "comprise", and grammatical variants thereof, are intended to represent "open" or "inclusive" language such that they include recited elements but also permit inclusion of additional, unrecited elements.

As used herein, the term "about", in the context of concentrations of components of the formulations, typically means +/- 5% of the stated value, more typically +/- 4% of the stated value, more typically +/- 3% of the stated value, more typically, +/- 2% of the stated value, even more typically +/- 1% of the stated value, and even more typically +/- 0.5% of the stated value.

Throughout this disclosure, certain embodiments may be disclosed in a range format. It should be understood that the description in range format is merely for convenience and brevity and should not be construed as an inflexible limitation on the scope of the disclosed ranges. Accordingly, the description of a range should be considered to have specifically disclosed all the possible sub-ranges as well as individual numerical values within that range. For example, description of a range such as from 1 to 6 should be considered to have specifically disclosed sub-ranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Certain embodiments may also be described broadly and generically herein. Each of the narrower species and subgeneric groupings falling within the generic disclosure also form part of the disclosure. This includes the generic description of the embodiments with a proviso or negative limitation removing any subject matter from the genus, regardless of whether or not the excised material is specifically recited herein.

### Disclosure of Optional Embodiments

Exemplary, non-limiting embodiments of compounds provided herein will now be disclosed, sometimes for reference.

Described herein for reference is the compound or salt thereof of the following formula: 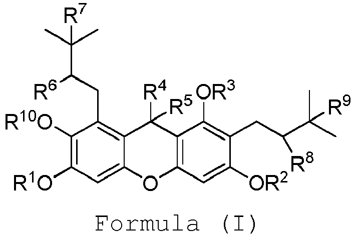 wherein
each of R¹ and R² are each independently is hydrogen, alkyl, alkenyl, alkynyl, cycloalkyl, cycloalkenyl, aralkyl, heterocycloalkyl, heterocycloalkenyl, heteroaryl, heteroaralkyl, -(C=O)R¹¹, -(C=O)OR¹¹, -(C=O)NR¹²R¹³, -SO₂R¹¹,-SO₂NR¹²R¹³, or R¹⁴, wherein R¹⁴ is 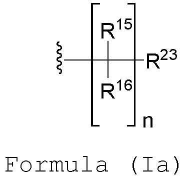 or 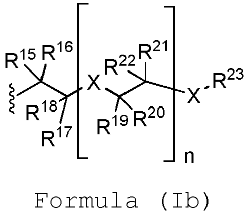 wherein
n is 0-20;
X is -O-, -(C=O)-, -O(C=O)-, -(C=O)O-, -N(R¹¹)-,-(C=O)N(R¹¹), -N(R¹¹)(C=O)-, -O(C=O)N(R¹¹)-, -N(R¹¹)(C=O)O-, -N(R¹²)(C=O)N(R¹³)-, -NR¹¹SO₂-, or -SO₂NR¹¹-;
each of R¹⁵, R¹⁶, R¹⁷, R¹⁸, R¹⁹, R²⁰, R²¹, and R²² independently for each occurrence is hydrogen, alkyl, alkenyl, alkynyl, cycloalkyl, cycloalkenyl, cycloalkenyl, aralkyl, heteroaryl, heterocycloalkyl, heterocycloalkenyl, halide, cyano, nitro, isocyanate, -R¹⁴, -OR¹¹, -SR¹¹, -(C=O)R¹¹,-(C=O)OR¹¹, -O(C=O)R¹¹, -O(C=O)OR¹¹, -(C=O)NR¹²R¹³,-O(C=O)NR¹²R¹³, -NR¹²(C=O)OR¹³, -NR¹²R¹³, -N(R¹²)₂R¹³, -N(R¹²)-NR¹²R¹³, -NR¹²(C=O)R¹³, -(S=O)R¹¹, -SO₂R¹¹, -SO₃R¹¹, -OSO₃R¹¹,-OPO₂OR¹¹, -SO₂NR¹²R¹³, -(C=NR¹²)NR¹²R¹³, -NR¹³(C=NR¹²)R¹¹, or-N(R¹³)(C=NR¹²)NR¹²R¹³; or any two instances of R¹⁵, R¹⁶, R¹⁷, R¹⁸, R¹⁹, R²⁰, R²¹, R²², or R²³ taken together with the carbon to which they are bonded form (C=O); or any two instances of R¹⁵, R¹⁶, R¹⁷, R¹⁸, R¹⁹, R²⁰, R²¹, R²², or R²³ taken together with the carbon to which they are bonded form a 3-8 membered carbocylic or heterocyclic ring; or any two instances of R¹⁵, R¹⁶, R¹⁷, R¹⁸, R¹⁹, R²⁰, R²¹, R²², or R²³ taken together form a bond;
R²³ independently for each occurrence is hydrogen, alkyl, alkenyl, alkynyl, cycloalkyl, cycloalkenyl, aralkyl, heteroaryl, heterocycloalkyl, heterocycloalkenyl, halide, cyano, nitro, isocyanate, -R¹⁴, -OR¹¹, -SR¹¹, -(C=O)R¹¹,-(C=O)OR¹¹, -O(C=O)R¹¹, -O(C=O)OR¹¹, -(C=O)NR¹²R¹³,-O(C=O)NR¹²R¹³, -NR¹²(C=O)OR¹³, -NR¹²R¹³, -N(R¹²)₂R¹³, -N(R¹²)-NR¹²R¹³, -NR¹²(C=O)R¹³, -(S=O)R¹¹, -SO₂R¹¹, -SO₃R¹¹, -OSO₃R¹¹,-OPO₂OR¹¹, -PO₂OR¹¹, -SO₂NR¹²R¹³, -(C=NR¹²)NR¹²R¹³,-NR¹³(C=NR¹²)R¹¹, -NR¹¹(C=NR¹²)R¹³, -N(R¹³)(C=NR¹²)NR¹²R¹³, 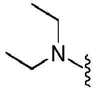 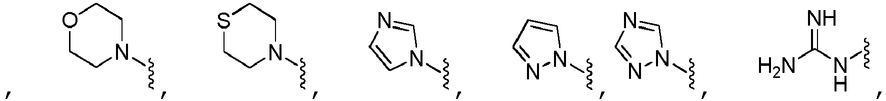 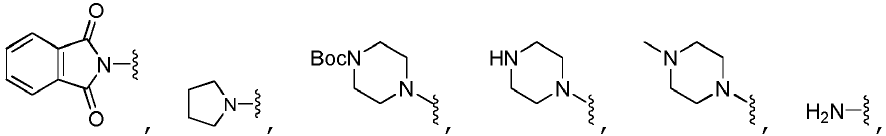 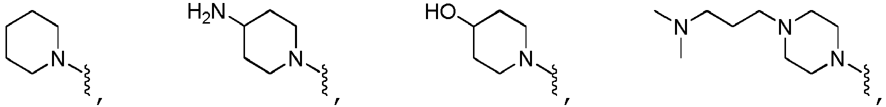 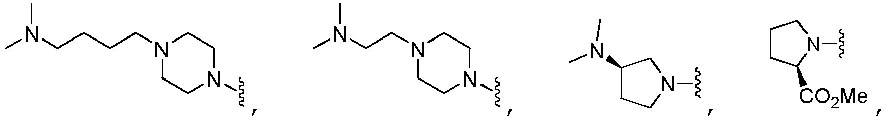 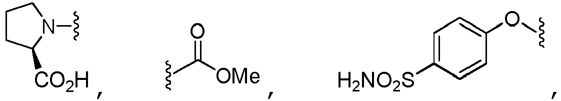 -OPO₂OH, -OSO₃H, 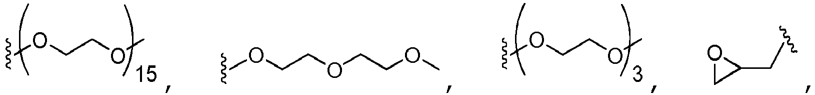 or 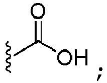
each of R³ and R¹⁰ independently for each occurrence is hydrogen, alkyl, alkenyl, alkynyl, cycloalkyl, cycloalkenyl, aralkyl, heterocycloalkyl, heterocycloalkenyl, heteroaryl, heteroaralkyl, -O(C=O)R¹¹, -O(C=O)OR¹¹, -O(C=O)NR¹²R¹³, -OSO₃R¹¹, or -OPO₂OR¹¹;
each of R⁴ and R⁵ independently is hydrogen, alkyl, alkenyl, alkynyl, cycloalkyl, cycloalkenyl, aralkyl, heterocycloalkyl, heterocycloalkenyl, heteroaryl, heteroaralkyl, -OR¹¹, -O(C=O)R¹¹, -O(C=O)OR¹¹, or-O(C=O)NR¹²R¹³; or R⁴ and R⁵ taken together with the carbon to which they are bonded form (C=O);
each of R⁶, R⁷, R⁸, and R⁹ independently for each occurrence is hydrogen; or R⁶ and R⁷ taken together form a bond; or R⁸ and R⁹ taken together form a bond;
R¹¹ independently for each occurrence is hydrogen, alkyl, alkenyl, alkynyl, cycloalkyl, cycloalkenyl, aralkyl, heterocycloalkyl, heterocycloalkenyl, heteroaryl, heteroaralkyl, or R¹⁴; and
each of R¹² and R¹³ independently for each occurrence is hydrogen, alkyl, alkenyl, alkynyl, cycloalkyl, cycloalkenyl, aralkyl, heterocycloalkyl, heterocycloalkenyl, heteroaryl, heteroaralkyl, or R¹⁴; or R¹² or R¹³ taken together form a 3-8 membered heterocyclic ring, wherein at least one of R¹ or R² is R¹⁴.
   R¹⁴ may be -(CH₂)ₙR²⁴ and n may be 1-20. n may be 2-12, 2-10, 2-8, 2-6, or 2-4.
   R¹⁰ may be alkyl.
   R⁴ and R⁵ taken together with the carbon to which they are bonded may form (C=O).
   R⁶ and R⁷ taken together may form a bond and R⁸ and R⁹ taken together may form a bond.
   Each of R¹ and R² may be R¹⁴.
   Each of R¹ and R² may be (CH₂)ₙR²³ and R²³ independently for each occurrence may be heteroaryl, heterocycloalkyl, heterocycloalkenyl, halide, -OR¹¹, -SR¹¹, -(C=O)R¹¹,-(C=O)OR¹¹, -O(C=O)R¹¹, -O(C=O)OR¹¹, -(C=O)NR¹²R¹³,-O(C=O)NR¹²R¹³, -NR¹²(C=O)OR¹³, -NR¹²R¹³, -N(R¹²)₂R¹³, -N(R¹²)-NR¹²R¹³, -NR¹²(C=O)R¹³, -(S=O)R¹¹, -SO₂R¹¹, -SO₃R¹¹, -OSO₃R¹¹,-OPO₂OR¹¹, -SO₂NR¹²R¹³, -(C=NR¹²)NR¹²R¹³, -NR¹³(C=NR¹²)R¹¹, or-N(R¹³)(C=NR¹²)NR¹²R¹³.
   R²³ may be selected from the group consisting of 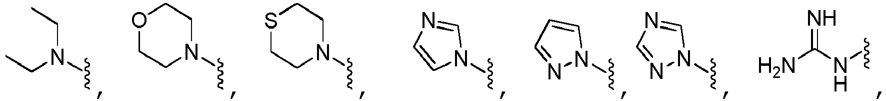 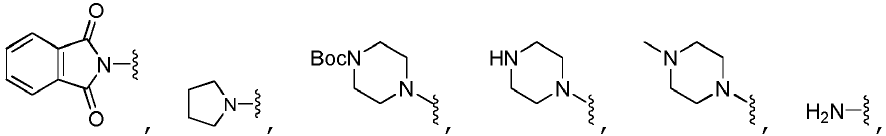 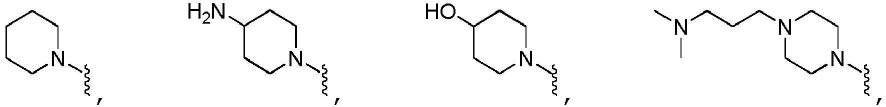 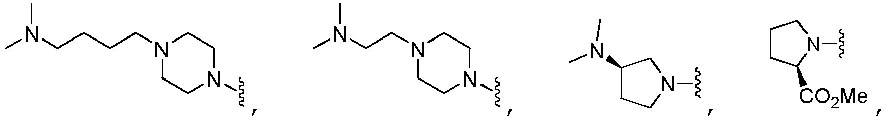 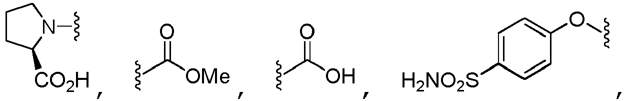 -OPO₂OH, -OSO₃H, 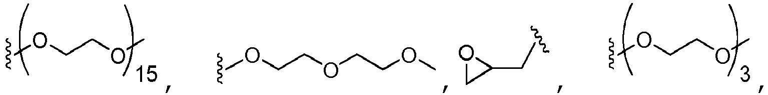 and bromide.
   TA compound or salt thereof according to the invention is of the following formula: 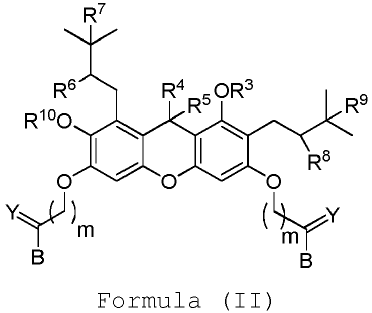
wherein
m is 1;
Y is O;
B is NHR²⁴;
each of R³ and R¹⁰ independently for each occurrence is hydrogen, alkyl, alkenyl, alkynyl, cycloalkyl, cycloalkenyl, aralkyl, heterocycloalkyl, heterocycloalkenyl, heteroaryl, heteroaralkyl, -O(C=O)R¹¹, -O(C=O)OR¹¹, -O(C=O)NR¹²R¹³, -OSO₃R¹¹, or -OPO₂OR¹¹;
R⁴ and R⁵ taken together with the carbon to which they are bonded form (C=O);
R⁶ and R⁷ taken together form a bond; R⁸ and R⁹ taken together form a bond;
R⁶ and R⁷ taken together form a bond; R⁸ and R⁹ taken together form a bond;
R¹¹ is independently for each occurrence hydrogen, alkyl, alkenyl, alkynyl, cycloalkyl, cycloalkenyl, aralkyl, heterocycloalkyl, heterocycloalkenyl, heteroaryl, or heteroaralkyl;
each of R¹² and R¹³ independently for each occurrence is hydrogen, alkyl, alkenyl, alkynyl, cycloalkyl, cycloalkenyl, aralkyl, heterocycloalkyl, heterocycloalkenyl, heteroaryl, or heteroaralkyl; or R¹² or R¹³ taken together form a 3-8 membered heterocyclic ring; R²⁴ is 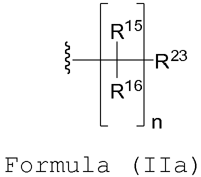 or
wherein
n is 0;
and R²³ is 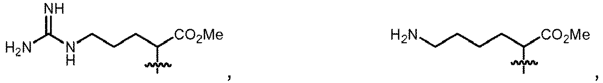 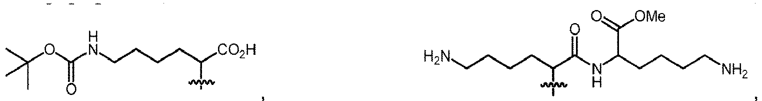 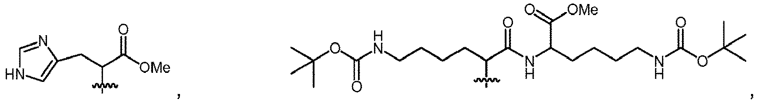 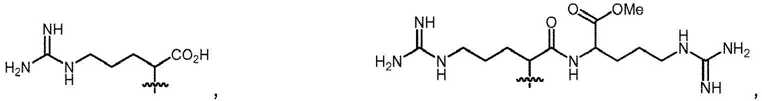 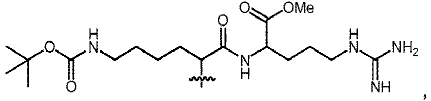 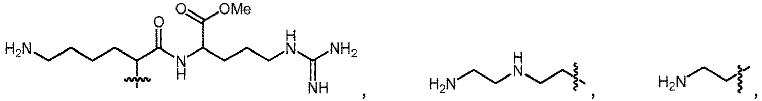 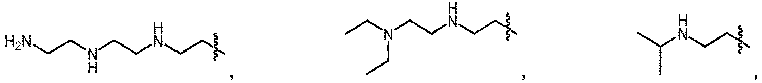 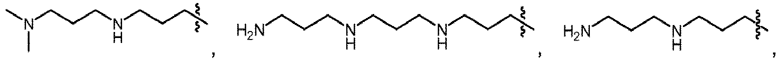 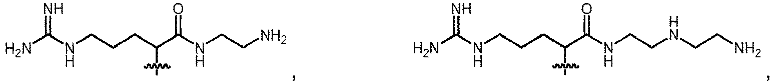 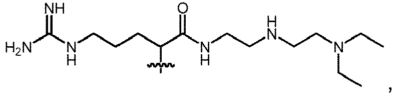 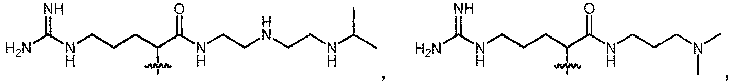 or

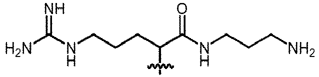

For reference R²⁴ and/or R²³ was previously referred to and selected from the group consisting of 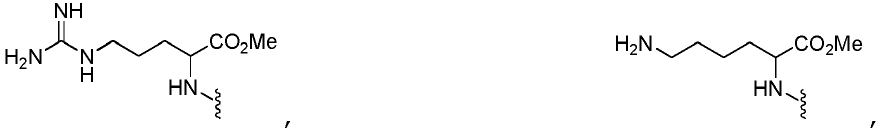 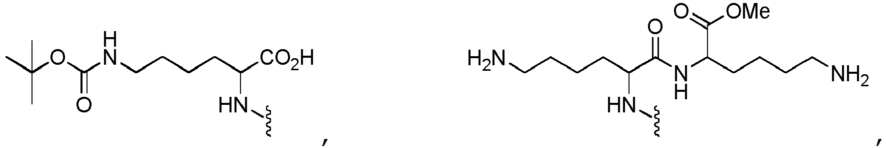 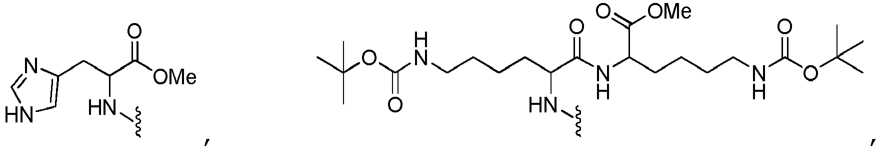 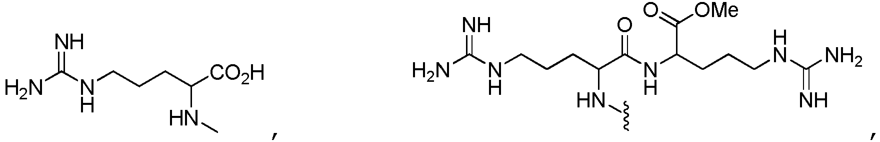 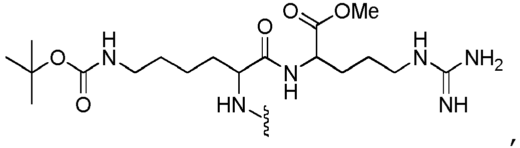 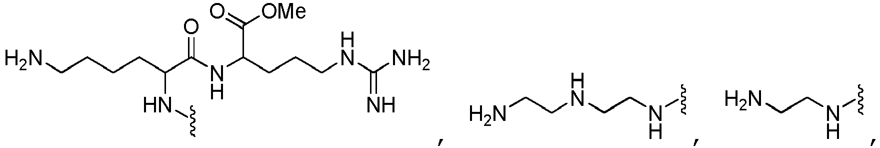 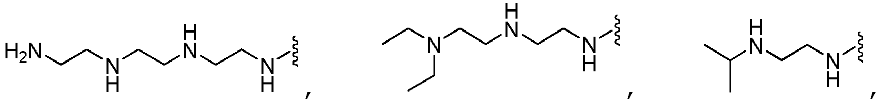 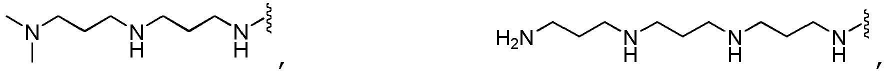 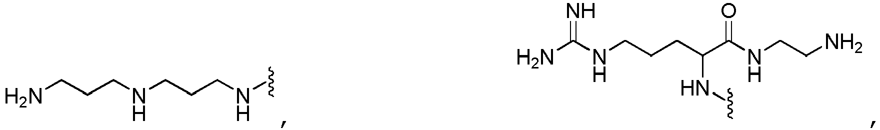 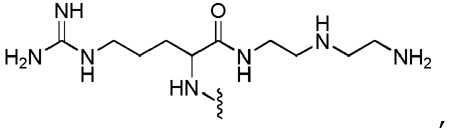 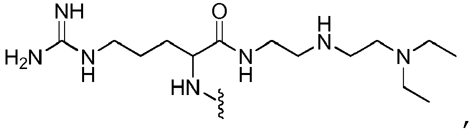 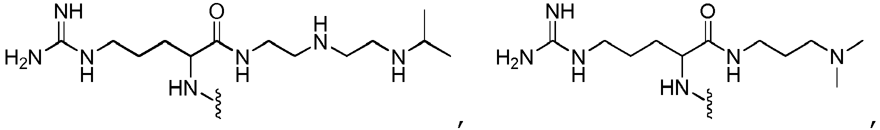 and 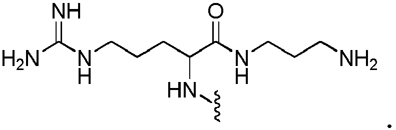 For reference, it is also described herein the remaining compounds or salts thereof of the following formula: 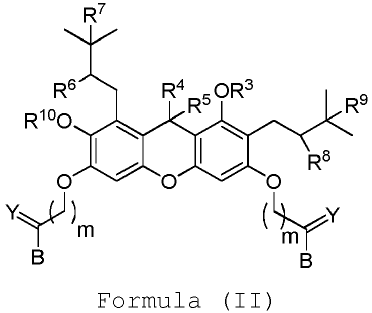 wherein
m is 0-10;
Y is O or H₂;
B is OH, OR¹¹, or NR¹¹R²⁴;
each of R³ and R¹⁰ independently for each occurrence is hydrogen, alkyl, alkenyl, alkynyl, cycloalkyl, cycloalkenyl, aralkyl, heterocycloalkyl, heterocycloalkenyl, heteroaryl, heteroaralkyl, -O(C=O)R¹¹, -O(C=O)OR¹¹, -O(C=O)NR¹²R¹³, -OSO₃R¹¹, or -OPO₂OR¹¹;
each of R⁴ and R⁵ is independently hydrogen, alkyl, alkenyl, alkynyl, cycloalkyl, cycloalkenyl, aralkyl, heterocycloalkyl, heterocycloalkenyl, heteroaryl, heteroaralkyl, -OR¹¹, -O(C=O)R¹¹, -O(C=O)OR¹¹, or-O(C=O)NR¹²R¹³; or R⁴ and R⁵ taken together with the carbon to which they are bonded form (C=O);
each of R⁶, R⁷, R⁸, and R⁹ independently for each occurrence is hydrogen; or R⁶ and R⁷ taken together form a bond; or R⁸ and R⁹ taken together form a bond;
R¹¹ is independently for each occurrence is hydrogen, alkyl, alkenyl, alkynyl, cycloalkyl, cycloalkenyl, aralkyl, heterocycloalkyl, heterocycloalkenyl, heteroaryl, or heteroaralkyl; or R¹¹ and R²⁴ taken together with the nitrogen to which they are attached form a 3-8 membered heterocyclic ring;
each of R¹² and R¹³ independently for each occurrence is hydrogen, alkyl, alkenyl, alkynyl, cycloalkyl, cycloalkenyl, aralkyl, heterocycloalkyl, heterocycloalkenyl, heteroaryl, or heteroaralkyl; or R¹² or R¹³ taken together form a 3-8 membered heterocyclic ring; R²⁴ is 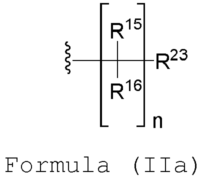 or 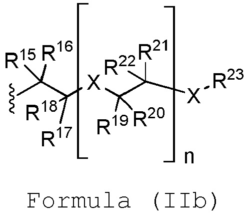
wherein
n is 0-12;
X is -O-, -(C=O)-, -O(C=O)-, -(C=O)O-, -N(R¹¹)-,-(C=O)N(R¹¹), -N(R¹¹)(C=O)-, -O(C=O)N(R¹¹)-, -N(R¹¹)(C=O)O-, -N(R¹²)(C=O)N(R¹³)-, -NR¹¹SO₂-, or -SO₂NR¹¹-;
   each of R¹⁵, R¹⁶, R¹⁷, R¹⁸, R¹⁹, R²⁰, R²¹, and R²² independently for each occurrence is hydrogen, alkyl, alkenyl, alkynyl, cycloalkyl, cycloalkenyl, aralkyl, heteroaryl, heterocycloalkyl, heterocycloalkenyl, halide, cyano, nitro, isocyanate, -R²⁴, -OR¹¹, -SR¹¹, -(C=O)R¹¹,-(C=O)OR¹¹, -O(C=O)R¹¹, -O(C=O)OR¹¹, - (C=O)NR¹²R¹³,-O(C=O)NR¹²R¹³, -NR¹²(C=O)OR¹³, -NR¹²R¹³, -N(R¹²)₂R¹³, -N(R¹²)-NR¹²R¹³, -NR¹²(C=O)R¹³, -(S=O)R¹¹, -SO₂R¹¹, -SO₃R¹¹, -OSO₃R¹¹,-OPO₂OR¹¹, -PO₂OR¹¹, -SO₂NR¹²R¹³, - (C=NR¹²)NR¹²R¹³,-NR¹³(C=NR¹²)R¹¹, -NR¹¹(C=NR¹²)R¹³, or -N(R¹³)(C=NR¹²)NR¹²R¹³; or any two instances of R¹⁵, R¹⁶, R¹⁷, R¹⁸, R¹⁹, R²⁰, R²¹, R²², or R²³ taken together with the carbon to which they are bonded form (C=O); or any two instances of R¹⁵, R¹⁶, R¹⁷, R¹⁸, R¹⁹, R²⁰, R²¹, R²², or R²³ taken together with the carbon to which they are bonded form a 3-8 membered carbocylic or heterocyclic ring; or any two instances of R¹⁵, R¹⁶, R¹⁷, R¹⁸, R¹⁹, R²⁰, R²¹, R²², or R²³ taken together form a bond; and R²³ independently for each occurrence is hydrogen, alkyl, alkenyl, alkynyl, cycloalkyl, cycloalkenyl, aralkyl, heteroaryl, heterocycloalkyl, heterocycloalkenyl, halide, cyano, nitro, isocyanate, an amino acid, a dipeptide, a tripeptide, an oligopeptide, -R²⁴, -OR¹¹,-SR¹¹, -(C=O)R¹¹, -(C=O)OR¹¹, -O(C=O)R¹¹, -O(C=O)OR¹¹,-(C=O)NR¹²R¹³, -O(C=O)NR¹²R¹³, -NR¹²(C=O)OR¹³, -NR¹²R¹³,-N(R¹²)₂R¹³, -N(R¹²)-NR¹²R¹³, -NR¹²(C=O)R¹³, -(S=O)R¹¹, -SO²R¹¹, -SO₃R¹¹, -OSO₃R¹¹, -OPO₂OR¹¹, -PO₂OR¹¹, -SO₂NR¹²R¹³,-(C=NR¹²)NR¹²R¹³, -NR¹³(C=NR¹²)R¹¹, -NR¹¹(C=NR¹²)R¹³,-N(R¹³)(C=NR¹²)NR¹²R¹³,
R¹⁰ may be alkyl.
   R⁴ and R⁵ taken together with the carbon to which they are bonded may form (C=O).
   R⁶ and R⁷ taken together may form a bond and R⁸ and R⁹ taken together may form a bond.
   M may be 1; B may be NHR²⁴, and R²⁴ may be selected from the group consisting of 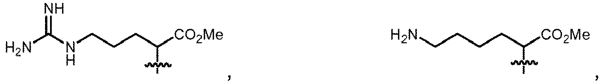 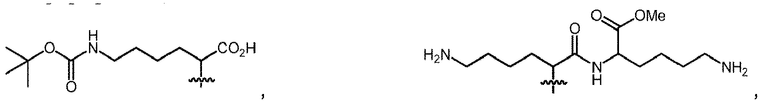 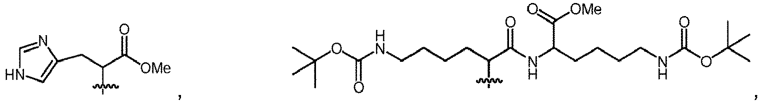 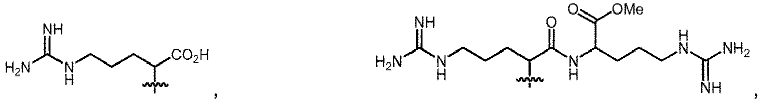 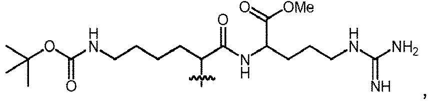 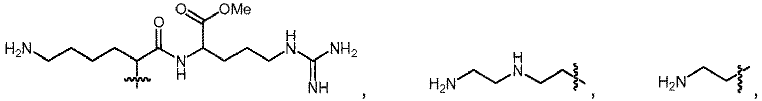 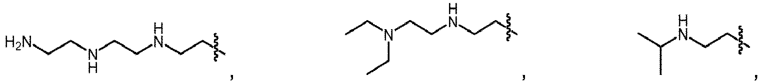 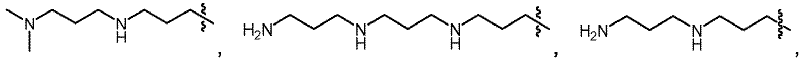 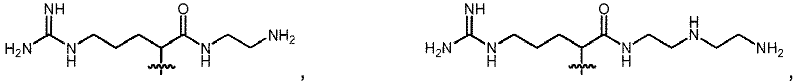 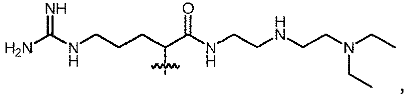 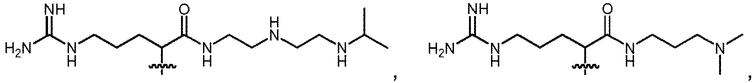 or 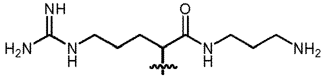 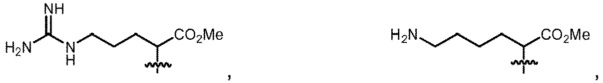 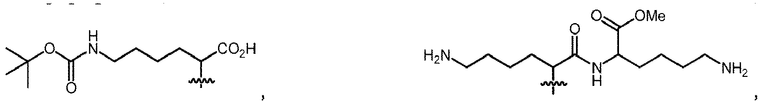 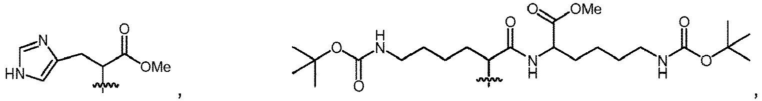 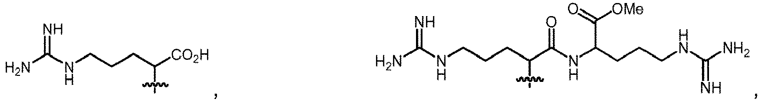 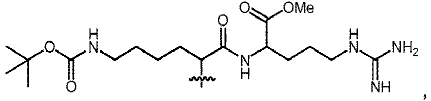 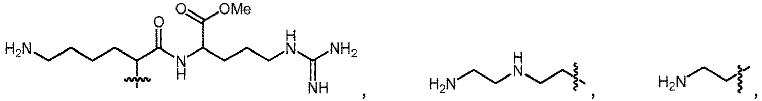 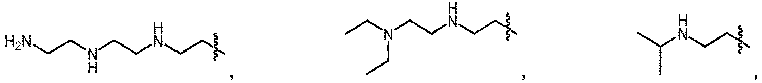 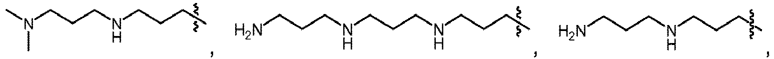 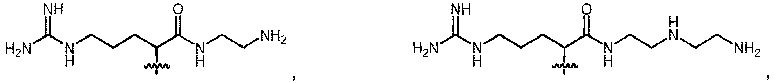 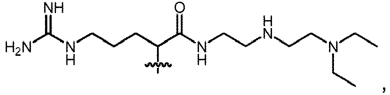 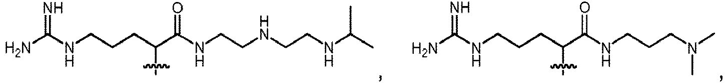 or

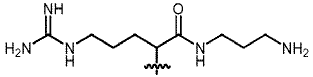

For reference, it is described herein the compound or salt thereof of the following formula: 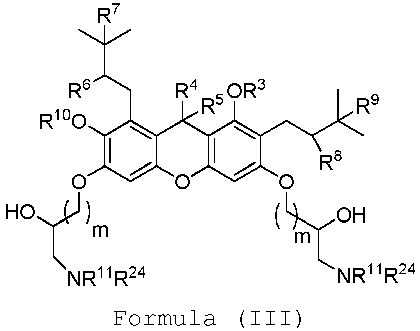 wherein
m is 1-10;
each of R³ and R¹⁰ independently for each occurrence is hydrogen, alkyl, alkenyl, alkynyl, cycloalkyl, cycloalkenyl, aralkyl, heterocycloalkyl, heterocycloalkenyl, heteroaryl, heteroaralkyl, -O(C=O)R¹¹, -O(C=O)OR¹¹, -O(C=O)NR¹²R¹³, -OSO₃R¹¹, or -OPO₂OR¹¹;
R⁴ and R⁵ are independently selected from hydrogen, alkyl, alkenyl, alkynyl, cycloalkyl, cycloalkenyl, aralkyl, heterocycloalkyl, heterocycloalkenyl, heteroaryl, heteroaralkyl, -OR¹¹, -O(C=O)R¹¹, -O(C=O)OR¹¹, and-O(C=O)NR¹²R¹³; or R⁴ and R⁵ taken together with the carbon to which they are bonded form (C=O);
each of R⁶, R⁷, R⁸, and R⁹ independently for each occurrence is hydrogen; or R⁶ and R⁷ taken together form a bond; or R⁸ and R⁹ taken together form a bond;
R¹¹ is independently for each occurrence is hydrogen, alkyl, alkenyl, alkynyl, cycloalkyl, cycloalkenyl, aralkyl, heterocycloalkyl, heterocycloalkenyl, heteroaryl, heteroaralkyl; or R¹¹ and R²⁴ taken together with the nitrogen to which they are attached form a 3-8 membered heterocyclic ring;
each of R¹² and R¹³ independently for each occurrence is hydrogen, alkyl, alkenyl, alkynyl, cycloalkyl, cycloalkenyl, aralkyl, heterocycloalkyl, heterocycloalkenyl, heteroaryl, or heteroaralkyl; or R¹² or R¹³ taken together form a 3-8 membered heterocyclic ring; R²⁴ is 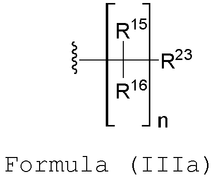 or 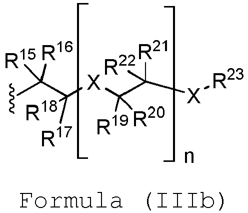
wherein
n is 0-12;
X is -O-, -(C=O)-, -O(C=O)-, -(C=O)O-, -N(R¹¹)-, -(C=O)N(R¹¹)-, -N(R¹¹)(C=O-, -O(O=O)N(R¹¹)-, -N(R¹¹)(C=O)O-, -N(R¹²)(C=O)N(R¹³)-, -NR¹¹SO₂-, or -SO₂NR¹¹-;
each of R¹⁵, R¹⁶, R¹⁷, R¹⁸, R¹⁹, R²⁰, R²¹, and R²² independently for each occurrence is hydrogen, alkyl, alkenyl, alkynyl, cycloalkyl, cycloalkenyl, aralkyl, heteroaryl, heterocycloalkyl, heterocycloalkenyl, halide, cyano, nitro, isocyanate, -R²⁴, -OR¹¹, -SR¹¹, -(C=O)R¹¹, -(C=O)OR¹¹, -O(C=O)R¹¹, -O(C=O)OR¹¹, -(C=O)NR¹²R¹³, -O(C=O)NR¹²R¹³,-NR¹²(C=O)OR¹³, -NR¹²R¹³, -N(R¹²)₂R¹³, -N(R¹²)-NR¹²R¹³,-NR¹²(C=O)R¹³, -(S=O)R¹¹, -SO₂R¹¹, -SO₃R¹¹, -OSO₃R¹¹, -OPO₂OR¹¹, -PO₂OR¹¹, -SO₂NR¹²R¹³, -(C=NR¹²)NR¹²R¹³, -NR¹³(C=NR¹²)R¹¹,-NR¹¹(C=NR¹²)R¹³, or -N(R¹³)(C=NR¹²)NR¹²R¹³; or any two instances of R¹⁵, R¹⁶, R¹⁷, R¹⁸, R¹⁹, R²⁰, R²¹, R²², or R²³ taken together with the carbon to which they are bonded form (C=O); or any two instances of R¹⁵, R¹⁶, R¹⁷, R¹⁸, R¹⁹, R²⁰, R²¹, R²², or R²³ taken together with the carbon to which they are bonded form a 3-8 membered carbocylic or heterocyclic ring; or any two instances of R¹⁵, R¹⁶, R¹⁷, R¹⁸, R¹⁹, R²⁰, R²¹, R²², or R²³ taken together form a bond;
R²³ independently for each occurrence is hydrogen, alkyl, alkenyl, alkynyl, cycloalkyl, cycloalkenyl, aralkyl, heteroaryl, heterocycloalkyl, heterocycloalkenyl, halide, cyano, nitro, isocyanate, -R¹⁴, -OR¹¹, -SR¹¹, -(C=O)R¹¹,-(C=O)OR¹¹, -O(C=O)R¹¹, -O(C=O)OR¹¹, -(C=O)NR¹²R¹³,-O(C=O)NR¹²R¹³, -NR¹²(C=O)OR¹³, -NR¹²R¹³, -N(R¹²)₂R¹³, -N(R¹²)-NR¹²R¹³, -NR¹²(C=O)R¹³, -(S=O)R¹¹, -SO₂R¹¹, -SO₃R¹¹, -OSO₃R¹¹,-OPO₂OR¹¹, -SO₂NR¹²R¹³, -(C=NR¹²)NR¹²R¹³, -NR¹³(C=NR¹²)R¹¹, 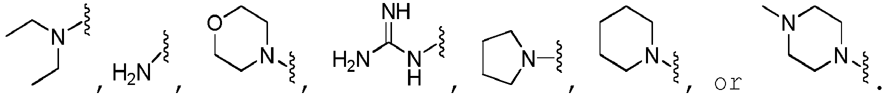
   R¹⁰ may be alkyl.
   R⁴ and R⁵ taken together with the carbon to which they are bonded may form (C=O).
   R⁶ and R⁷ taken together may form a bond and R⁸ and R⁹ taken together may form a bond.
   R²⁴ may be

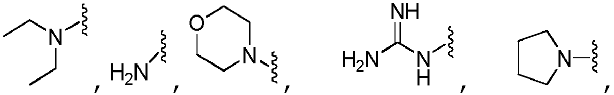

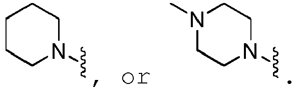

For reference, it is also described herein the compound or salt thereof of the following formula: 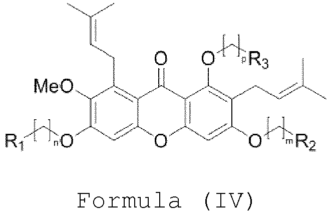 wherein
n, m and p are each independently an integer from 0 to 20; and R₁, R₂ and R₃ are each independently selected from the group consisting of Hydrogen, alkyl, alkenyl, alkynyl, thioalkyl, cycloalkyl, cycloalkenyl, heterocycloalkyl, halo, carboxyl, haloalkyl, haloalkynyl, hydroxyl, alkoxy, thioalkoxy, alkenyloxy, haloalkoxy, haloalkenyloxy, nitro, amino, amino acid, a non-natural amino acid, an oligopeptide, guanidine, morpholino, triazole, nitroalkyl, nitroalkenyl, nitroalkynyl, nitroheterocyclyl, alkylamino, dialkylamino, alkenylamine, alkynylamino, acyl, alkenoyl, alkynoyl, acylamino, diacylamino, acyloxy, alkylsulfonyloxy, heterocycloxy, heterocycloamino, haloheterocycloalkyl, alkylsulfenyl, alkylcarbonyloxy, keto, thioxo, alkylthio, acylthio, aryl, heteroaryl, alkylaryl, alkylheteroaryl, cyano, cyanate, isocyanate,-C(O)NH(alkyl), -C(O)N(alkyl)₂, -COOH, -SO₃H -PO₄H₂, polyethylene glycol and sulphonamide.

For reference it is also described herein a compound selected from the group consisting of: 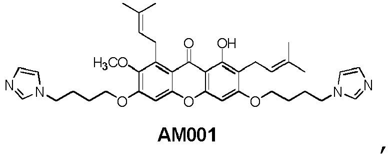 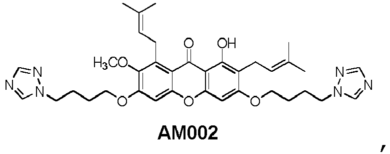 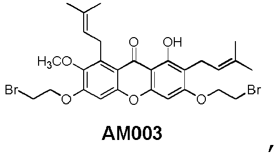 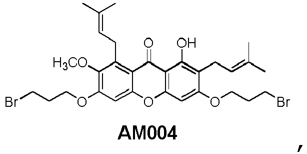 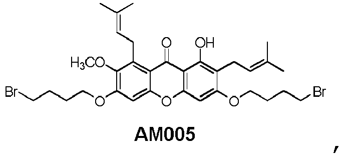 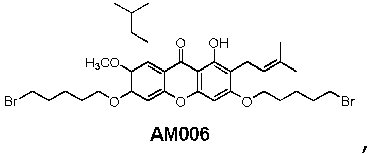 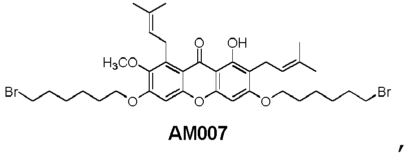 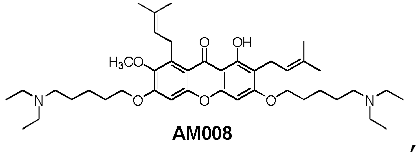 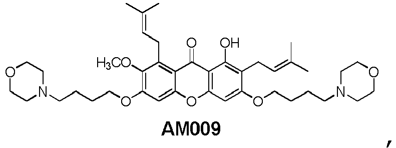 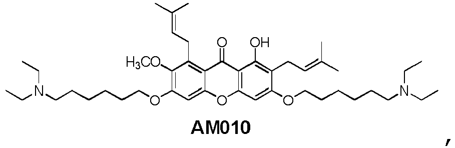 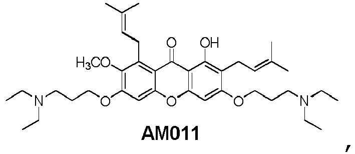 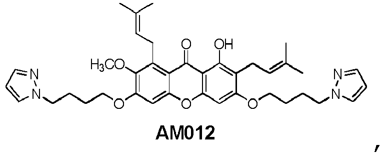 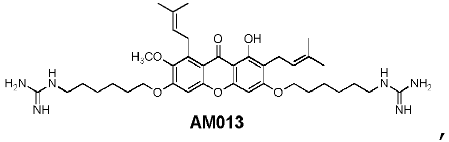 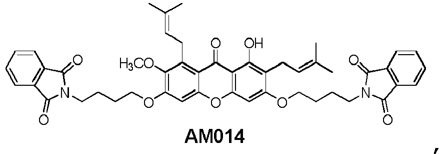 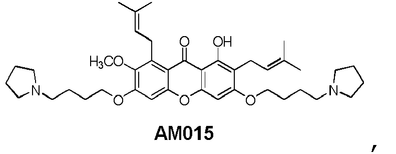 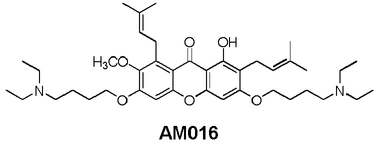 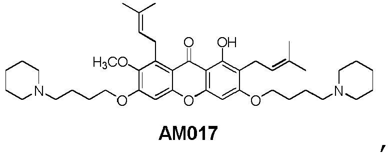 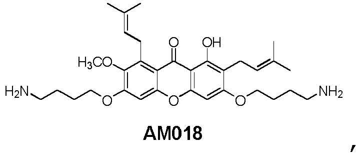 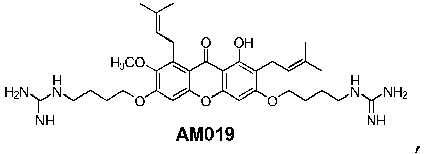 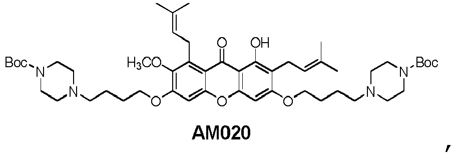 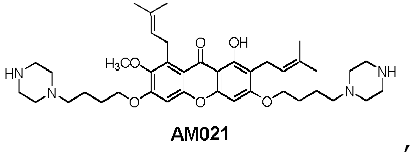 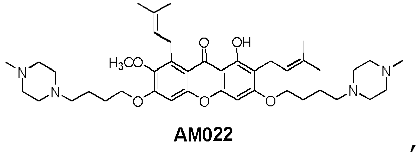 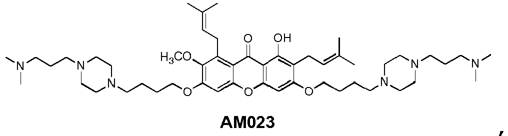 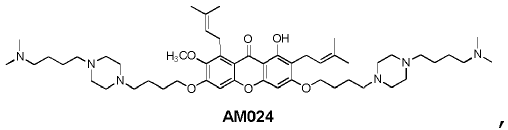 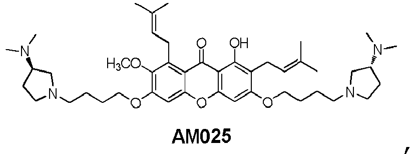 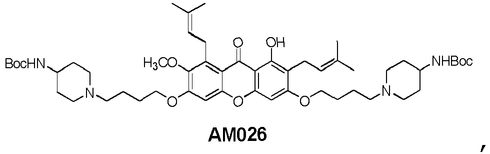 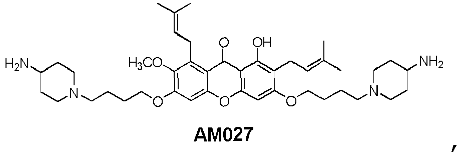 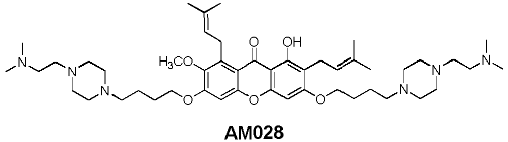 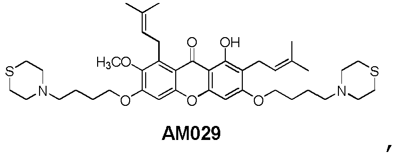 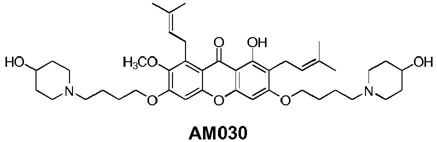 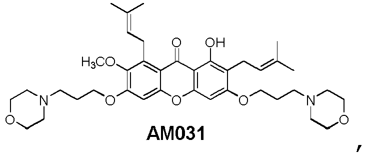 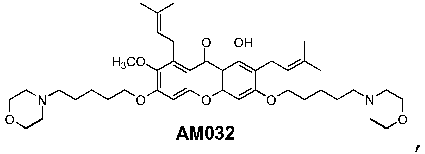 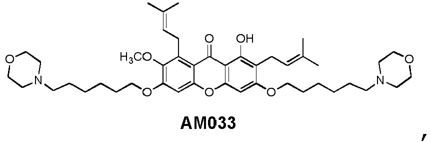 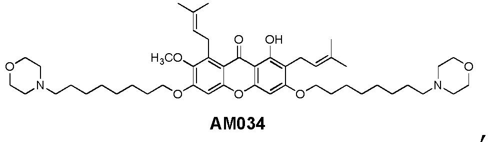 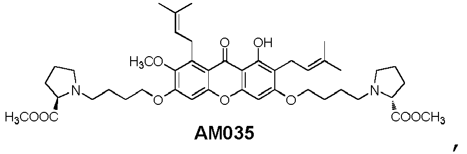 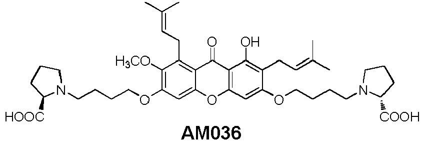 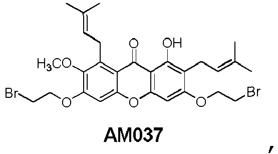 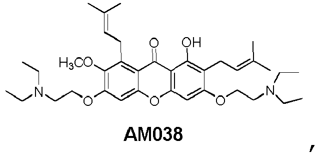 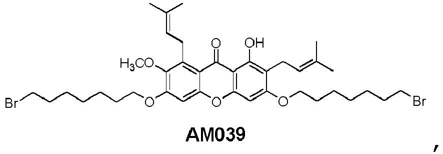 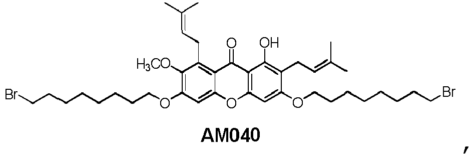 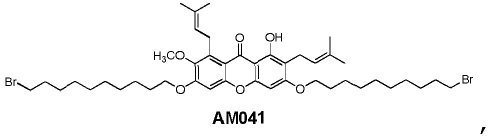 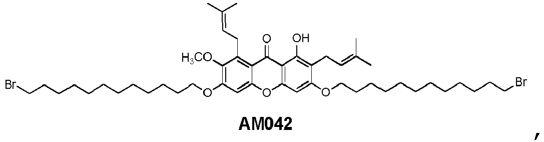 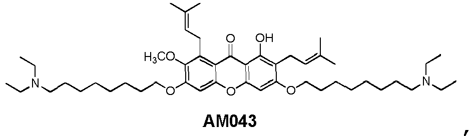 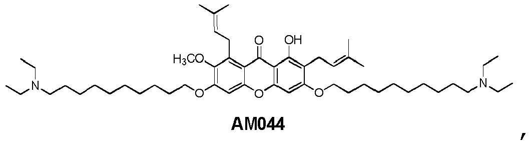 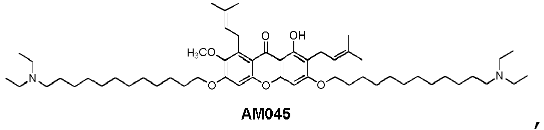 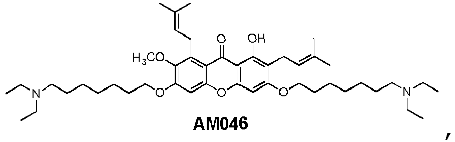 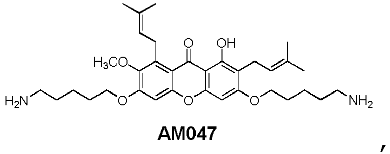 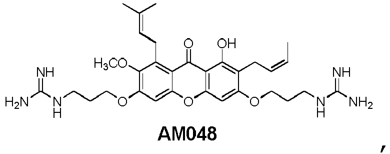 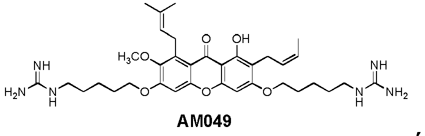 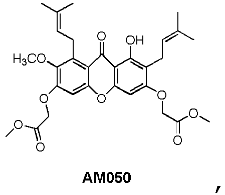 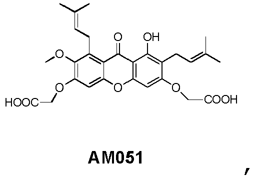 In certain embodiments, the compound is selected from the group consisting of:

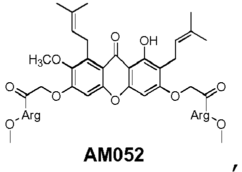

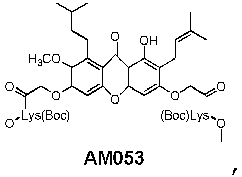

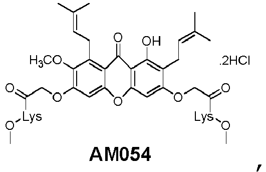

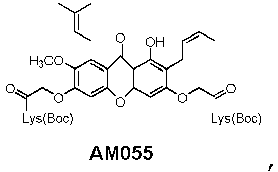

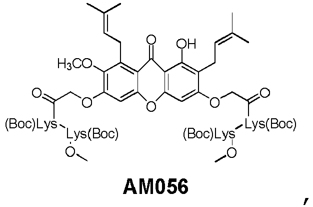

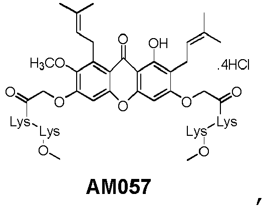

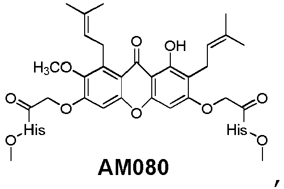

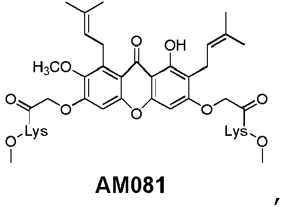

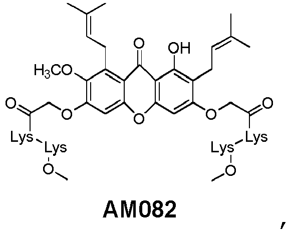

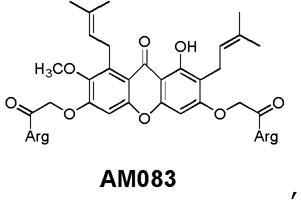

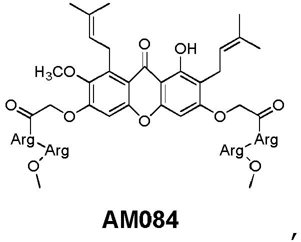

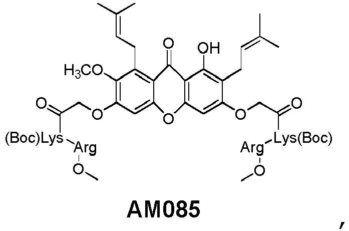

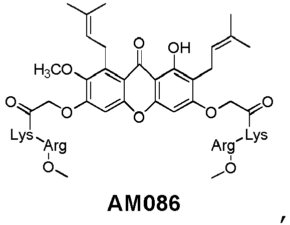

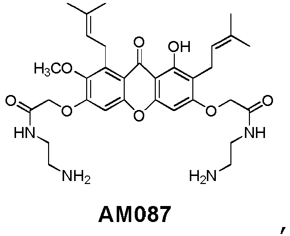

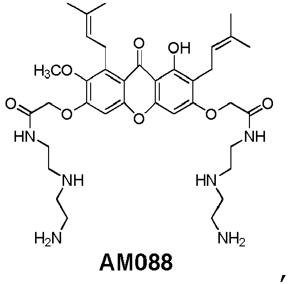

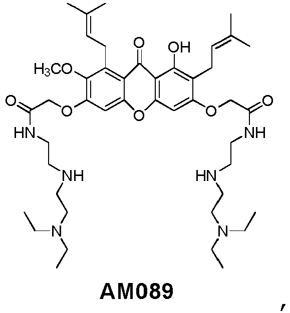

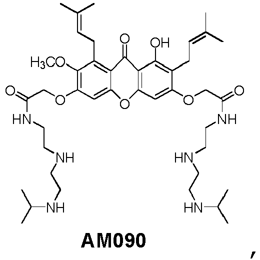

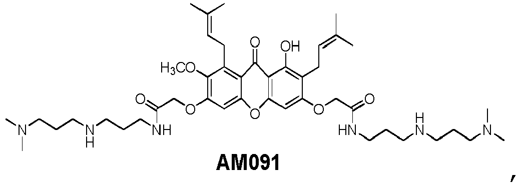

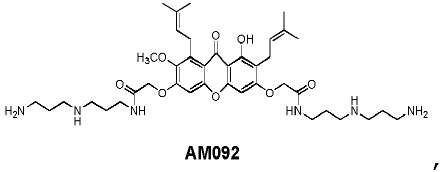

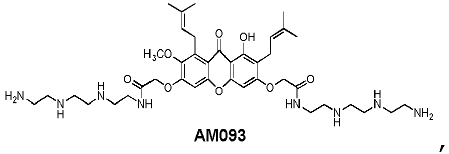

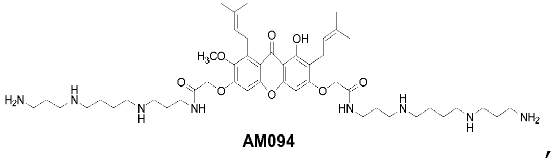

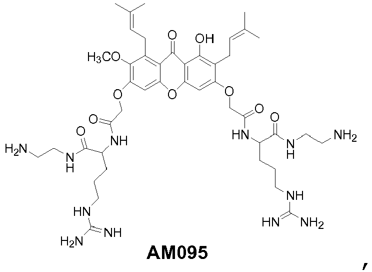

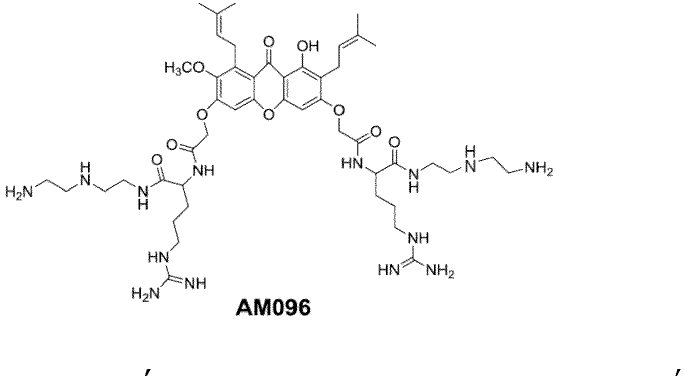

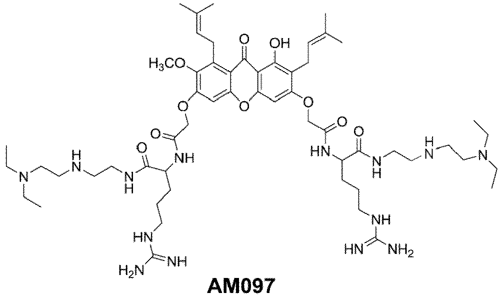

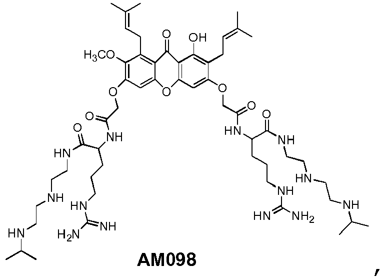

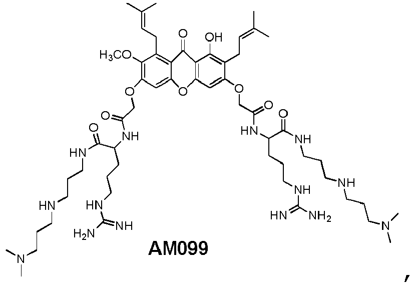

For reference it is also described herein a compound selected from the group consisting of: 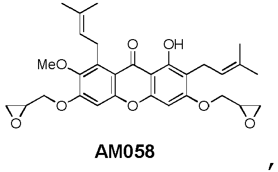 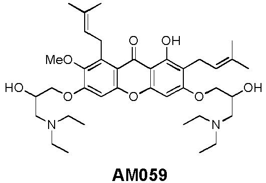 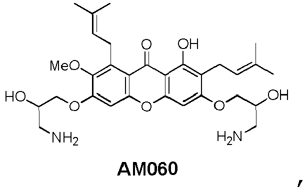 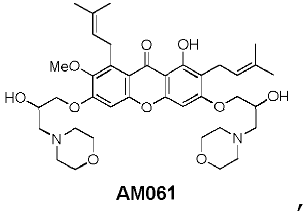 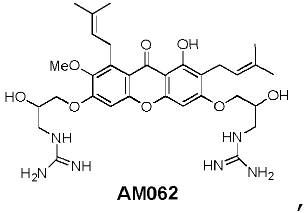 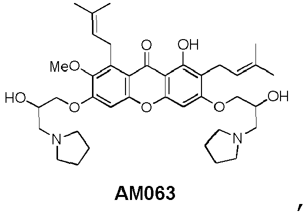 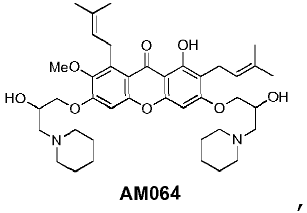 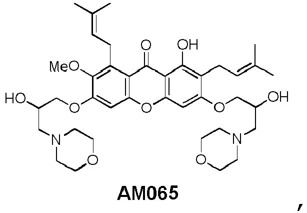 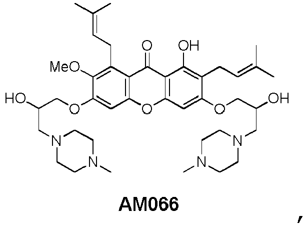 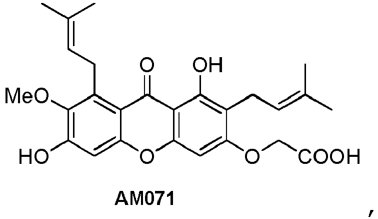 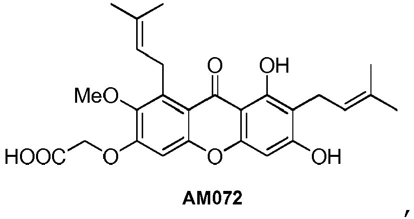 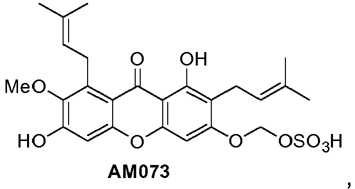 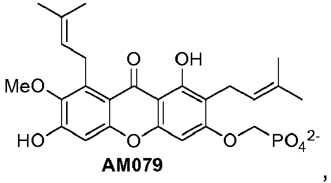 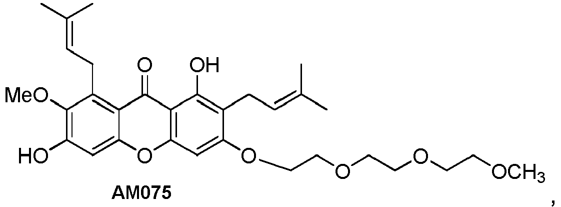 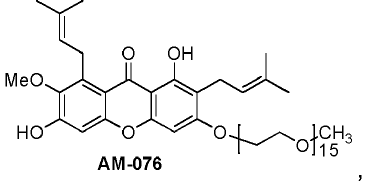 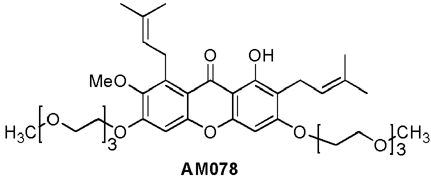 and

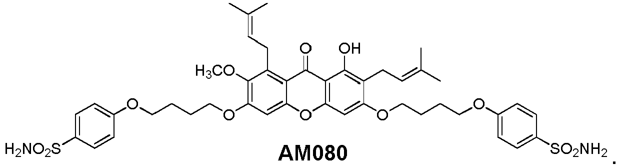

Also described herein for reference is a compound of the formula:

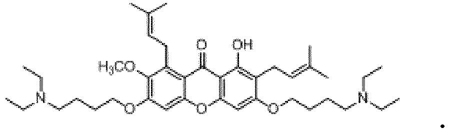

In another embodiment, the compound has the formula:

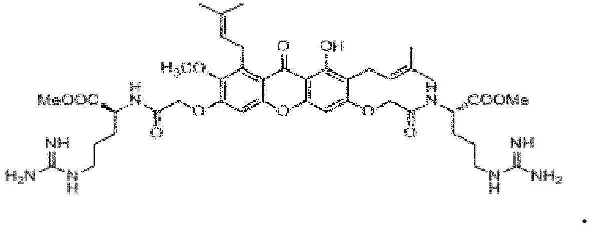

Also described herein for reference is a compound that is a guanidine modified alpha-mangostin or a salt thereof represented by the following structure.

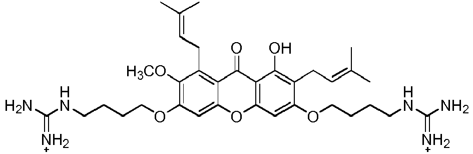

The guanidine modified alpha-mangostin may be prepared according to the following reaction scheme:

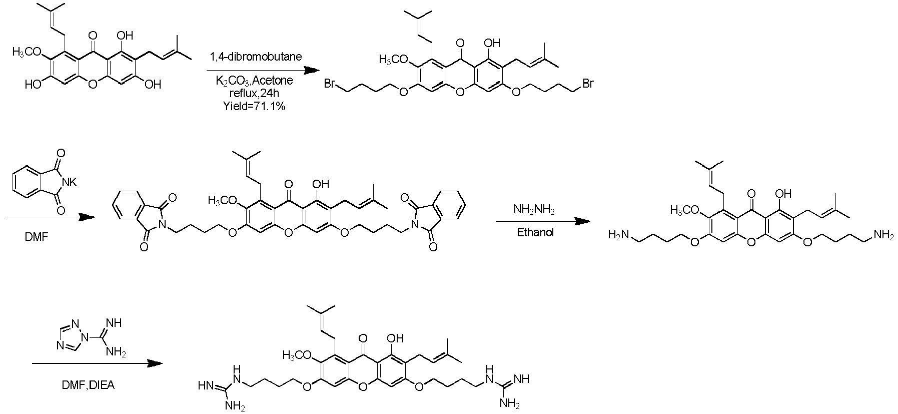

The guanidine modified alpha-mangostin or a salt thereof may be selected from the group consisting of the following:

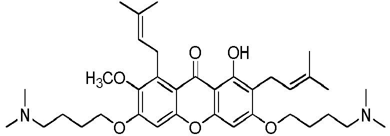

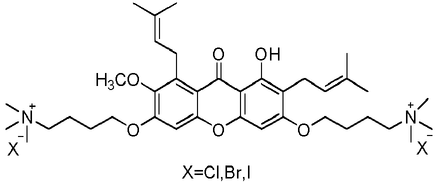

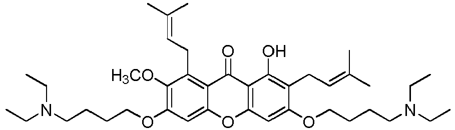

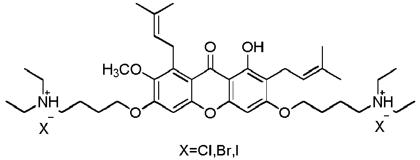

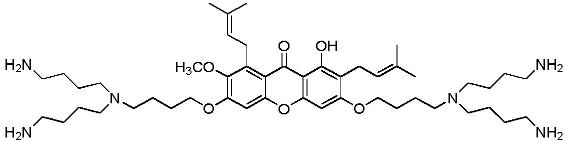

The compound as described herein may be a saturated or unsaturated nitrogen heterocycle modified alpha-mangostin compound.

The saturated or unsaturated nitrogen heterocycle compound may be a morpholine modified alpha-mangostin compound as follows:

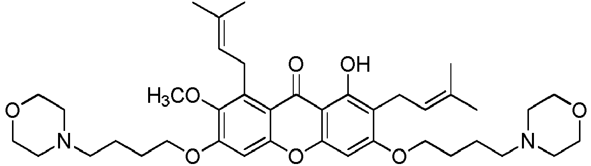

The saturated or unsaturated nitrogen heterocycle compound may be a 1,2,4-triazole modified alpha-mangostin compound as follows:

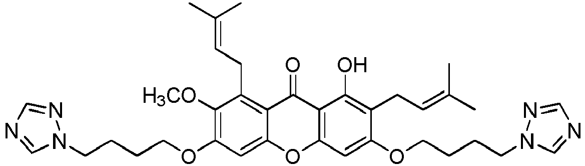

The saturated or unsaturated nitrogen heterocycle compound may be a pyrrolidine modified alpha-mangostin compound as follows:

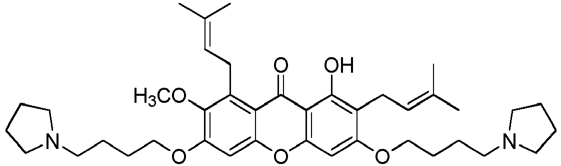

The compound may be modified with one or more anionic functional groups, for example mono, di, tri or more substituted.

The modifications may also include different lengths of hydrophobic and hydrophilic linkers.

The compound as described herein may be modified at one or more of A, B, C, D, E, F, as shown in the following structure:

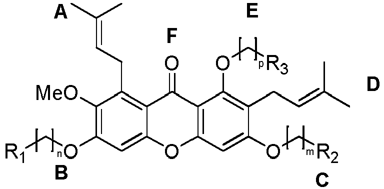

Suitable anionic functional groups may include but are not limited to triazoles, -CO₂H, -SO₃H -OSO₃H -PO₃H₂, and -OPO₃H₂.

For reference it is described hereina compound selected from the group consisting of: 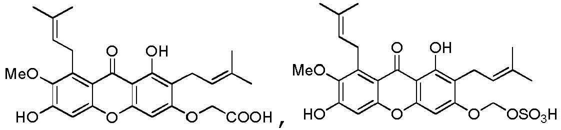 and

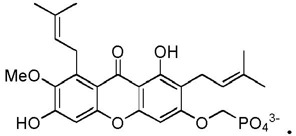

For reference it is also described herein a compound selected from the group consisting of: 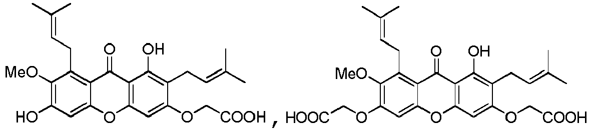 and

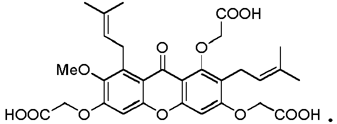

For reference, it is also described herein a compound with the following structure: 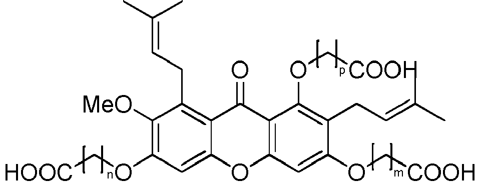 wherein n, m and p are each independently selected from an integer from 0 - 20.

The compound may have the following structure: 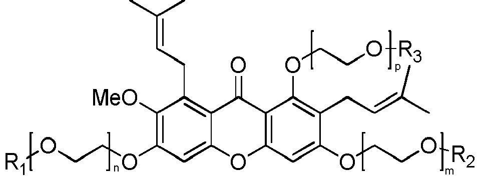 wherein n, m and p are each independently selected from an integer from 0 - 20.

The compound may be a neutral modified alpha-mangostin. Suitable neutral modifications may include but are not limited to halogen, alkyl, sulfonamide or polyethylene glycols (PEG).The compound may have the following structure: 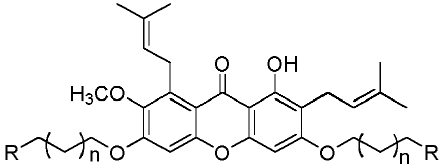 wherein:
n is an integer of from 0-20
R is as previously defined for R₁, R₂ and R₃ described herein.

The compound may be a bromo-modified alpha-mangostin having the following structure:

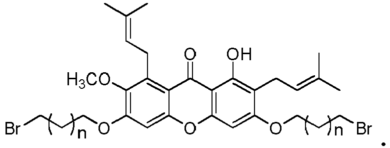

The bromo-modified alpha-mangostin may be synthesised by the following reaction scheme:

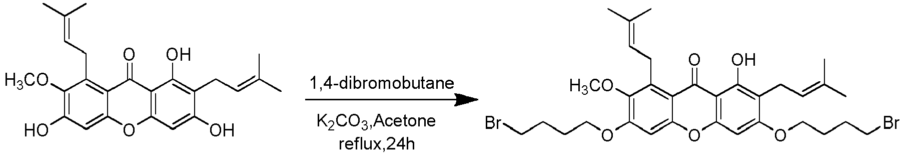

The compound as described herein for reference may have the following structure: 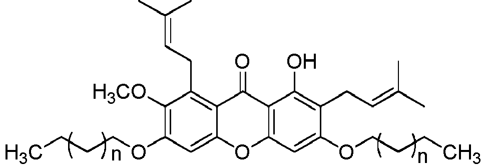 wherein, n is an integer from 2-20.

For reference a compound as described herein may have the following structure: 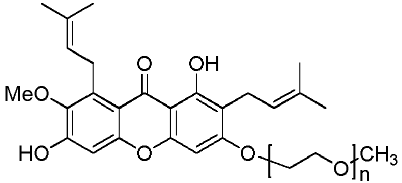 For reference the compound as described herein may have the following structure:

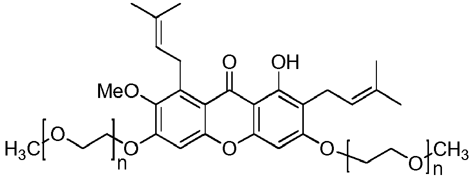

For reference there is also described herein a compound that is a sulfonamide modified alpha-mangostin. The sulfonamide modified alpha-mangostin may have the following structure:

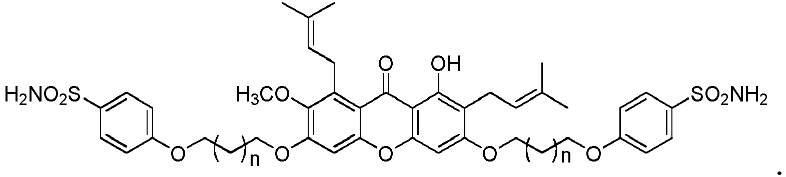

The sulfonamide modified alpha-mangostin may be synthesised by the following reaction scheme:

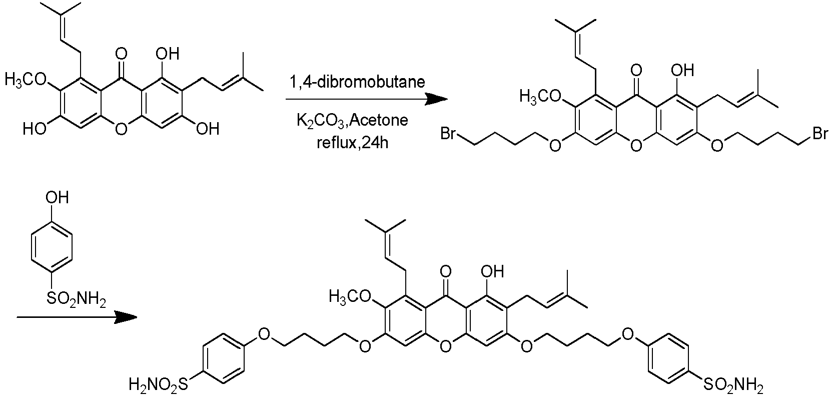

Also described herein for reference is an alpha-mangostin peptoid or salt thereof. The alpha-mangostin molecule may be modified with one or more natural (coded) or unnatural (uncoded) amino acids. Advantageously this modification may impart both a peptide-like structure and peptide-like properties to the alpha-mangostin molecule.

The alpha-mangostin peptoid may have the following general structure: 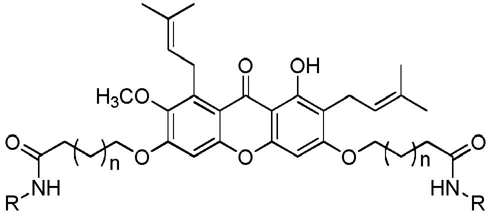
wherein n is an integer of from 0-20;
R may be a basic amino acid group, an acidic amino acid group, a neutral amino acid group, an unnatural amino group or an oligopeptide.

The natural amino acid may be arginine, lysine or histidine.The acidic amino acid may be aspartic acid or glutamic acid. The neutral amino acid may comprise a nucleophilic, hydrophobic, aromatic or amide group. For example, glycine, alanine, serine, threonine, cysteine, valine, leucine, isoleucine, methionine, proline, phenylalanine, tyrosine, tryptophan, asparagine or glutamine. The unnatural amino acid may be ornithine. The oligopeptide may comprise 2-8 amino acid residues.

The alpha mangostin peptoid may be synthesised according to the following reaction scheme:

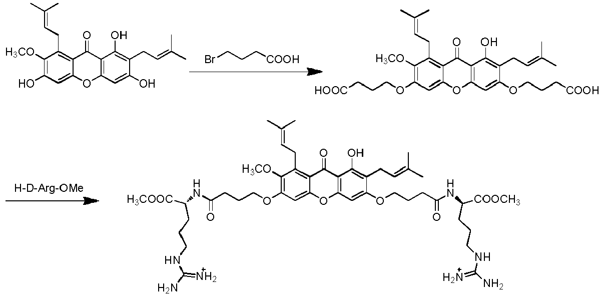

The alpha-mangostin peptoid may have the following structure:

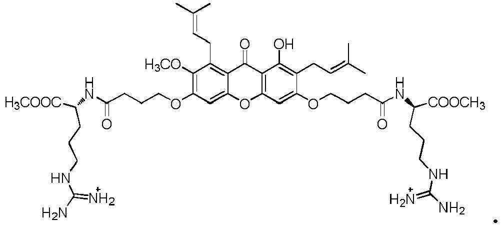

The alpha-mangostin peptoid may have the following structure:

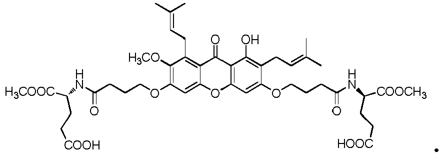

The alpha-mangostin peptoid may have the following structure:

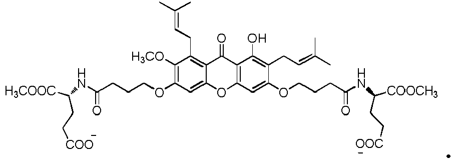

The alpha-mangostin peptoid may have the following structure:

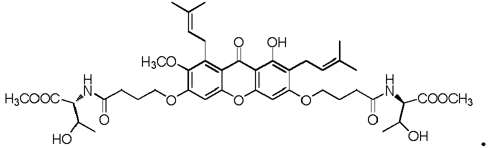

The alpha-mangostin peptoid may have the following structure:

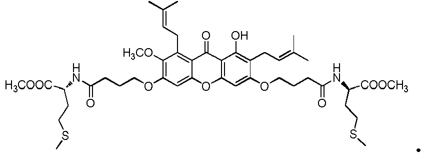

The alpha-mangostin peptoid may have the following structure:

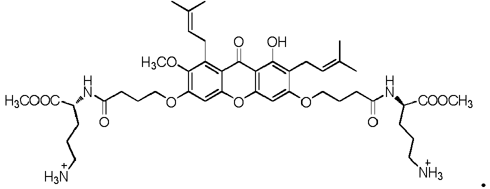

The alpha-mangostin peptoid may have the following structure:

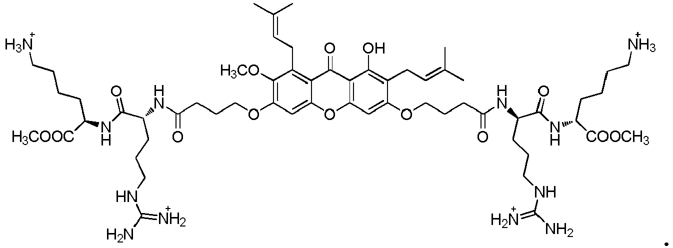

The alpha-mangostin peptoid may have the following structure:

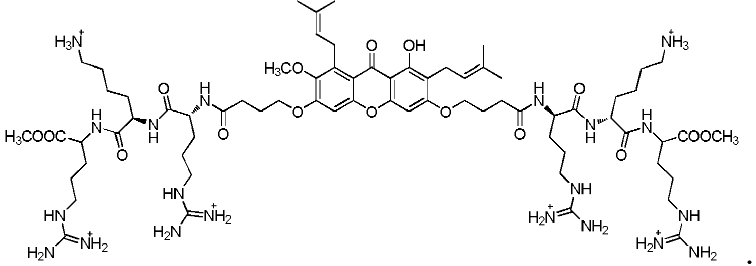

In another embodiment there is provided a composition comprising a compound as described herein, together with a carrier.

In another embodiment there is provided a pharmaceutical composition comprising a compound as described herein, together with a pharmaceutically acceptable carrier.

In another embodiment there is provided a compound or composition as described herein, for use as a medicament.

In accordance with the present disclosure, when used for the treatment or prevention of a microbial infection, compound(s) of the disclosure may be administered alone. Alternatively, the compounds may be administered as a pharmaceutical, veterinarial, agricultural, or industrial formulation which comprises at least one compound according to the disclosure. The compound (s) may also be present as suitable salts, including pharmaceutically acceptable salts.

In accordance with the present disclosure, the compounds of the disclosure may be used in combination with other known treatments or antimicrobial agents, including antifungal treatments, antibiotics, disinfectants, and the like. Suitable agents are listed, for example, in the Merck Index, An Encyclopoedia of Chemicals, Drugs and Biologicals, 12th Ed.,1996.

Combinations of active agents, including compounds of the disclosure, may be synergistic.

The administration of the compound or composition as described herein with another active agent may be done simultaneously, sequentially or separately.

Suitable pharmaceutically acceptable salts of compounds according to the present disclosure may be prepared by mixing a pharmaceutically acceptable acid such as hydrochloric acid, sulfuric acid, methanesulfonic acid, succinic acid, fumaric acid, maleic acid, benzoic acid, phosphoric acid, acetic acid, oxalic acid, carbonic acid, tartaric acid, or citric acid with the compounds of the invention. Suitable pharmaceutically acceptable salts of the compounds of the present invention therefore include acid addition salts.

Representative acid addition salts include acetate, adipate, alginate, ascorbate, asparate, benzenesulfonate, benzoate, bisulfate, borate, butyrate, camphorate, camphorsulfonate, citrate, digluconate, cyclopentanepropionate, dodecylsulfate, ethanesulfonate, fumarate, glucoheptonate, glycerophosphate, hemisulfate, heptonate, hexanoate, hydrobromide, hydrochloride, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, toluenesulfonate, undecanoate, valerate salts, and the like. Representative alkali or alkaline earth metal salts include sodium, lithium potassium, calcium, magnesium, and the like, as well as non-toxic ammonium, quaternary ammonium, and amine cations, including, but not limited to ammonium, tetramethylammonium, tetraethylammonium, methylamine, dimethylamine, trimethylamine, triethylamine, ethylamine, triethanolamine and the like.

Convenient modes of administration include injection (subcutaneous, intravenous, and the like), oral administration, inhalation, transdermal application, topical creams or gels or powders or eyedrops or topical cosmetic products, or rectal administration.

Depending on the route of administration, the formulation and/or compound may be coated with a material to protect the compound from the action of enzymes, acids and other natural conditions which may inactivate the therapeutic activity of the compound. The compound may also be administered parenterally or intraperitoneally.

Dispersions of the compounds according to the invention may also be prepared in water (as an aqueous dispersion), glycerol, liquid polyethylene glycols, and mixtures thereof and in oils. Under ordinary conditions of storage and use, pharmaceutical preparations may contain a preservative to prevent the growth of microorganisms.

Pharmaceutical compositions suitable for injection include sterile aqueous solutions (where water soluble) or dispersions and sterile powders for the extemporaneous preparation of sterile injectable solutions or dispersions. Ideally, the composition is stable under the conditions of manufacture and storage and may include a preservative to stabilise the composition against the contaminating action of microorganisms such as bacteria and fungi.

In one embodiment of the disclosure, the compound(s) of the disclosure may be administered orally, for example, with an inert diluent or an assimilable edible carrier. The compound(s) and other ingredients may also be enclosed in a hard or soft shell gelatin capsule, compressed into tablets, or incorporated directly into an individual's diet. For oral therapeutic administration, the compound(s) may be incorporated with excipients and used in the form of ingestible tablets, buccal tablets, troches, capsules, elixirs, suspensions, syrups, wafers, and the like.

Suitably, such compositions and preparations may contain at least 1% by weight of active compound. The percentage of the compound(s) of formula (I) in pharmaceutical compositions and preparations may, of course, be varied and, for example, may conveniently range from about 2% to about 90%, about 5% to about 80%, about 10% to about 75%, about 15% to about 65%; about 20% to about 60%, about 25% to about 50%, about 30% to about 45%, or about 35% to about 45%, of the weight of the dosage unit. The amount of compound in therapeutically useful compositions is such that a suitable dosage will be obtained.

Supplementary active compounds may also be incorporated into the compositions according to the present invention. It is especially advantageous to formulate parenteral compositions in dosage unit form for ease of administration and uniformity of dosage.

In one embodiment, the carrier may be an orally administrable carrier.

Another form of a pharmaceutical composition is a dosage form formulated as enterically coated granules, tablets or capsules suitable for oral administration.

Also included in the scope of this invention are delayed release formulations.

Compounds of the invention may also be administered in the form of a "prodrug". A prodrug is an inactive form of a compound which is transformed *in vivo* to the active form. Suitable prodrugs include esters, phosphonate esters etc, of the active form of the compound.

In one embodiment, the compound may be administered by injection. In the case of injectable solutions, the carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. Prevention of the action of microorganisms can be achieved by including various anti-bacterial and/or anti-fungal agents. Suitable agents are well known to those skilled in the art and include, for example, parabens, chlorobutanol, phenol, benzyl alcohol, ascorbic acid, thimerosal, and the like. In many cases, it may be preferable to include isotonic agents, for example, sugars, polyalcohols such as mannitol, sorbitol, sodium chloride in the composition. Prolonged absorption of the injectable compositions can be brought about by including in the composition an agent which delays absorption, for example, aluminium monostearate and gelatin.

Sterile injectable solutions can be prepared by incorporating the compound/composition in the required amount in an appropriate solvent with one or a combination of ingredients enumerated above, as required, followed by filtered sterilisation. Generally, dispersions are prepared by incorporating the compound/composition into a sterile vehicle which contains a basic dispersion medium and the required other ingredients from those enumerated above.

Tablets, troches, pills, capsules and the like can also contain the following: a binder such as gum gragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin or a flavouring agent such as peppermint, oil of wintergreen, or cherry flavouring. When the dosage unit form is a capsule, it can contain, in addition to materials of the above type, a liquid carrier. Various other materials can be present as coatings or to otherwise modify the physical form of the dosage unit. For instance, tablets, pills, or capsules can be coated with shellac, sugar or both. A syrup or elixir can contain the analogue, sucrose as a sweetening agent, methyl and propylparabens as preservatives, a dye and flavouring such as cherry or orange flavour. Of course, any material used in preparing any dosage unit form should be pharmaceutically pure and substantially non-toxic in the amounts employed. In addition, the analogue can be incorporated into sustained-release preparations and formulations.

Preferably, the pharmaceutical composition may further include a suitable buffer to minimise acid hydrolysis. Suitable buffer agent agents are well known to those skilled in the art and include, but are not limited to, phosphates, citrates, carbonates and mixtures thereof.

Single or multiple administrations of the pharmaceutical compositions according to the invention may be carried out. One skilled in the art would be able, by routine experimentation, to determine effective, non-toxic dosage levels of the compound and/or composition of the invention and an administration pattern which would be suitable for treating the diseases and/or infections to which the compounds and compositions are applicable.

Further, it will be apparent to one of ordinary skill in the art that the optimal course of treatment, such as the number of doses of the compound or composition of the invention given per day for a defined number of days, can be ascertained using convention course of treatment determination tests.

Generally, an effective dosage per 24 hours may be in the range of about 0.0001 mg to about 1000 mg per kg body weight; suitably, about 0.001 mg to about 750 mg per kg body weight; about 0.01 mg to about 500 mg per kg body weight; about 0.1 mg to about 500 mg per kg body weight; about 0.1 mg to about 250 mg per kg body weight; or about 1.0 mg to about 250 mg per kg body weight. More suitably, an effective dosage per 24 hours may be in the range of about 1.0 mg to about 200 mg per kg body weight; about 1.0 mg to about 100 mg per kg body weight; about 1.0 mg to about 50 mg per kg body weight; about 1.0 mg to about 25 mg per kg body weight; about 5.0 mg to about 50 mg per kg body weight; about 5.0 mg to about 20 mg per kg body weight; or about 5.0 mg to about 15 mg per kg body weight.

Alternatively, an effective dosage may be up to about 500 mg/m². For example, generally, an effective dosage is expected to be in the range of about 25 to about 500 mg/m², about 25 to about 350 mg/m², about 25 to about 300 mg/m², about 25 to about 250 mg/m², about 50 to about 250 mg/m², and about 75 to about 150 mg/m².

In one embodiment, the compounds as described herein may have a minimum inhibitory concentration selected from the group consisting of about 0.0001 to about 1000 µg/ml; about 0.001 to about 500 µg/ml; about 0.001 to about 100 µg/ml; about 0.001 to about 50 µg/ml; about 0.01 to about 10 µg/ml about 0.04 to about 5 µg/ml; and about 0.04 to about 1 µg/ml.

In one embodiment, the compounds and compositions as described herein may also be used to protect medical devices from microbial contamination. Such antimicrobial protect may be achieved by immobilizing, for example covalently, the compound or composition to a surface of a medical device, for example a catheter or a medical device implant. Alternatively, the compound or composition could be impregnated in a non-metal medical device. Impregnation may advantageously provide continued protection for the device as the surface wears down over time or through use.

In one embodiment, the compounds and compositions as described herein may be applied to a surface as a disinfectant. They may be applied as a solution or in an aerosol.

In another embodiment, a wet wipe in the form of an absorbent sheet comprising a formulation of the compound or composition as described herein may be provided. The wet wipe may be used to disinfect surfaces, including hospital, industrial or domestic surfaces such a work tops or a patient's skin prior to, or after, an invasive or other surgical procedure.

In another embodiment there is provided a kit comprising a compound or composition as described herein and directions for use.

In another embodiment, there is provided a kit comprising the pharmaceutical dosage form as described herein and directions for use.

In another embodiment there is provided the use of a compound or composition as described herein, in the preparation or manufacture of a medicament for the treatment of a microbial infection.

In another embodiment, there is provided a compound as disclosed herein for use in a method for treating a microbial infection comprising the step of administering an effective amount of a compound or composition as described herein to a patient in need of such treatment.

In one embodiment, the microbial infection may be caused by a bacterial infection caused by a Gram positive or Gram negative bacteria. In another embodiment, the microbial infection may be caused by Mycobacteria or a fungus.

In one embodiment the Gram negative bacteria may be Pseudomonas spp.

In one embodiment the Gram positive bacteria may be selected from the group consisting of *Streptococcus spp., Staphylococcus spp., Bacillus spp., Carynebacterium spp., Clostridium spp., Listeria spp.,* and *Enterococcus spp.*

In one embodiment, the *Staphylococcus spp. is Staphylococcus aureus.* In another embodiment, the *Staphylococcus spp.* is Methicillin resistant *Staphylococcus aureus* (MRSA).

It is an advantage of the compounds as described herein that they are able to eliminate 99.9% MRSA in a sample in 10-20 minutes. It is a further advantage of the compounds as described herein that they can be used both *in vitro* and *in vivo* with minimal cytotoxicity.

In one embodiment, the *Streptococcus spp.* is *Streptococcus epidermis* or *Streptococcus faecium.*

In one embodiment, the *Bacillus spp.* is *Bacillus cereus.*

In one embodiment, the *Carynebacterium spp.* is *Carynebacterium diptheriae.*

In one embodiment, the *Clostridium spp.* is selected from the group consisting of *Clostridium botulinum, Clostridium difficile, Clostridium perfringens, Clostridium tetani and Clostridium sordellii.*

In one embodiment, the *Bacillus spp.* is *Bacillus cereus.*

In one embodiment, the *Listeria spp.* is *Listeria monocytogenes.*

In one embodiment, the *Bacillus spp.* is *Bacillus cereus.*

In one embodiment, the Enterococcus spp. is *Enterococcus faecalis.*

In another embodiment, the microbial infection is a fungal infection. The fungal infection is caused by *Cryptococcus gattii, Cryptococcus neoformans, Tinea pedis, Tinea cruris, Tinea corpora, Tinea faciei, Tinea capitis, Tinea manuum, Tinea unguium, Tinia versicolor, Candida spp., Sporothrix schenckii* and *Aspergillus spp.*

In one embodiment, the microbial disease is caused by including *Mycobacterium tuberculosis, Mycobacterium bovis* and *Mycobacterium leprae.*

In one embodiment, the compound or composition as described herein is to be administered with one or more further active agents. Combinations of active agents, including compounds and compositions as described herein, may be synergistic.

The administration of the medicament as described herein with the one or more further active agents may be done simultaneously, sequentially or separately.

### Brief Description of Drawings

The accompanying drawings illustrate a disclosed embodiment and serves to explain the principles of the disclosed embodiment. It is to be understood, however, that the drawings are designed for purposes of illustration only, and not as a definition of the limits of the invention.
Fig. 1 shows a kill curve for MRSA strain DM21455 exposed to compound AM-016 as described herein.
Fig. 2 shows an absorbance curve of varying concentrations of AM-016 in a saline solution.
Fig. 3 shows an absorbance curve of varying concentrations of AM-016 in water.
Fig. 4 shows the results of an in vivo cytotoxicity assay on a rabbit eye.
Fig. 5 is shows the results of a comparative test for cytotoxicity between AM-016 and vancomycin in a rabbit corneal wound healing model.
Fig. 6 graphically illustrates the percentage wound closing in a rabbit corneal wound healing model over a 3 day period using vancomycin, AM-016 or saline solution.
Fig. 7 graphically illustrates a drug resistance curve for vancomycin and AM-016.
Fig 8. is a plot MICs (µg/mL) of AM016 against E. *faecalis* ATCC29212, *S. aureus* DM21455 (MRSA), vancomycin intermediate-resistance *S. aureus* (VISA) and *S. aureus* ATCC 700699.
Fig. 9 shows the results of a depolarization study of AM-016 with Clinical *Staphylococcus aureus* DM4001 (source: Eye) .
Fig. 10 shows the results of a depolarization study of AM-005 with Clinical *Staphylococcus aureus* DM4001 (source: Eye).
Fig 11 shows the effect of membrane permeabilization induced by compounds described herein using SYTOX green assay.
Fig. 12 shows the results of observable SYTOX green fluorescence emission induced by compounds described herein against Gram-negative bacteria (*E. coli* ATCC8739).
Fig. 13 shows the effect of membrane permeabilization of compounds described herein against Gram-positive and Gram-negative bacteria.
Fig. 14 shows fluorescence microscopy images demonstrating that active compounds as described herein induce a significant amount of SYTOX green stained *S*. *aureus* (fluoresces in green).
Fig 15 shows the results of an extracellular ATP leakage assay.
Fig. 16 shows the antimicrobial activity of AM-016, vancomycin and daptomycin against strain MRSA-21455.
Fig. 17 shows show the antimicrobial activity of AM-016, vancomycin and daptomycin against strain MRSA-09080R.
Fig. 18 shows show the antimicrobial activity of AM-016, vancomycin and daptomycin against strain MRSA-42412
Fig. 19 shows show the antimicrobial activity of AM-016, vancomycin and daptomycin against strain MRSA-21595.
Fig. 20 shows show the antimicrobial activity of AM-016, vancomycin and daptomycin against strain MRSA-700699.

### Examples

Non-limiting examples of the invention, including the best mode, and a comparative example will be further described in greater detail by reference to specific Examples, which should not be construed as in any way limiting the scope of the invention which is defined by the appended set of claims. The Example section also includes compounds that are for reference only.

### Examples

### Example 1 - Synthesis of Alpha Mangostin Derivatives

The hydroxyl groups of alpha-mangostin at C3 and C6 position were modified to mimic an antimicrobial peptide structure, which consists of a hydrophobic core and cationic side groups. The general modification strategy is described below.

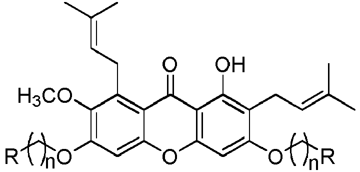

The above structure illustrates the general structure of the synthesized compounds described herein. Functional groups were introduced to the 3,6- position of alpha mangostin to mimic the antimicrobial peptides. "n" refers to the length of link space, from 1 to 20. The R- moieties are selected from different functional group, such as halogen, aliphatic amines, aromatic amines, amino acid, guanidine and the like. The alpha-mangostin based synthetic antibiotics were classified into three types by the properties of the R- functional groups, including cationic modification, neutral modification and anionic modification.

### 1.1 Cationic modification of alpha-mangostin

### Synthesis of cationic alpha-mangostin derivatives by dibromo-substituted approach.

We first synthesized different length spacers of ω-bromoalkyl substituted alpha mangostin, the length of the spacer was from 2-20. The intermediates were conjugated with three types of amines to obtain cationic modification of the alpha-magostin derivatives. These three types of amines including linear aliphatic amines such as ethanamine, propan-1-amine, dimethylamine, diethyl amine, dipropylamine, diisopropylamine, triethylamine, trimethylamine, tripropylamine, n-propylpentan-1-amine, butan-1-amine; cyclic aliphatic amines such as thiazolidine, isoxazolidine, oxazolidine, pyrrolidine, morpholine, piperazine, thiomorpholine, 1-methylimidazolidine; and aromatic amines such as 1,2,4-triazole, 1H-imidazole, pyrazole, pyridine, pyridazine, pyrimidine, 1H-1,2,3-triazole and the like (see Scheme 1-1)

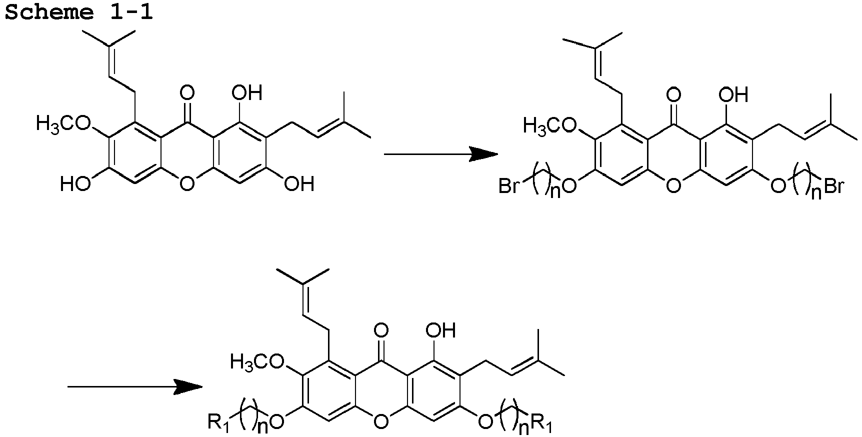

### Scheme 1-1: The general synthesis route of cationic alpha-mangostin derivatives by dibromo-substituted approach.

Specific examples of this type of analogue are illustrated as below:

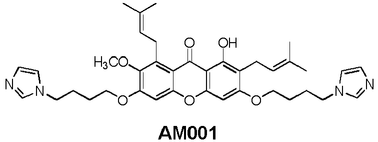

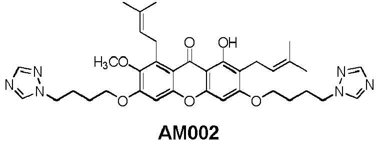

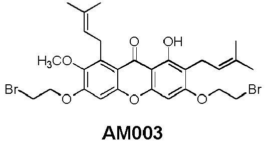

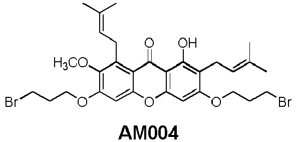

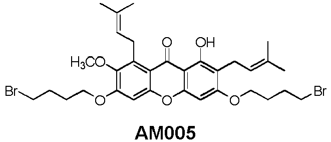

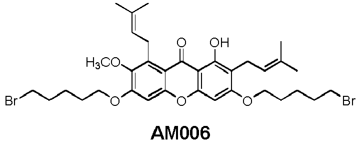

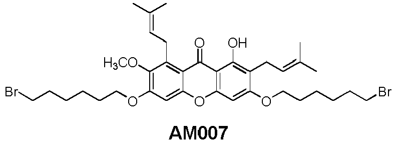

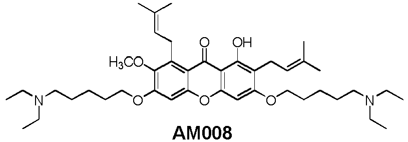

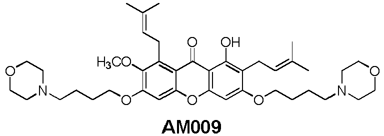

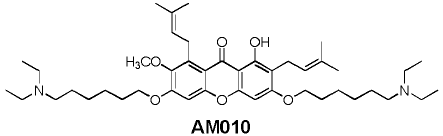

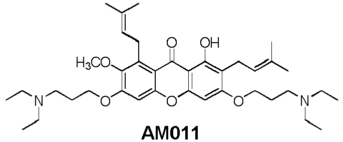

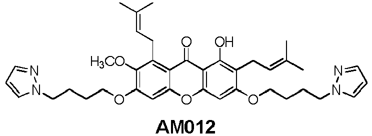

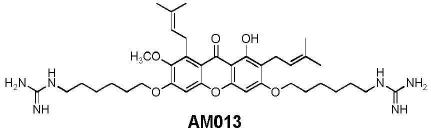

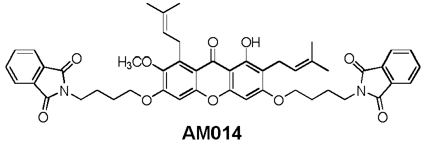

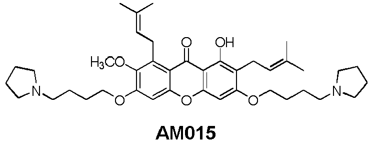

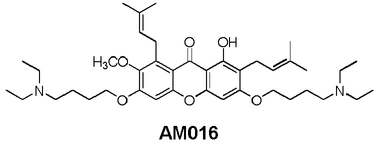

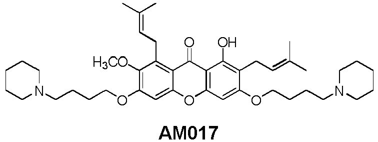

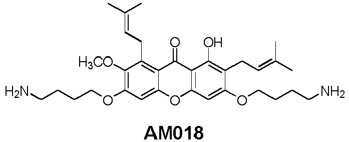

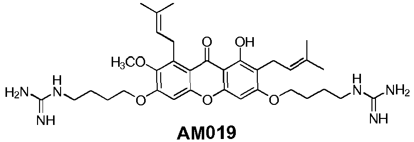

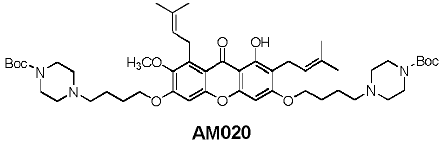

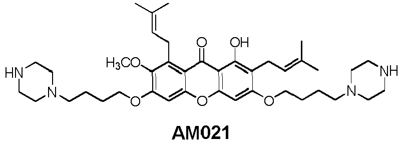

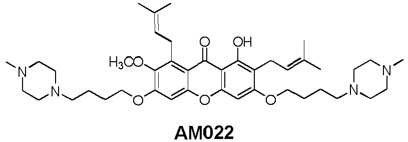

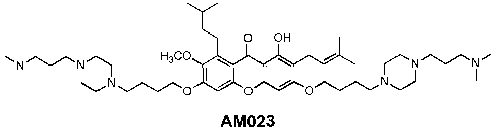

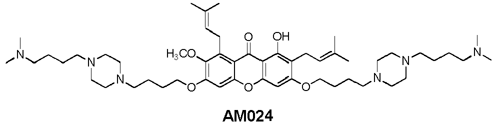

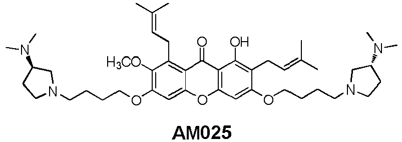

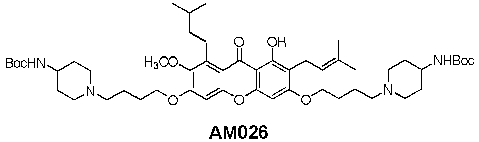

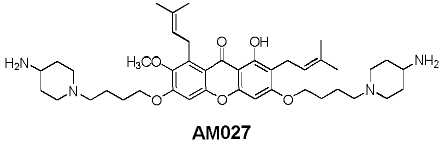

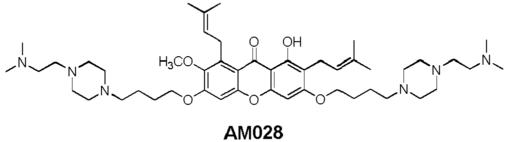

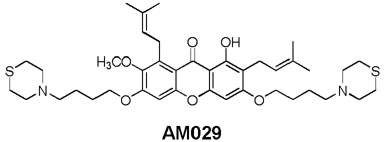

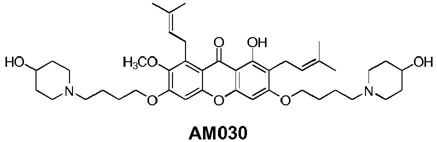

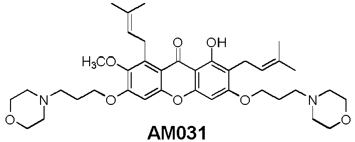

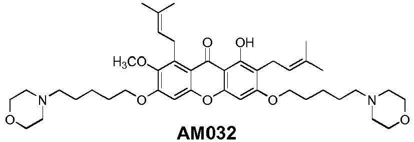

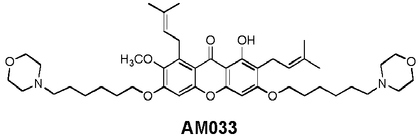

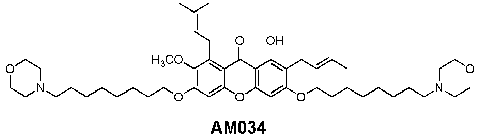

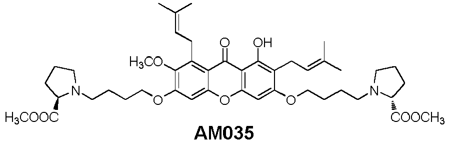

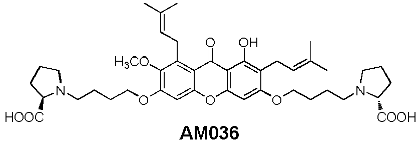

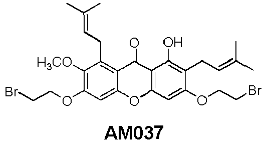

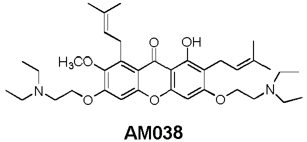

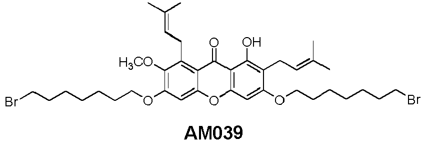

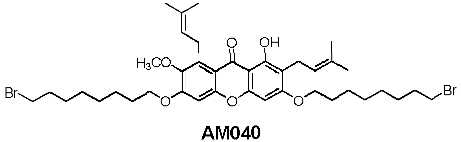

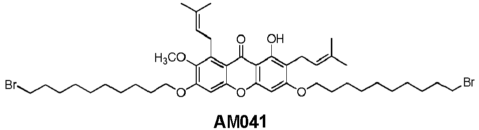

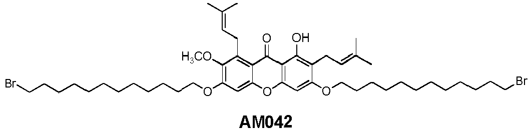

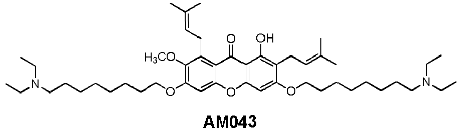

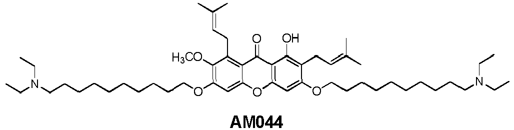

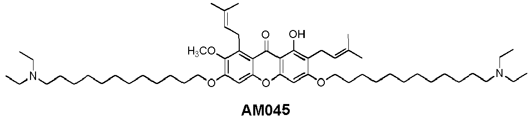

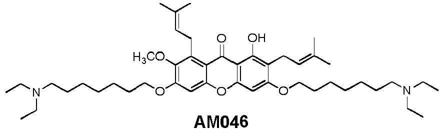

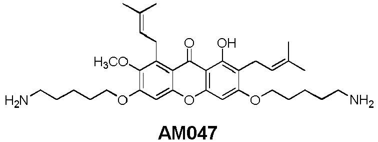

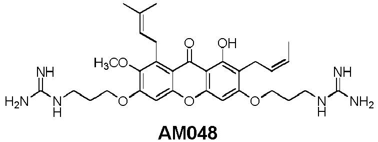

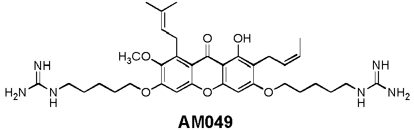

### Synthesis of AM005

alpha-Mangostin (1.0 g, 2.44 mmol) was dissolved in 15 mL of acetone, then potassium carbonate (1.6 g, 12.20 mmol) and 1, 4-dibromobutane (4.34 mL, 36.6 mmol) were added. The mixture was refluxed for 24 h. After the reaction was completed (TLC), the solvent was removed under reduced pressure. The oil residue was diluted with EtOAc and washed twice with saturated brine and once with water. The organic phase was dried over anhydrous Na₂SO₄ then purified via silica gel column chromatography (petroleum ether/EtOAc, 20/1, v/v), affording 1.27 g of product AM005 as a light yellow solid in 76.5% yield.

### Synthesis of AM012

To a solution of AM005 (100 mg, 0.147 mmol) in acetone (4 mL), 1H-pyrazole (100 mg, 1.47 mmol) and potassium carbonate (101 mg, 7.35 mmol) were added. The mixture was refluxed for 48 h. After the end of the reaction, the solvent was removed under reduced pressure. The residue was diluted with 50 mL of ethyl acetate and washed three times with saturated brine, dried over anhydrous Na₂SO₄. After removal of solvent, the residual mixture was purified via silica gel column chromatography (EtOAc/MeOH/Et3N, 100/2/1, v/v), affording 73.9 mg of product AM012 as a light yellow solid in 76.8% yield.

### Synthesis of AM016

To a solution of AM005 (100 mg, 0.147 mmol) in DMSO (4 mL), diethylamine (4 mL) was added. The mixture was stirred at room temperature for 3 h. After the end of the reaction, the mixture was diluted with 50 mL ethyl acetate, then washed with aqueous NaHCO₃ and saturated brine (each three times). The organic phase was dried over anhydrous Na₂SO₄ and concentrated under vacuum. The residual crude oil was purified via silica gel column chromatography (EtOAc/MeOH/Et3N, 100/2/1, v/v), affording 80.8 mg of pure product AM016 as a yellow oil in 82.7% yield.

### 1.1.2 Synthesis of cationic alpha-mangostin derivatives by a dicarboxyl -substituted approach.

Cationic modified alpha-mangostin derivatives were synthesized using a dicarboxyl substituted alpha-mangostin derivative. In order to obtain cationic modification alpha-mangostin derivatives, the intermediate was conjugated with different types of amines or peptides, such as arginine, histidine, lysine, spermidine, N,N-dimethyldipropylenetriamine, diethylenetriamine, ethanamine, N,N-diethyldiethylenetriamine, triethylenetetramine, pentaethylenehexamine, ethylenediamine, propan-1-amine, dimethylamine, diethylamine, 1,3-di-boc-2-(2-hydroxyethyl)guanidine et al. Some of this AM series analogues, especially AM-052, showed excellent antimicrobial activity against Gram-positive and Gram-negative bacteria, low cytotoxicity with a lower hemolytic activity with HC₅₀ of 238µg/mL. (Schemel-2) .

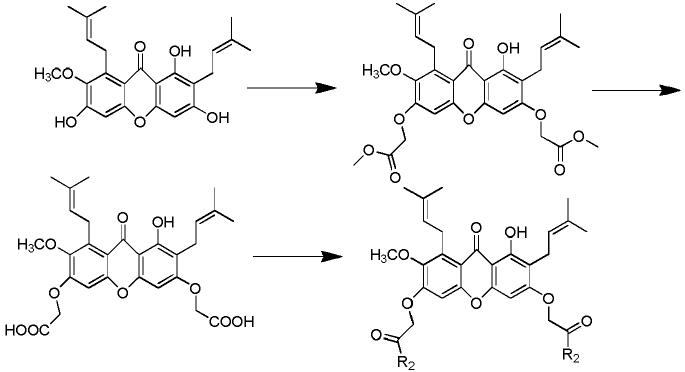

### Scheme 1-2: General synthesis route of key intermediate compounds of dicarboxyl-alkyl-substituted mangostin and some target molecules of cationic modification of alpha-mangostin by mimicking of AMPs. (Arg = Arginine, Lys = Lysine, His = Histidine).

Specific examples of this type of analogue are illustrated as below:

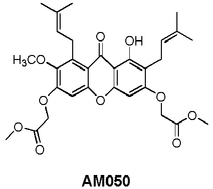

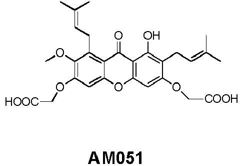

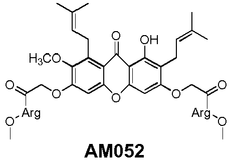

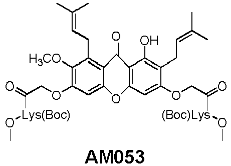

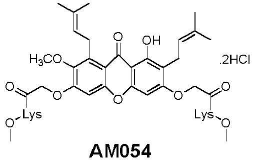

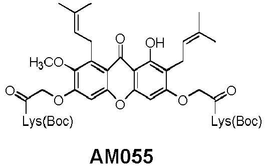

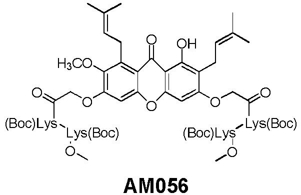

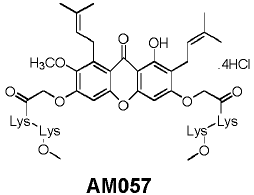

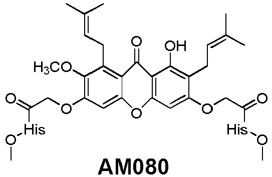

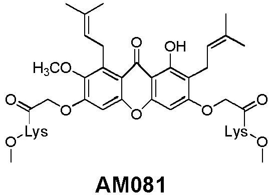

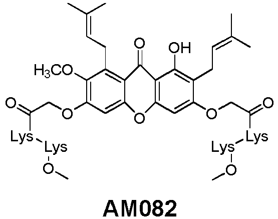

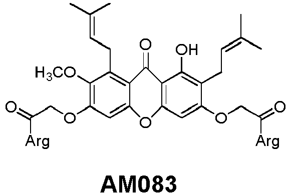

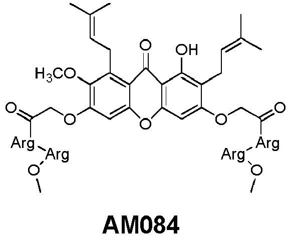

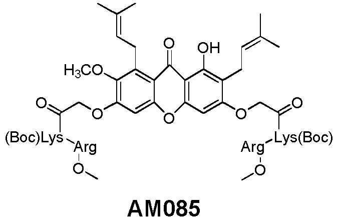

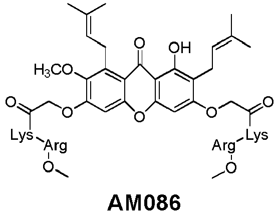

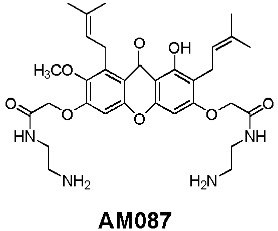

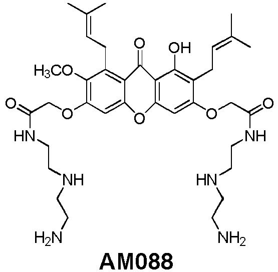

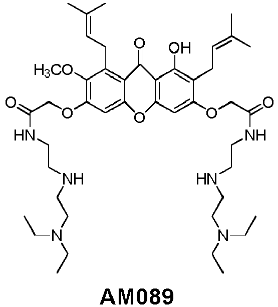

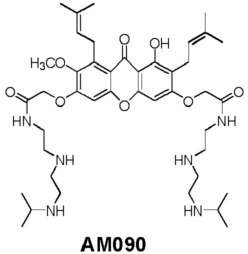

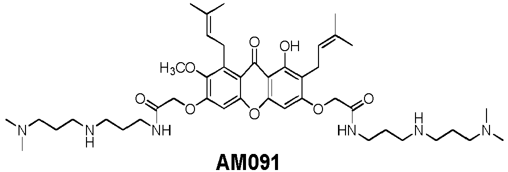

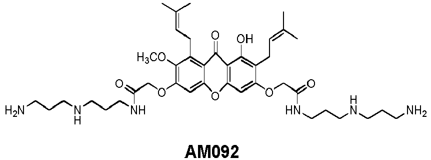

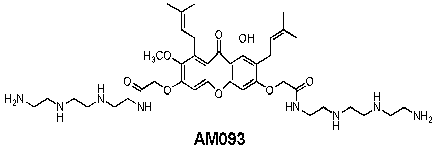

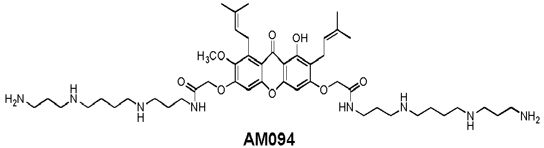

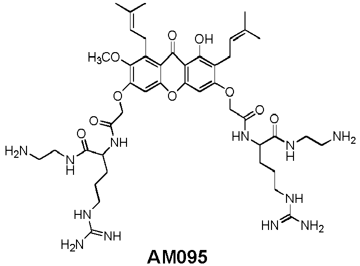

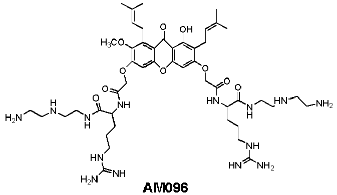

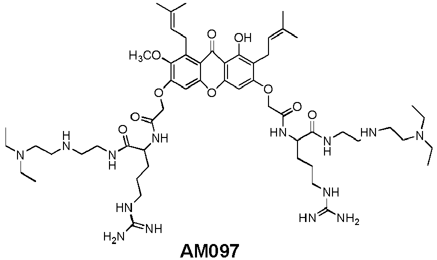

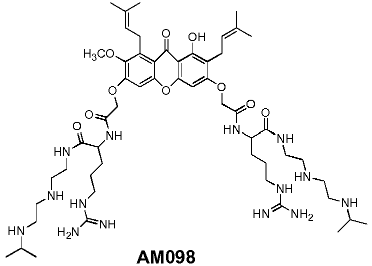

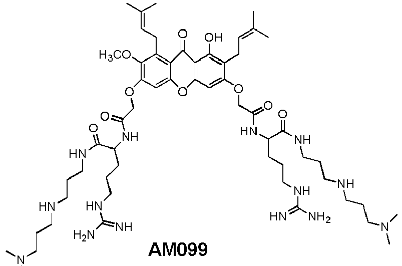

### Synthesis of AM050

A mixture of alpha-mangostin (0.5g, 1.22mmol), methyl bromoacetate (1.12 g, 7.3mmol) and KOH (341.6mg, 6.1mmol) in ethanol (30mL) was refluxed for 72h. After cooling down, the mixture was diluted with ethyl acetate and washed with 3 times NaCl solution (3×50ml). Organic phase was dried over anhydrous sodium sulfate. The solvent was evaporated to give crude product as oil. And then continue the next step reaction without further purification.

### Synthesis of AM051

To a solution of AM50 in 20ml THF, was added a solution of LiOH(87.84mg, 3.66mmol) in 10ml water. After stirring at room temperature for 2h, DCM was added and the layers were separated. The mixture was washed with 3 times DCM (3X20ml). And then the aqueous layer was acidified with diluted hydrochloric acid. The mixture was diluted with butanol and washed with 3 times NaCl solution (3×50ml). Organic phase was dried over anhydrous sodium sulfate. The solvent was evaporated to generate crude residue, which was purified by column chromatography (silica gel, PE/EtOAc/CH₃COOH, 3:1:0.04) to give 345.6mg yellow solid. The two step yield is 53.8%.

### Synthesis of AM052

HOBt (64.1mg, 0.475mmol) was added to AM51 (100mg, 0.19mmol) in anhydrous DMF (5 mL). At -10°C, DIC (59.9mg, 0.475mmol), H-Arg-OMe·2HCl (124mg, 0.475mmol) were added, and stir at -10°C for 1h. After stirring at room temperature overnight, the mixture was diluted with ethyl acetate and washed with 3 times NaCl solution (3×50ml). Organic phase was dried over anhydrous sodium sulfate. The solvent was evaporated to generate crude residue, which was purified by chromatography to give yellow solid.

### 1.1.3 Synthesis of cationic alpha-mangostin derivatives by diepoxy ethyl-substituted approach.

Cationic modified alpha-mangostin derivatives were also synthesized using diepoxy ethyl-substituted alpha mangostin derivatives. In order to obtain cationic modification alpha-mangostin derivatives, the intermediate was conjugated with different types of amines, such as ethanamine, propan-1-amine, dimethylamine, diethyl amine, dipropylamine, diisopropylamine, triethylamine, trimethylamine, tripropylamine, n-propylpentan-1-amine, butan-1-amine, thiazolidine, isoxazolidine, oxazolidine, pyrrolidine, morpholine, piperazine, thiomorpholine, 1-methylimidazolidine, 1,2,4-triazole, 1H-imidazole, pyrazole, pyridine, pyridazine, pyrimidine, 1H-1,2,3-triazole and the like. (see Scheme 1-3).

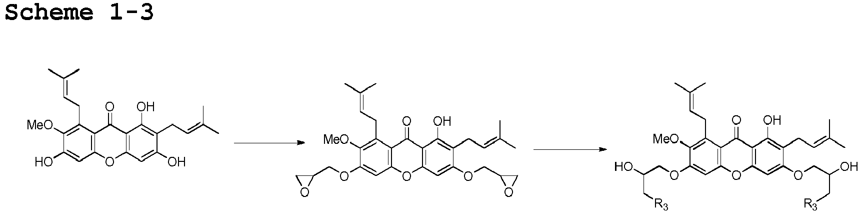

### Scheme 1-3: General synthesis route of key intermediate compounds of diepoxy substituted mangostin and target molecules of cationic modification of alpha-mangostin.

Specific examples of this type of analogue are illustrated as below:

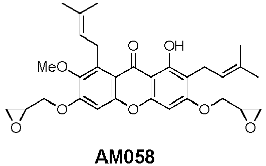

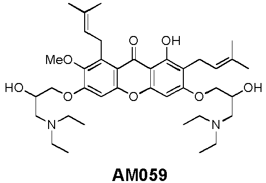

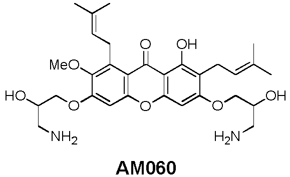

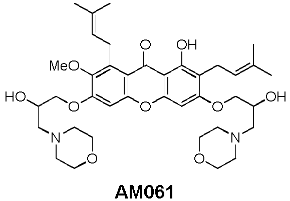

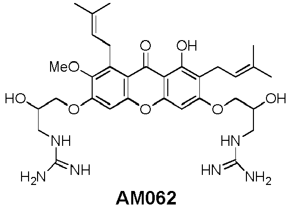

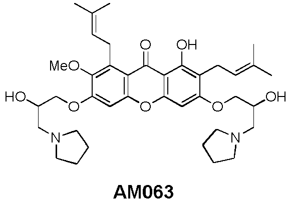

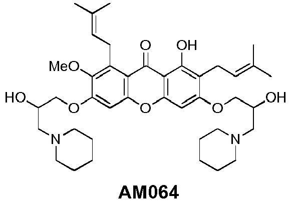

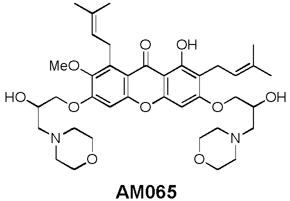

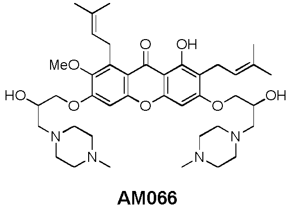

### Synthesis of AM058

A mixture of alpha-mangostin (0.5g, 1.22mmol), 1-bromo-2,3-epoxypropane (2.507 g, 18.3mmol) and KOH (341.6mg, 6.1mmol) in ethanol (30mL) was refluxed for 24h. After cooling down, the mixture was diluted with ethyl acetate and washed with 3 times NaCl solution (3×50ml). Organic phase was dried over anhydrous sodium sulfate. The solvent was evaporated to generate crude residue, which was purified by column chromatography (silica gel, PE/EtoAc/, 3:1) to give yellow solid (yield: 133.9mg, 21%)

### Synthesis of AM059

To a solution of AM058 (100 mg, 0.191 mmol) in methanol (4 mL), diethylamine (2 mL) was added. The mixture was refluxed for 6 h. After the end of the reaction, the mixture was evaporated to remove the excess amine and solvent, and then diluted with 40 mL ethyl acetate, then washed with aqueous NaHCO₃ and saturated brine (each three times). The organic phase was dried over anhydrous Na₂SO₄ and concentrated under vacuum. The residual crude oil was purified purified by column chromatography (silica gel, EtOAc/MeOH/Et₃N, 100/2/0.5, v/v) to give yellow solid.
(yield: 93.3mg, 73.1%).

### 1.2 Synthesis of anionic modification of alpha-mangostin.

In addition to the cationic modification of alpha-mangostin, anionic modifications of alpha-mangostin were synthesized (see Scheme 1-4).

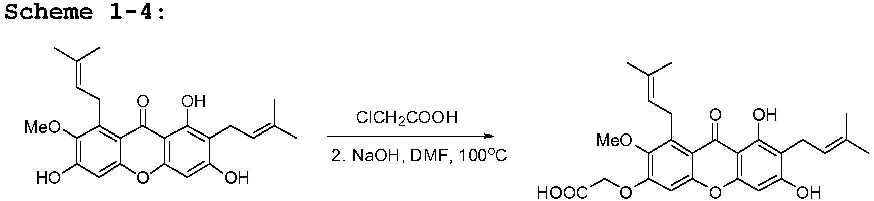

### Scheme 1-4: General synthesis of anionic alpha-mangostin analogues.

Specific examples of this type of analogue are illustrated as below:

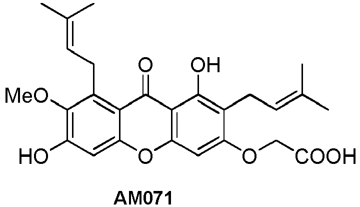

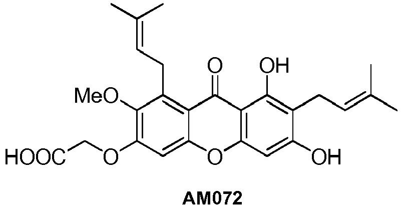

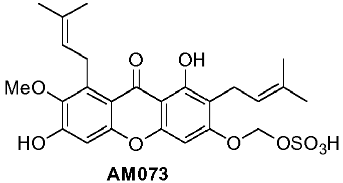

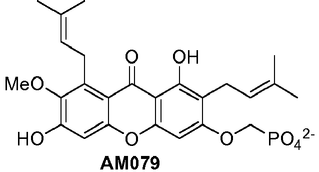

### Synthesis of AM071 and AM072

Alpha-mangostin (201.6 mg, 0.491 mmol) was reacted with chloroacetic acid (826.8 mg, 8.75 mmol) and NaOH (0.576 mg) in DMSO (5 ml) with stirring at 75 °C for 72 h. The reaction mixture was cooled down and hydrochloric acid was added until no additional precipitate was formed. Solvent was removed, then the residual oil was washed 3 times with saturated NaCl solution. The organic extracts were combined and dried overnight with anhydrous Na₂SO₄. Solvent was removed under reduced pressure. The resulting residue was purified by column chromatography (PE/EtoAc/AA,70/40/1,V/V/V). Yield: AM071, 45.2 %; AM072, 16.9%

### 1.3.Neutral hydrophilic modification of alpha-mangostin.

Neutral modified alpha-mangostin derivatives were synthesised. In contrast to cationic and anionic modified alpha mangostin, neutral functional group without charge carrier such as halogens, alkyl groups, sulfonamide or polyethylene glycols (PEG) were used to modify the alpha-mangostin structure (see Scheme 1-5)

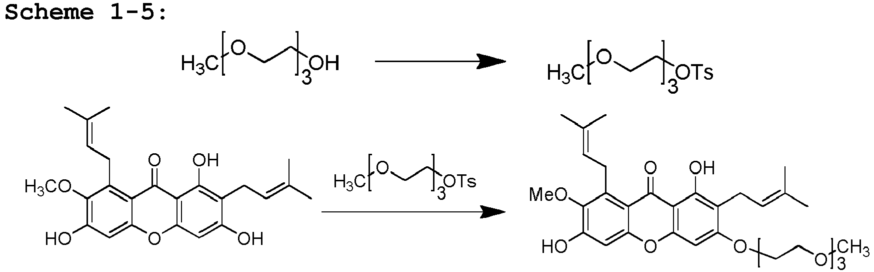

### Scheme 1-5: General synthesis of AM series of PEG-mangostin conjugate

The specific examples of this type of analogue are illustrated below:

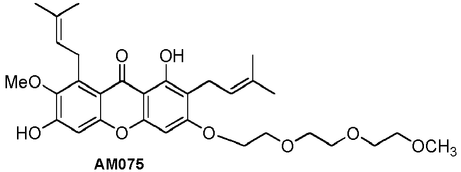

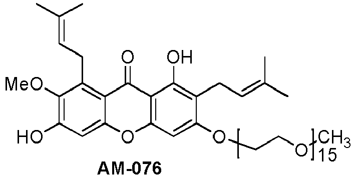

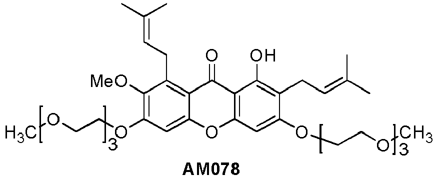

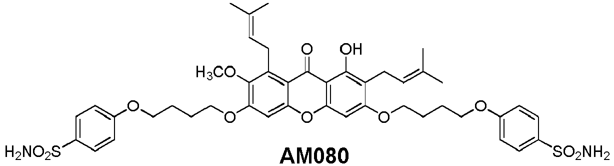

### Synthesis of AM-076

A mixture of alpha-mangostin (100 mg, 0.244 mmol), PEG-OTs (0.4542 g, 0.502 mmol) and K₂CO₃ (0.0488 g, 0.353 mmol) in DMF (5 ml) was stirred at 100 °C. After the end of the reaction, the mixture was diluted with ethyl acetate and washed 3 times with NaCl solution. The resulting residue was purified by silica gel column chromatography (EtoAc/MeOH, 5/3, V/V) to give compound AM-076 as a brownish-yellow liquid in yield of 57.1%.

### Example 2 Biological activity

**2.1 Minimum inhibition concentrations (MIC) and Selectivity.** The following tables show the antimicrobial activity of some of the compounds described herein.

**Table 2-1. Antibacterial Activity (MIC), in vitro toxicity (HC₅₀) and Selectivity of α-mangostin derivatives.**

| Compound | n | R | pKa of conjugated amine, R-H | MIC₉₉ [µg mL⁻¹] | | HC₅₀ [µg mL⁻¹]^{a} | Selectivity [HC₅₀/MIC₉₉] | |
|---|---|---|---|---|---|---|---|---|
| | | | | MRSA^{b} | *S.aureus*^{c} | | MRSA | *S.aureus* |
| **AM-011** | 3 | 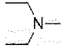 | 10.98 | 0.39 | 0.78 | 16.27 | 41.72 | 20.86 |
| **AM-016** | 4 | 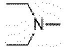 | 10.98 | 0.39 | 0.39 | 19.6 | 50.26 | 50.26 |
| **AM-008** | 5 | 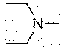 | 10.98 | 0.78 | 1.56 | 14 | 17.95 | 8.97 |
| **AM-010** | 6 | 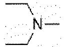 | 10.98 | 1.56 | 1.56 | 26.5 | 16.99 | 16.99 |
| **AM-015** | 4 | 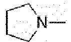 | 11.27 | 1.56 | 1.56 | 25 | 16.03 | 16.03 |
| **AM-017** | 4 | 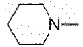 | 11.22 | 1.56 | 0.78 | 19.25 | 12.34 | 24.68 |
| **AM-009** | 4 | 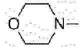 | 8.36 | >200 | >200 | >200 | -^{d} | - |
| **AM-012** | 4 | 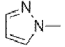 | 2.52 | >200 | >200 | >200 | - | - |
| **AM-002** | 4 | 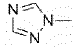 | 2.20 | >200 | >200 | >200 | - | - |
| **AM-005** | 4 | Br | NA^{e} | >200 | >200 | >200 | - | - |
| **AM-000** | 0 | H | NA | 1.56 | 1.56 | 9 | 5.77 | 5.77 |

### Example 2 - Antimicrobial activity of Alpha-Mangostin derivative AM016

The anti-microbial activity of AM016 (3,6-bis-[4-(diethylamino)butoxy)]-1-hydroxy-7-methoxy-2,8-bis(3-methylbut-2-enyl)-9H-xanthen-9-one; or 3,6-O-bis[4-(diethylamino)butyl]-α-mangostin) was tested.

As can be seen in Table 1A, compound AM0016 demonstrates very good antimicrobial activity against Gram positive bacteria, specifically a series of MRSA strains. Table 1a and 1b show the minimum inhibition concentration (MIC) (µg/ml)/(µM) of compound AM-016 needed for antimicrobial activity.

AM-016 also demonstrates a broad antimicrobial activity against Gram negative bacteria. AM016 is also effective against Gram negative bacteria (Table 1b).

AM-016 eliminates 99.9% of MRSA in a sample within 20 minutes of application (Figure 1)

**Table 1a**

| MRSA strain | AM-016 (µg/ml) | AM-016 (µM) |
|---|---|---|
| MRSA DM21455 | 0.39 | 0.59 |
| Source : Eye | | |
| Clinical Staphylococcus aureus DM4001 | 1.56 | 2.35 |
| Source : Eye | | |
| MRSA DM09808R | 1.56 | 2.35 |
| Source : Eye | | |
| Clinical Staphylococcus aureus DM4400R | 0.78 | 1.17 |
| Source : Cornea | | |
| Clinical Staphylococcus aureus DM4583R | 0.78 | 1.17 |
| Source : Eye | | |
| MRSA DB57964/04 | 0.78 | 1.17 |
| Clinical Staphylococcus aureus | 1.56 | 2.35 |
| DM4299 | | |
| MRSA DM21595 | 0.78 | 1.17 |
| Source : Wound | | |
| MRSA DR42412 | 1.56 | 2.35 |
| Source : Sputum | | |
| MRSA DR68004 | 1.56 | 2.35 |
| Source : Blood | | |
| MRSA QC Strain ATCC BAA1026 | 3.125 | 4.70 |
| Staphylococcus aureus ATCC29213 | 0.39 | 0.59 |
| Staphylococcus aureus ATCC 6538 | 1.56 | 2.35 |
| Straphylococcus aureus ATCC 6538P | 0.78 | 1.17 |
| Straphylococcus aureus ATCC 29737 | 1.56 | 2.35 |
| Straphylococcus aureus ATCC 25923 | 3.125 | 4.70 |

**Table 1b**

| Bacteria strain | AM-016 (µg/ml) | AM-016 (µM) |
|---|---|---|
| Streptococcus faecium ATCC 10541 | 0.195 | 0.295 |
| Streptococcus epidermidis ATCC 12228 | 0.78 | 1.17 |
| Bacillus cereus ATCC11778 | 3.125 | 4.70 |
| Pseudomonas aeruginosa ATCC27853 | 50 | 75.20 |
| Klebsiella pneumoniae ATCC10031 | 40 | 60.16 |
| Enterococcus faecalis ATCC 29212 | 0.78 | 1.17 |

### Table 1a and 1b: Minimum inhibition concentration (MIC) (µg/ml)/(µM) of compound AM-016 for eliminating Gram positive and Gram negative bacteria.

### Example 2 - Antimicrobial activity of AM-052 against against MRSA and Gram-negative bacteria

AM-052 shows excellent antimicrobial activity against MRSA and Gram-negative bacteria, with low hemolytic activity with HC50 of 238 (µg/mL) (See Table 3).

**Table 2: Summary of MIC99 (µg/mL), HC50 (µg/mL) and Selectivity Index of AM-052**

| Strain of bacteria | MIC99 | HC50 | Selectivity Index |
|---|---|---|---|
| Methicillin-resistant Staphylococcus aureus DM09808R | 3.125 | 238 | 76 |
| Methicillin-resistant Staphylococcus aureus DM21455 Source: Eye | 1.56 | 238 | 153 |
| Staphylococcus aureus ATCC 29213 | 6.25 | 238 | 38 |
| Bacillus cereus ATCC 11778 | 3.125 | 238 | 76 |
| Pseudomonas aeruginosa ATCC27853 | 12.5 | 238 | 19 |
| Klebsiella pneumoniae ATCC10031 | 6.25 | 238 | 38 |

### Example 3 - Basic physiochemical properties of AM016 and AM052

### 3.1 Solubility

Table 3-1 shows the solubility of compounds AM016 and AM052 in different media.

**Table 3-1:**

| | **Solubility (ug/ml)** | |
|---|---|---|
| **Solvents** | **AM016** | **AM052** |
| **Water** | 72.1±3.6 | >2mg/ml |
| **Saline** | 133.3±11.5 | >1mg/ml |
| **DMSO** | >7mg/ml | >2mg/ml |
| **PBS** | >3mg/ml | not soluble |

### Table 3-1: Solubility of AM016 and AM052 in different media

The determination of solubility was made as follows: AM016 with known weight was added with 1 mL saline, pure water or phosphate buffer saline (PBS) 20 mM at pH7. The solvent was added stepwise until the compound was dissolved completely. Then, the stock solution was diluted to series of diluents and the absorbance of each diluent was determined.

In PBS buffer AM-016 has excellent solubility. The solubility in PBS is 3 mg/ml.

In saline solution Figure 2 shows that the solubility of AM016 is 130 µg/mL.

In pure water solubility is 70 µg/ml, see Figure 3.

### 3.2 pH value of AM016 and AM052 in different media

**Table 3-2 pH value of AM016 in different media**

| **Solvents** | **Concentration of compound (**µg /ml) | **pH of AM0016** | **Concentration of compound (**µg /ml) | **pH of AM052** |
|---|---|---|---|---|
| **Water** | 50 | 6.44±0.27 | 50 | 6.52±0.29 |
| | 70 | 6.72±0.23 | 100 | 7.66±0.33 |
| **Saline** | 50 | 6.84±0.21 | 50 | 5.33±0.05 |
| | 100 | 6.78±0.05 | 100 | 5.33±0.01 |
| **PBS** | 50 | 6.96±0.01 | 50 | - |
| | 100 | 6.97±0.01 | 100 | - |

The pH values were obtained using an electronic pH meter.

### Example 4 - Minimum inhibition concentration (MIC) (µg/ml)/(µM) of compounds AM002, AM005, AM008, AM009, AM010

AM002, AM005, AM008, AM009, AM010 were tested against Gram positive bacteria including several strains of Staphylococcus aureus. The results are shown in Table 2. It can be seen that compounds AM008, AM009 and AM010 display very good antimicrobial activity against certain strains of *Staphylococcus aureus.* AM-005, which is a neutral hydrophobic modification of alpha-mangostin by incorporation of dibromo alkyl substitutions, did not show activity against Gram-positive bacteria at 50 ug/ml (see Table 4).

**Table 4: Minimum inhibition concentration (MIC) (µg/ml)/(µM) of compounds AM002, AM005, AM008, AM009, AM010**

| S/N | MRSA DM09808R Source : Eye | MRSA DM21455 Source : Eye | MRSA DM21595 Source : Eye | Clinical Staphylococcus aureus DM4001 | Bacilus Cereus ATCC11778 | Staphylococcus aureus ATCC29213 |
|---|---|---|---|---|---|---|
| AM-002 | >50 | ND | ND | ND | >50 | >50 |
| AM-005 | >50 | >50 | ND | ND | ND | >50 |
| AM-008 | | 0.78 | | | | 1.56 |
| AM-009 | | >25 | | | | >25 |
| AM-010 | | 1.56 | | | | 1.56 |

| S/N | Staphylococcus aureus ATCC6538 | Klebsiella pneumoniae ATCC10031 | Pseudomonas aeruginosa ATCC27853 | Escherichia coli ATCC8739 | | |
|---|---|---|---|---|---|---|
| AM-002 | ND | >100 | 30 | >100 | | |
| AM-005 | ND | ND | >50 | ND | | |

### Example 5 - Biological activity of compounds AM071 and AM072.

**Table 5 shows the biological activity of compounds AM071 and AM072 against certain strains of Gram positive bacteria.**

| **Bacteria** | **AM-071 (µg/ml)** | **AM-071 (mM)** | **AM-072 (µg/ml)** | **AM-072 (mM)** |
|---|---|---|---|---|
| MRSA DM21455 | 25 | 53.36 | 6.25 | 13.34 |
| Source: Eye | | | | |
| *Bacillus* ATCC11778 *cereus* | 6.25 | 13.34 | 6.25 | 13.34 |
| Clinical *Staphylococcus* DM4001 *aureus* | 25 | 53.36 | 6.25 | 13.34 |
| Source: Eye | | | | |
| *Staphylococcus* ATCC29213 *aureus* | 12.5 | 26.68 | 6.25 | 13.34 |

### Table 5: MIC data of AM-071 and AM-072 for Gram positive bacteria

As can be seen from Table 5 above, compounds AM071 and AM072 do not demonstrate very good antimicrobial activity against *Staphylococcus aureus* or *Bacillus cereus*

### Example 6 - In vivo cytotoxicity test of AM-016

AM-016 does not show cytotoxicity in the rabbit eye after 3 days of application of drug (Figure 5a and b).

Initial testing was carried out on a rabbit eye, using five applications per day at a concentration of 400 µg/mL (513-1026× MIC). No observable toxic effect was observed on the mouse cornea after AM016 was applied topically. Figure 5a shows the rabbit eye before application of AM016 and Figure 5b shows the rabbit eye after topical application of AM016.

### Example 7 - Effect of AM016 on wound healing of rabbit cornea

Figure 5 shows the results of a comparative test for cytotoxicity between AM-016 and vancomycin in a rabbit corneal wound healing model. The particular model used was a 5mm diameter corneal epithelial abrasion model. Wound healing was monitored by a slit lamp using flourescein disclosure dye so that the size of the wound could be measured. The compounds were applied topically three times per day.

As can be seen AM-016 does not exhibit any cytotoxicity to the rabbit eye after 3 days of application of drug (see Figure 5) compared with a normal control.

Figure 6 graphically illustrates the percentage wound closing over the same 3 days using vancomycin, AM-016 or saline solution. As can be seen, the application of AM-016 does not delay wound healing indicating that AM-016 does not display any cytotoxic effects.

### Example 8 - Drug resistance test of AM-016 with a MRSA strain from an eye

Figure 7 shows that MRSA DM21455 has difficulty in developing drug resistance towards AM-016 during 20 serial passages of bacteria grown in media with a constant concentration of AM-016. In contrast, MRSA DM21455 develops drug resistance more quickly against vancomycin when compared to AM-016.

Figure 8 shows the results of a multipassage resistance selection study to investigate the propensity of *S. aureus* and *Enterococcus faecalis* to develop endogeneous mutational resistance against AM016 during prolonged passages at the sub-inhibitory concentrations.

Resistance is defined as >4-fold increase in the original MIC developed in the multipassage study. Figure 8 shows that there was no evidence of resistance or cross-resistance against AM016 in any of the 4 strains tested.

### Example 9 - Action mechanism study of AM-016

Membrane potential-sensitive dye 3,3'-dipropylthiadicarbocyanine iodide (DiSC3) was used to determine the cytoplasmic membrane depolarization activity of AM-016.

An overnight culture of clinical Staphylococcus aureus DM4001 (source: Eye) was allowed to grow in Muller-Hinton broth (MHB) to OD620 = 0.3-0.4. Then, the bacteria was collected by centrifugation at 2800 r.p.m at 37°C for 30 minutes and washed with 5mM HEPES buffer at pH7. Then, the bacteria were re-suspended in the same buffer with with 0.2mM EDTA to obtain an OD620 value of 0.9-0.1. The cell suspension was then incubated with 0.1M KCl and 0.4µM DiSC3 at 37°C for 30 minutes. Dye uptake was monitored by fluorospectrometry (Photon Technology International (PTI) Photomultiplier Detection Systems, model 814) in a stirred cuvette until the fluorescence signal was stable. The desired concentration of AM-016 was added and the fluorescence was monitored at an excitation wavelength of 622nm and at an emission wavelength of 670nm. A blank with dye in the absence of bacteria with same concentration of AM-016 was used as a control.

Figure 9 shows that when 12.5ug/mL of AM-016 was added to the *S. aureus* culture, fluorescence increases. This signifies that AM-016 causes membrane depolarization and therefore DISC₃ was released into medium and results in an increase in fluorescence. Thus, AM-016 is a potential membrane-target antimicrobial agent which is able to disrupt or dissipate the membrane potential of Gram positive bacteria. In contrast, interestingly, its precursor dibromo-substituted AM-005 (without tertiary amine as terminal groups) does not depolarize the membrane (Figure 10).

In addition, time killing (see Figure 1) and drug resistance (Figures 7 and 8) also support the suggested depolarization mechanism of AM-016.

Figure 1 shows that more than 99.9% MRSA was killed within 20 minutes. For membrane targeted antimicrobial agents, the elimination time is very fast as the uptake of antimicrobial agent by the bacteria is not needed. The bacteria will be killed upon contact with antimicrobial agent.

Figure 7 shows that MRSA has difficulty in developing drug resistance towards AM-016 during 20 serial passages. To develop drug resistance against a membrane target antimicrobial agent, bacteria have to change the composition of the cell wall or membrane to prevent interaction of the membrane with the antimicrobial agent. However, for substantial changes in the membrane composition to occur, the bacteria must undergo multiple rounds of mutation which may ultimately be incompatible with bacterial survival [1]. Vancomycin is an antimicrobial used to treat MRSA infection. The antimicrobial action mechanism of vancomycin involves inhibition of peptidoglycan polymerization. Drug resistance in *Staphylococcus aureas* is more pronounced for vancomycin when compared with AM-016. Taken together these results strongly suggest that AM-016 targets the membrane of bacteria and other microbes.

The depolarization study strongly suggests that AM-016 depolarizes a clinical *S. aureus* DM4001 bacteria membrane. In contrast the precursor dibromo-alkyl substituted AM-005 (without tertiary amine as terminal groups) does not depolarize the membrane. This result supports the MIC screening, the time killing and drug resistance results presented above.

### SYTOX Green assay

SYTOX green is a membrane impermeable dye. Interaction of SYTOX green with a nucleic acid enhances the fluorescence emission of SYTOX green significantly. S. aureus DM4001 was harvested at early exponential phase and suspended in 40 mM PBS until OD₆₂₀ of 0.09 was obtained. Then, the bacteria suspension was incubated with 3 µM of SYTOX Green in dark. The mixture was monitored in a stirring cuvette at an excitation wavelength of 504 nm and at an emission wavelength of 523 nm until the fluorescence was stable. Then, 10 µM of AM compounds were added and the changed of fluorescence emissions were monitored. Figure 4-2A shows that antimicrobial active compounds (AM-008, AM-010, AM-010, AM-011, AM-015, AM-016, AM-017) could induce membrane permeabilization and enhance the SYTOX green fluorescence emission. In contrast, inactive compounds including AM-002, AM-005, AM-009, AM-012, AM-044 and AM-045 were not able to trigger the membrane permeabilization (see Figure 11). AM-043 had weaker membrane permeabilization effect.

AM-008, AM-010, AM-011, AM-016, AM-015 and AM017 were found to be active against Gram-positive bacteria but inactive against Gram-negative bacteria. To further confirm that membrane permeabilization is important to kill the bacteria, the SYTOX green assay was tested using E. coli ATCC8739. Figure 12 shows that addition of all AM-series compounds tested did not induce membrane permeabilization of E. coli as there was no observable increased of SYTOX green fluorescence emission. Figure 13 shows the membrane permeabilization effects of selected compounds (active against Gram-positive bacteria) on Gram-positive and Gram-negative bacteria. The data clearly suggest that membrane targeting and inner membrane permeabilization are crucial for the compounds described herein to kill the bacteria.

In addition to the fluorescence spectroscopy, we further confirm the inner membrane targeting properties of active AM-series compounds using fluorescence microscopy (Figure 14). Large numbers of stained bacteria were observed in the culture incubated with active AM-series compounds (AM-008, AM-010, AM-011, AM-015, AM-016, AM-017) at 10 µM, clearly indicating that these AM-series compromised the living bacterial cytoplasmic membrane. In contrast, no significant staining was not observed in the culture treated with inactive compounds (AM-002, AM-005, AM-009, AM-012, AM-044, AM-045).

### Calcein Leakage Study

To further investigate antimicrobial action of the active compounds via inner membrane targeting, a calcein leakage study was performed. In brief, liposomes which mimic bacterial membrane (DOPE: DOPG=3:1) and red blood cell (DOPC: DOPS=3:1) were synthesized. The lipids were dissolved in methanol/chloroform (1:2, by volume). The solvent was then dried gently using a constant stream of nitrogen gas. Then, the lipid film was placed under vacuum for at least two hours. The dried lipid film was then hydrated with calcein solution (80 mM calcein, 50 mM HEPES, 100 mM NaCl, 0.3 mM EDTA, pH 7.4). The hydrated vesicles were freeze- thaw (frozen in liquid nitrogen and warmed in water bath) for at least 7 cycles. Extrusion method using mini-extruder (Avanti Polar Lipid Inc.) was used to produce homogeneous large unilamellar vesicles (LUVs). The extrusion was done for at least 10 cycles using a polycarbonate membrane (Whatman, pore size 100 nm) to obtain LUVs with diameter of 100 nm. To separate the calcein encapsulated vesicles from free calcein, gel filtration column Sephadex G-50 was used. The concentrations of eluted liposomes were determined using total phosphorus determination assay. Leakage of calcein from the liposome induced by AM-series compounds could be monitored using fluorescence spectroscopy (Photon Technology International Model 814) at an excitation wavelength of 490 nm and an emission wavelength of 520 nm. An aliquot of the LUV suspension and lipid to AM-series compounds ratio of 2, 4 and 8 were mixed in a stirred cuvette and the fluorescence emission intensity was monitored. 0.1% Triton X-100 was use to determine the intensity at 100% leakage. Percentage of leakage (%L) was calculated with %L= [(Iₜ - I₀)/(I_{∞} - I₀)]^{∗}100], where I₀ and Iₜ are intensity before and after addition of α-mangostin respectively and I_{∞} is intensity after addition of 0.1% triton X-100. Table 4-3A shows the % calcein leakage from the liposome (DOPE:DOPG= 3:1) induced by AM-series compounds. Inactive xanthones did not induce observable leakage up to compound to lipid ratio of 1:2. In addition, active AM-series compounds are also able to induce leakage of calcein from DOPC:DOPS= 3:1 liposomes, which corroborate with the HC50 values for the compounds (Table 4-3B). Those compounds with high low HC50 values have stronger leakage %. Compounds with high HC50 values show negligible calcein leakage (< 10%). To further investigate the inner membrane targeting action of AM-series compounds, *E. coli* lipid extract was used to construct the liposome. Although all AM-series compounds are inactive against E. coli, surprisingly, AM-010, AM-011, AM-008, AM-015, AM-016, AM017 were able to induce leakage of calcein from the *E. coli* lipid extract (Table 4-3C). Therefore, the outer layer of Gram-negative bacteria (LPS) may play important role to impede the AM-series compounds to reach inner membrane. Calcein leakage data strongly support the active compounds are inner membrane targeting.

**Table 6. Percentage leakage of calcein from DOPE:DOPG= 3:1 liposomes induced by compounds as described herein.**

| Liposome (DOPE:DOPG=3:1) | | | % Leakage | | |
|---|---|---|---|---|---|
| | | | AM-series | compounds: | Lipid ratio |
| | | | 1:2 | 1:4 | 1:8 |
| AM-011 | | | 52.35 | 60.39 | |
| AM-016 | | | 54.43 | 37.00 | 21.00 |
| AM-008 | | | 61.50 | 55.00 | 36.46 |
| AM-010 | | | 67.00 | 54.34 | 46.57 |
| AM-015 | | | | | |
| AM-017 | | | | | |
| AM-002, | AM-012, | AM-009, | | < 10 | |
| AM-005 | | | | | |

**Table 7. Percentage leakage of calcein from DOPC:DOPS= 3:1 liposomes induced by compounds described herein.**

| Liposome (DOPE:DOPG=3:1) | | | % Leakage | | |
|---|---|---|---|---|---|
| | | | AM-series | compounds: | Lipid ratio |
| | | | 1:2 | 1:4 | 1:8 |
| AM-011 | | | 75.19 | 58.19 | 34.10 |
| AM-016 | | | 88.43 | 72.76 | 37.66 |
| AM-008 | | | 90.87 | 61.34 | 33.54 |
| AM-010 | | | 91.00 | 62.05 | 37.31 |
| AM-015 | | | | | |
| AM-017 | | | | | |
| AM-002, | AM-012, | AM-009, | | < 10 | |
| AM-005 | | | | | |

**Table 8. Percentage leakage of calcein from E. coli lipid extract induced by compounds described herein.**

| Liposome (*E. coli* extract) | | | % Leakage | | |
|---|---|---|---|---|---|
| | | | AM-series | compounds: | Lipid ratio |
| | | | 1:2 | 1:4 | 1:8 |
| AM-011 | | | 69.52 | 14.72 | 5.05 |
| AM-016 | | | 57.82 | 58.43 | 31.70 |
| AM-008 | | | 79.97 | 50.49 | 24.06 |
| AM-010 | | | 73.85 | 48.11 | 28.51 |
| AM-015 | | | | | |
| AM-017 | | | | | |
| AM-002, | AM-012, | AM-009, | | < 10 | |
| AM-005 | | | | | |

### Extracellular ATP leakage assay

ATP leakage assay is also an important assay to study the inner membrane targeting property of a compound. ATP is released from a membrane compromised cells and the amount of ATP released can be detected. In contrast, if a compound does not induce membrane disruption, minimum ATP released will be detected. In this study, a bioluminescence assay was used using recombinant firefly luciferase and its substrate D-luciferin. ATP is required for luciferase to interact with luciferin to produce light. First, bacteria suspension (OD= 0.4) was incubated with AM-series compounds with desired concentration at 37°C for 10 minutes. Then, the suspension was centrifugated at 3000 r.p.m. for 5 minutes. Standard reaction solution containing 0.5 mM D-luciferin, 1.25 µg/mL firefly luciferase, 25 mM Tricine buffer at pH 7.8, 5 mM MgSO₄, 100 µM EDTA and 1 mM DTT was prepared and added into 96 well plates. 10 uL of supernatant of the culture solution was added and the ATP released was determined using Luminometer (TECAN Infinite M200Pro). 8 µM of nisin was used to trigger complete ATP leakage. Figure 4-4 shows the ATP leakage induced by the AM-series compounds. Compounds active against Gram-positive bacteria could induce strong ATP leakage. In contrast, inactive compounds were not able to induce ATP leakage. The result further shows that active AM-series compounds are inner membrane targeting (see Figure 15).

The results of the antimicrobial action studies clearly show that AM-series compounds are inner membrane targeting. The mechanism is well correlated with the antimicrobial properties of active AM-series compounds such as AM016. Figure 1 shows that more than 99.9% MRSA was killed within 20 minutes. For membrane target antimicrobial, the time killing is very fast as the uptake of antimicrobial by the bacteria is not needed. The bacteria will be killed upon it contacts with membrane target antimicrobial.

In addition, Figures 6 and 7 show that MRSA is difficult to develop drug resistance against AM-016 during 20 serial passages. To develop drug resistance against membrane target antimicrobial, bacteria have to change the compositions of cell wall or membrane to prevent the interaction with membrane target antimicrobial. However, tremendously changed of membrane composition requires multiple mutation and such alternation might be incompatible with cellular survival.

### Example 10 - Antimicrobial activity of AM-016 compared with vancomycin and daptomycin against various strains of Staphylococcus aureus

The antimicrobial activity of AM-016 was compared with vancomycin and daptomycin against various strains of *Staphylococcus aureus* using the Mueller Hinton broth dilution method.

Figures 16 to 20 show the antimicrobial activity of AM-016, vancomycin and daptomycin against various strains of *Staphylococcus aureus.* As can be seen AM-016 exhibits antimicrobial activity (as determined by the minimum inhibitory concentration in µg/ml) against strains MRSA-21455 (see Figure 16), MRSA-09080R (see Figure 17), MRSA-42412 (see Figure 18), MRSA-21595 (see Figure 19) and MRSA-700699 (see Figure 20). AM-016 shows superior antimicrobial activity against strains MRSA-09080R, MRSA-21595 and MRSA-42412 when compared to vancomycin. However, AM-016 shows superior antimicrobial activity against MRSA-700699 when compared to both daptomycin and vancomycin, with a minimum inhibitory concentration of 0.048 µg/ml.

The minimum inhibitory concentrations (MIC) are as follows:

| | MIC values (µg/ml) | | |
|---|---|---|---|
| | Daptomycin | Vancomycin | AM-016 |
| MRSA-21455 (Eye) | 0.78 | 0.78 | 1.56 |
| MRSA-09808R (Eye) | 0.195 | 0.395 | 3.125 |
| MRSA-42412 (Sputum) | 0.195 | 0.78 | 0.395 |
| MRSA-700699 | 0.78 | 6.25 | 0.095 |
| MRSA-21595 (Wound) | 0.395 | 0.78 | 0.395 |

The minimum inhibitory concentrations for AM-016 and vancomycin against certain other strains of Gram positive bacteria are also presented below:

| Strains | AM-016 | Vancomycin |
|---|---|---|
| Enterococcus faecalis ATCC 51299 VRE | 1.56 | 50 |
| Enterococcus faecalis 11: DS6527 (VRE 208/11) | 0.098 | Above 100 |
| Enterococcus faecalis 11: DU9345 VRE | 0.78 | 1.56 |
| Staphylococcus aureus 10: DB6506 (MRSA04/10) (VISA) | 0.78 | 1.56 |
| Staphylococcus aureus ATCC 700699 (VISA) | 0.39 | 6.25 |

As can be seen, AM-016 demonstrates superior antimicrobial properties compared to vancomycin.

### Example 11 - Antimicrobial activity of alpha-mangostin derivative compounds against Mycobacterium tuberculosis.

Table 10 shows the antimicrobial activity of some alpha mangostin derivatives described herein against *Mycobacterium tuberculosis.*

**Table 10. Antimicrobial activity against Mycobacterium tuberculosis, hemolytic activity and selectivity index of some typical AM-series compounds.**

| Code number | Mw | MIC100 nmol/ml (µg/ml) | Hemolytic concentration, HC50 (µg/ml) | Selectivity index |
|---|---|---|---|---|
| AM000 (alpha-mangostin) | 410.46 | NI | 6.5 | |
| AM002 | 656.77 | NI | | |
| AM005 | 680.46 | NI | | |
| AM008 | 692.97 | 4 (2.77) | 14 | 5.05 |
| AM009 | 692.88 | 8 (5.54) | >1000 | >180 |
| AM012 | 654.79 | NI | | |
| AM-013 (Gamma-mangostin) | 396.43 | 125 (49.55) | 25 | 0.50 |
| AM015 | 660.88 | 4 (2.64) | 25 | 9.46 |
| AM016 | 664.91 | 4 (2.66) | 19.6 | 7.37 |
| AM071 | 526.53 | 250 | 37.5 | 0.28 |
| AM072 | 556.64 | 250 | ND | |
| Ethambutol | 204.31 | 20 | | |

| | | | | |
|---|---|---|---|---|
| Note: NI: no activity at 250 nmol/mL | | | | |

As can be seen from the results presented in Table 10 AM-009 appears to have the best selectivity against *Mycobacterium tuberculosis.*

### References

[1] Ooi, et al, XF-73, a novel antistaphylococcal membrane-active agent with rapid bactericidal activity, Journal of Antimicrobial Chemotherapy (2009) 64, 735-740.

## Claims

1. A compound of Formula II or a salt thereof: 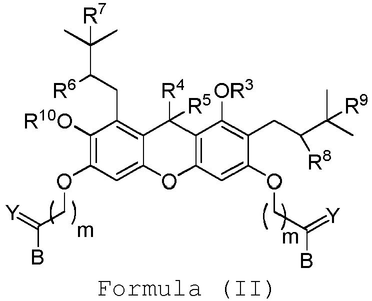 wherein
m is 1;
Y is O;
B is NHR²⁴;
each of R³ and R¹⁰ independently for each occurrence is hydrogen, alkyl, alkenyl, alkynyl, cycloalkyl, cycloalkenyl, aralkyl, heterocycloalkyl, heterocycloalkenyl, heteroaryl, heteroaralkyl,-O(C=O)R¹¹, -O(C=O)OR¹¹, -O(C=O)NR¹²R¹³, -OSO₃R¹¹, or -OPO₂OR¹¹;
R⁴ and R⁵ taken together with the carbon to which they are bonded form (C=O);
R⁶ and R⁷ taken together form a bond; R⁸ and R⁹ taken together form a bond;
R¹¹ is independently for each occurrence hydrogen, alkyl, alkenyl, alkynyl, cycloalkyl, cycloalkenyl, aralkyl, heterocycloalkyl, heterocycloalkenyl, heteroaryl, or heteroaralkyl;
each of R¹² and R¹³ independently for each occurrence is hydrogen, alkyl, alkenyl, alkynyl, cycloalkyl, cycloalkenyl, aralkyl, heterocycloalkyl, heterocycloalkenyl, heteroaryl, or heteroaralkyl; or R¹² or R¹³ taken together form a 3-8 membered heterocyclic ring;
R²⁴ is

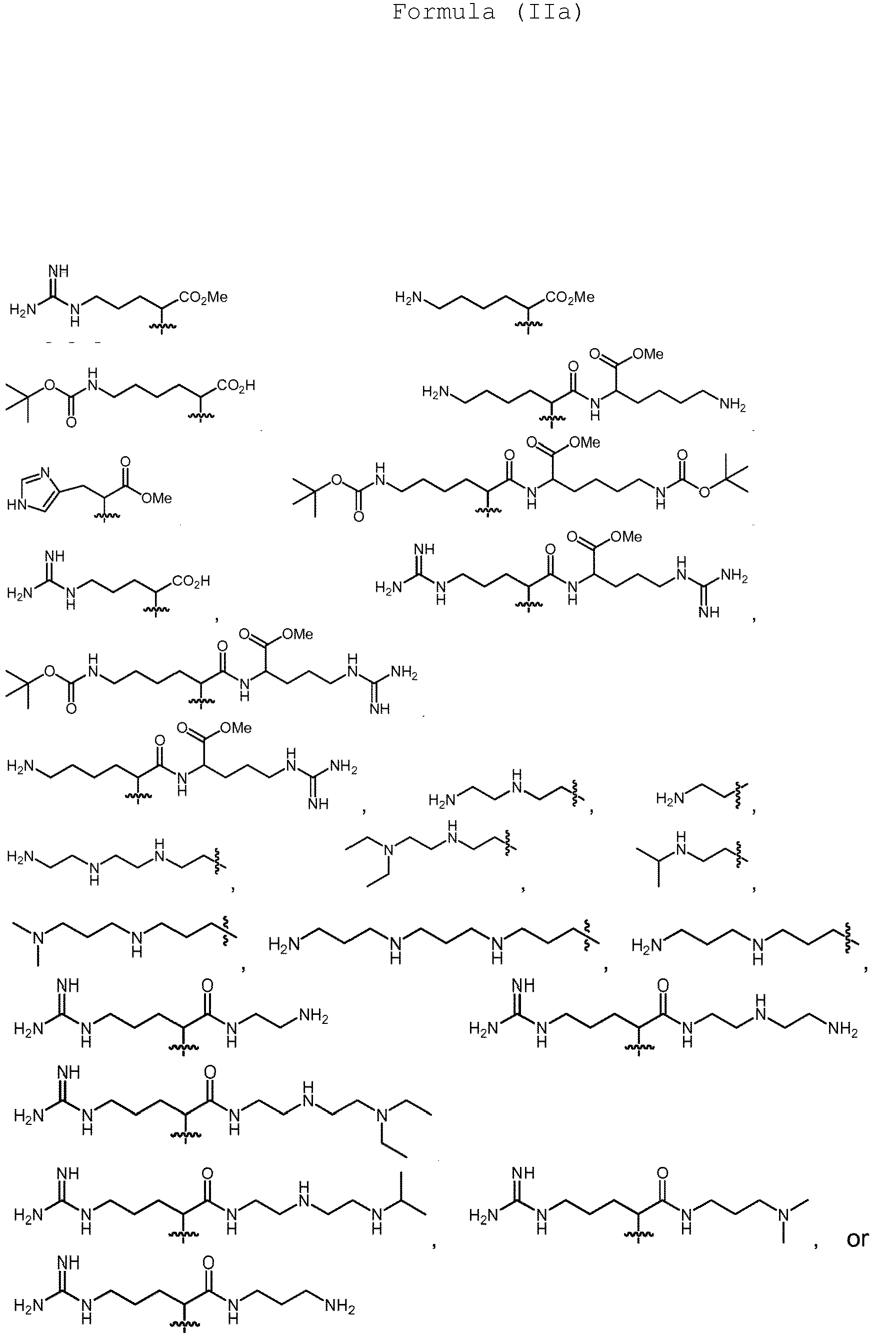

2. The compound of claim 1, wherein R¹⁰ is alkyl.

3. A compound of any one of claims 1-2, wherein the compound is selected from the group consisting of: 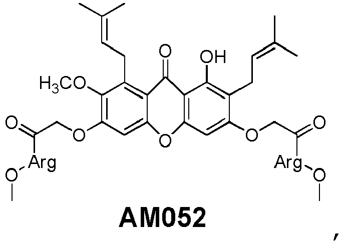 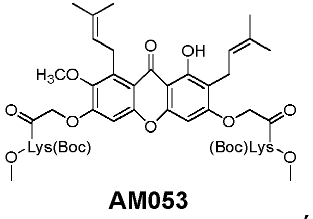 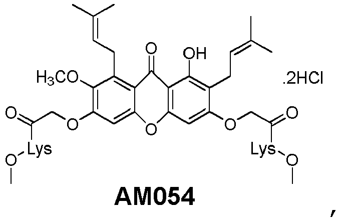 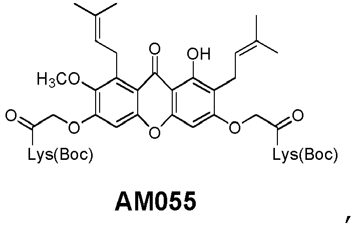 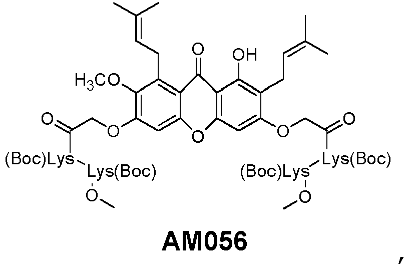 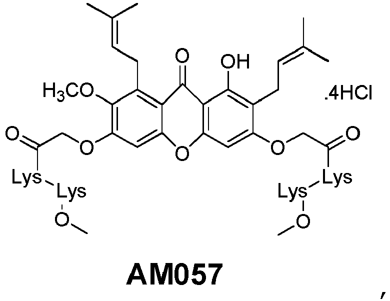 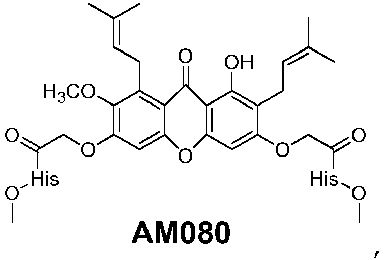 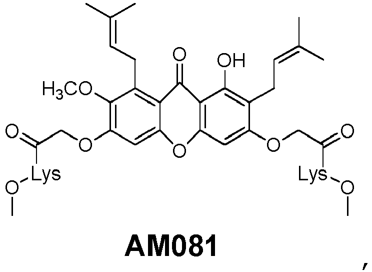 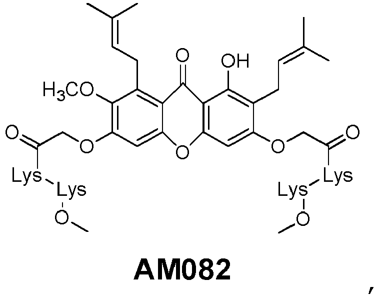 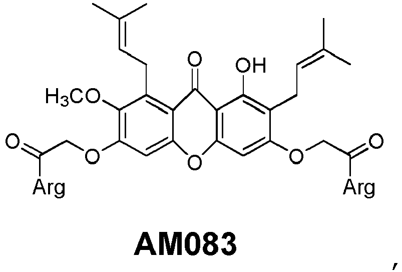 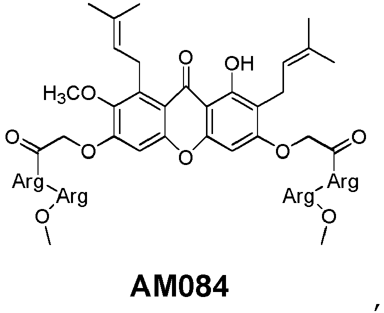 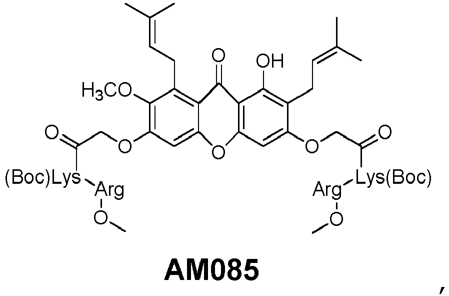 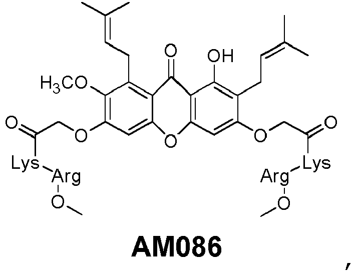 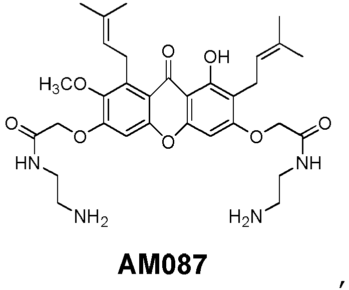 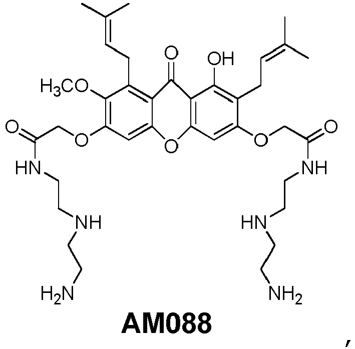 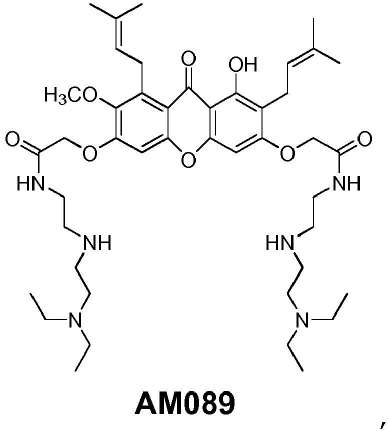 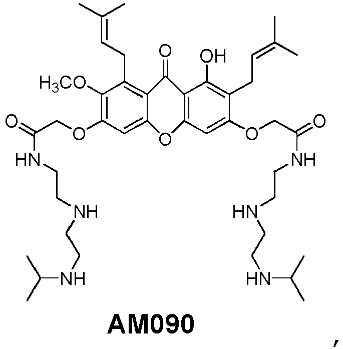 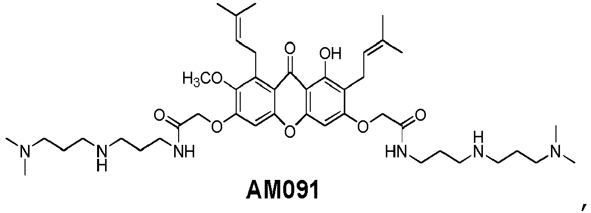 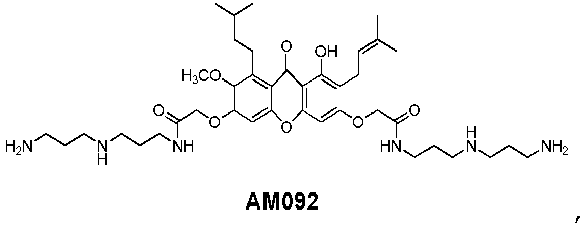 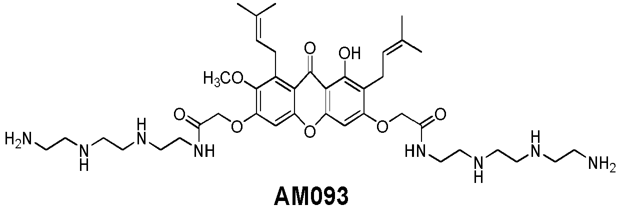 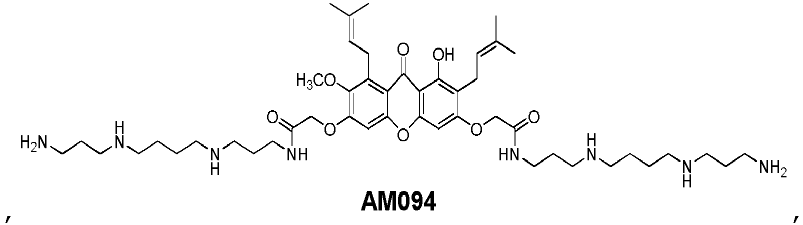 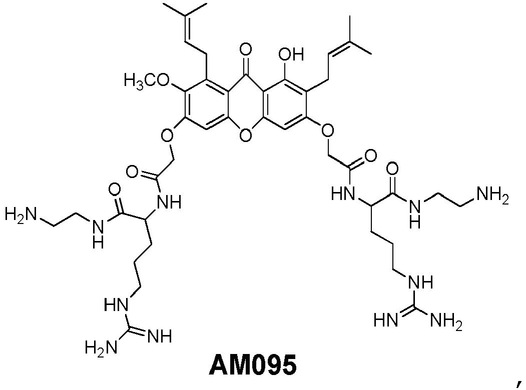 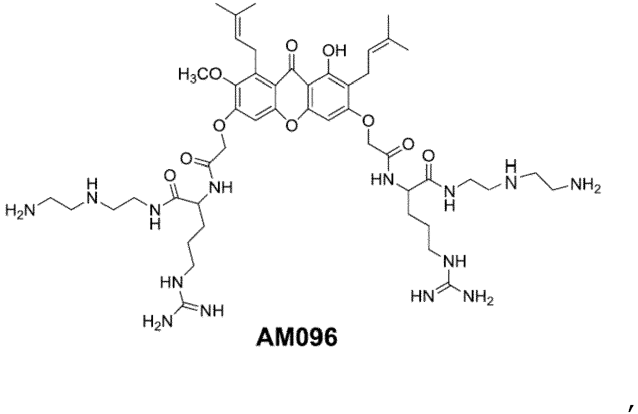 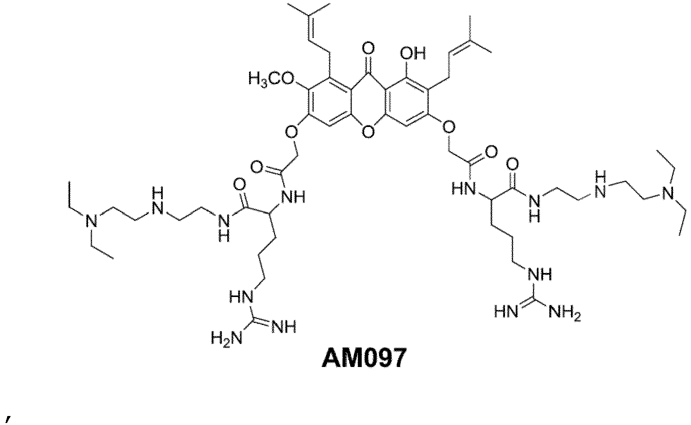 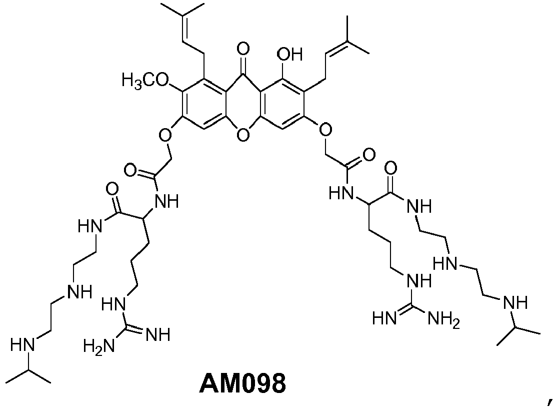 and

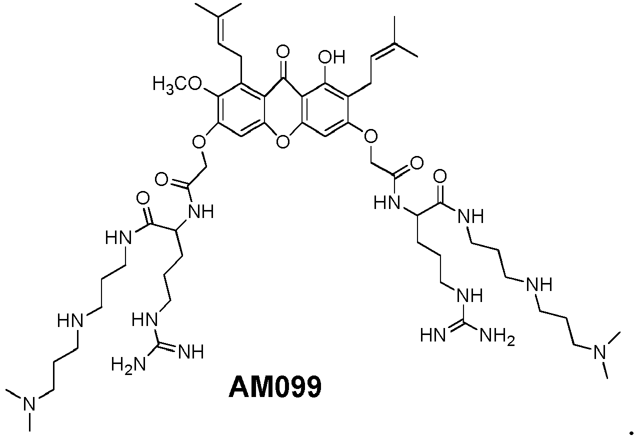

4. A pharmaceutical composition comprising a compound of any one of claims 1 to 3 together with a pharmaceutically acceptable carrier.

5. A compound as claimed in any one of claims 1 to 3, for use as a medicament.

6. The compound for use according to claim 5, wherein said use is as a medicament for the treatment of a microbial infection.

7. The compound for use as claimed in claim 6, wherein the microbial infection is a bacterial infection caused by Gram positive or Gram negative bacteria, a Mycobacteria or a fungus, wherein the Gram positive bacteria is selected from the group consisting of *Streptococcus spp., Staphylococcus spp., Bacillus spp., Carynebacterium spp., Clostridium spp., Listeria spp.,* and *Enterococcus spp.,* preferably wherein said Gram positive bacteria is *Staphylococcus aureus and preferably wherein* said *Staphylococcus aureus is* Methicillin resistant *Staphylococcus aureus.*

8. The compound for use as claimed in any one of claims 6 to 7, wherein said medicament is to be administered with one or more further active agents, optionally wherein the one or more further active agents are to be administered simultaneously, separately or sequentially with said medicament.

9. A pharmaceutical dosage form comprising a compound according to any one of claims 1 to 3 or a composition of claim 4.

10. A kit comprising a compound as claimed in any one of claims 1 to 3, a composition of claim 4 or a pharmaceutical dosage form of claim 9 and directions for use.

## Patentansprüche

1. Verbindung der Formel II oder ein Salz davon: 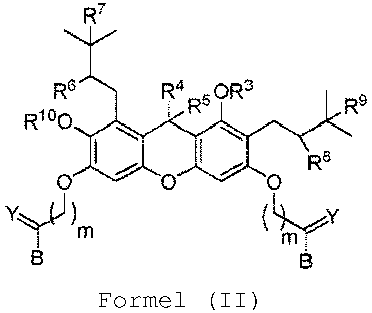 wobei
m 1 ist;
Y 0 ist;
B NHR²⁴ ist;
R³ und R¹⁰ jeweils unabhängig voneinander Wasserstoff, Alkyl, Alkenyl, Alkinyl, Cycloalkyl, Cycloalkenyl, Aralkyl, Heterocycloalkyl, Heterocycloalkenyl, Heteroaryl, Heteroaralkyl, -O(C=O)R¹¹, -O(C=O)OR¹¹, -O(C=O)NR¹²R¹³, -OSO₃R¹¹ oder -OPO₂OR¹¹ sind;
R⁴ und R⁵ zusammengenommen mit dem Kohlenstoff, an den sie gebunden sind, bilden (C=0);
R⁶ und R⁷ zusammengenommen eine Bindung bilden; R⁸ und R⁹ zusammengenommen eine Bindung bilden;
R¹¹ unabhängig für jedes Auftreten Wasserstoff, Alkyl, Alkenyl, Alkinyl, Cycloalkyl, Cycloalkenyl, Aralkyl, Heterocycloalkyl, Heterocycloalkenyl, Heteroaryl oder Heteroaralkyl ist;
jedes von R¹² und R¹³ unabhängig für jedes Auftreten Wasserstoff, Alkyl, Alkenyl, Alkinyl, Cycloalkyl, Cycloalkenyl, Aralkyl, Heterocycloalkyl, Heterocycloalkenyl, Heteroaryl oder Heteroaralkyl ist; oder R¹² oder R¹³ zusammengenommen einen 3-8-gliedrigen heterocyclischen Ring bilden;
R²⁴ ist

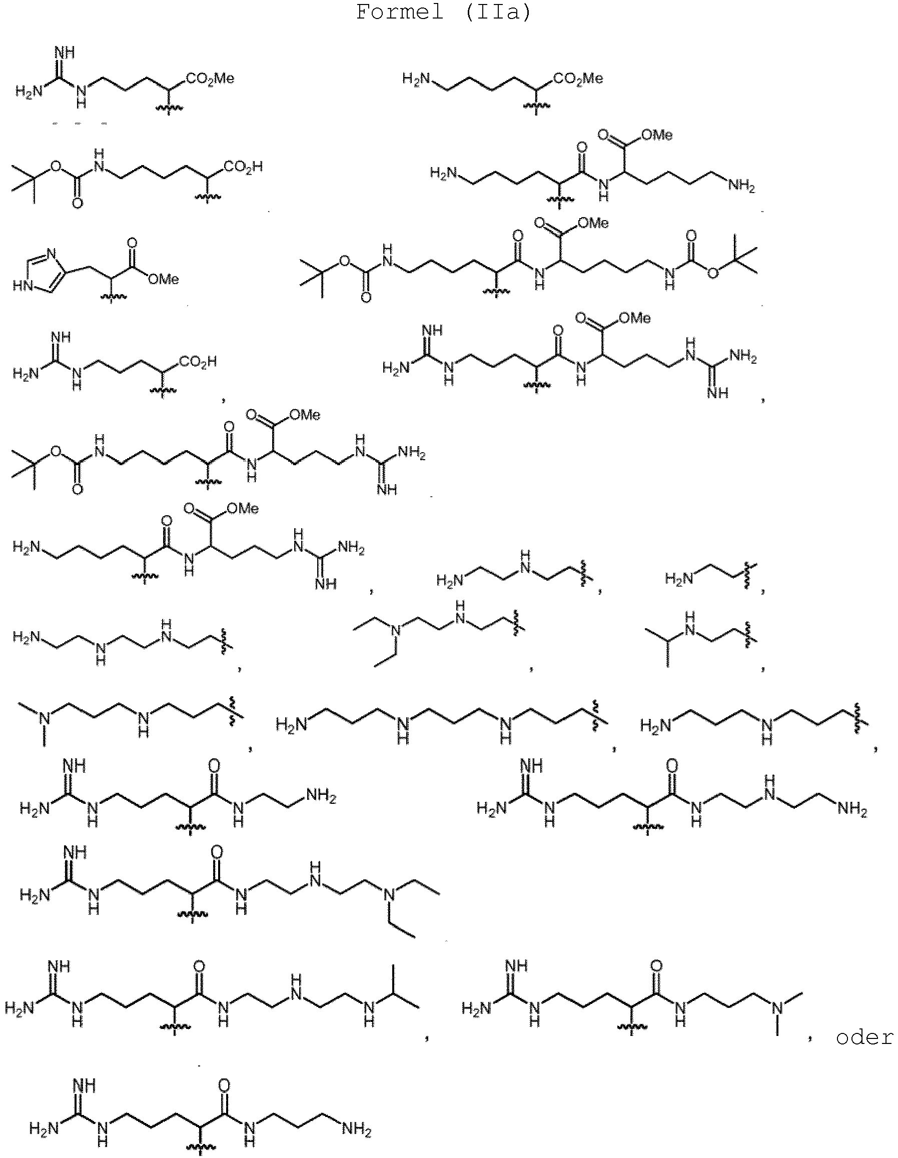

2. Verbindung nach Anspruch 1, wobei R¹⁰ Alkyl ist.

3. Verbindung nach einem der Ansprüche 1-2, wobei die Verbindung ausgewählt ist aus der Gruppe bestehend aus: 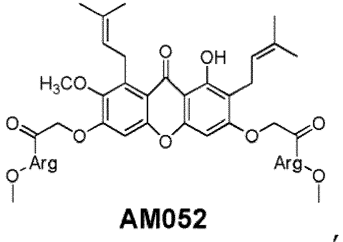 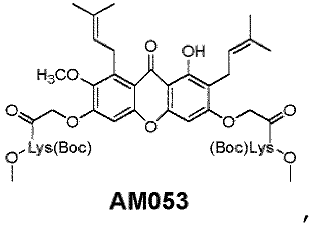 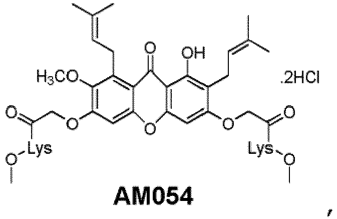 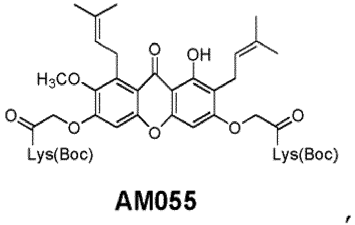 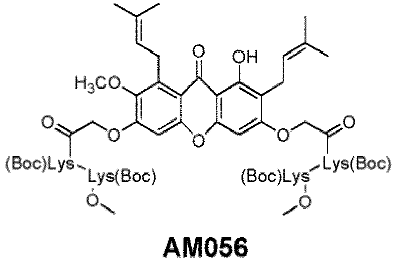 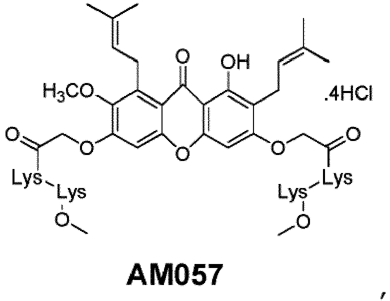 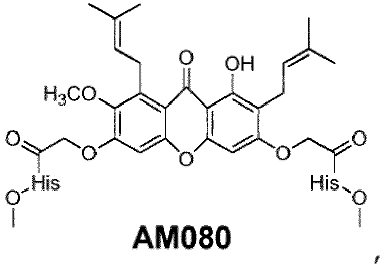 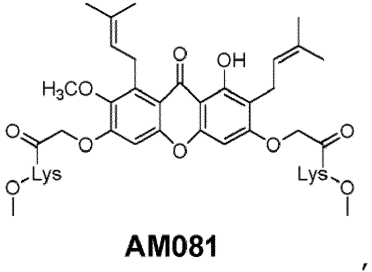 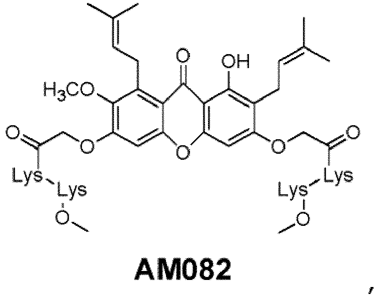 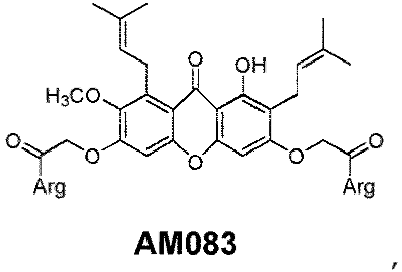 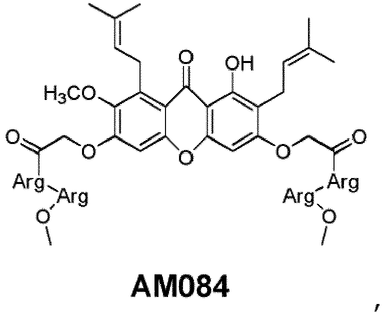 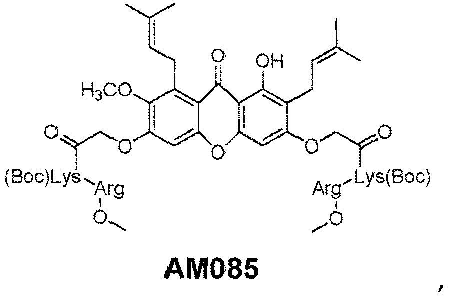 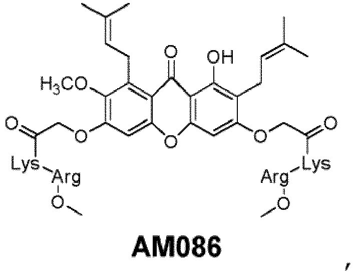 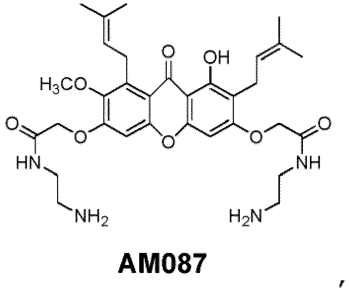 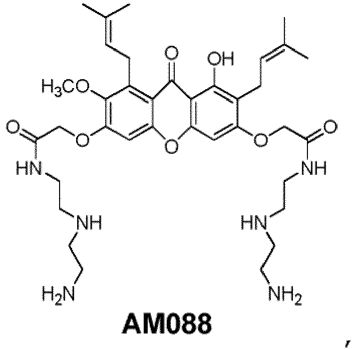 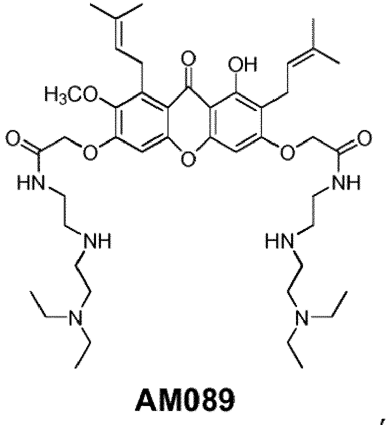 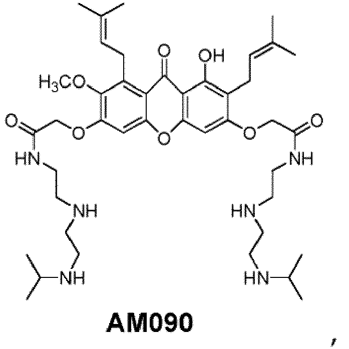 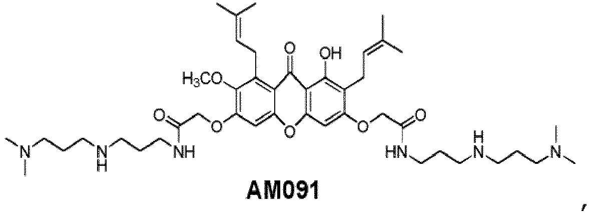 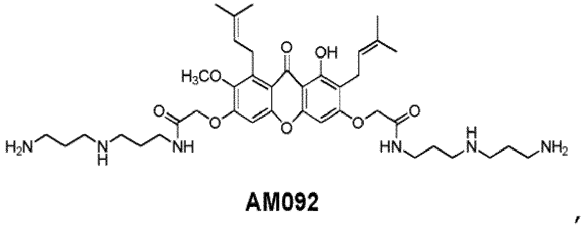 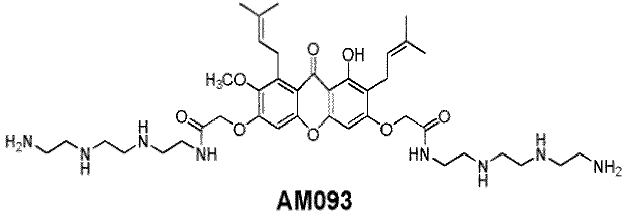 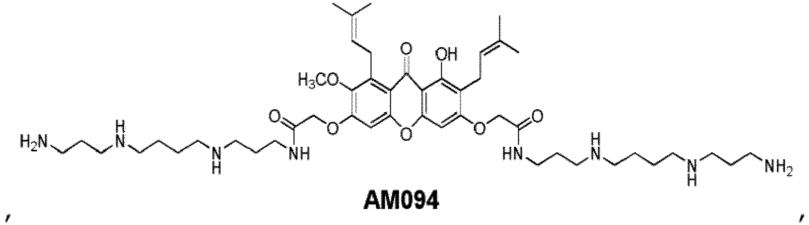 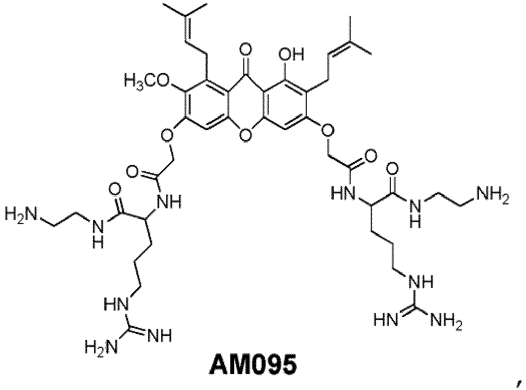 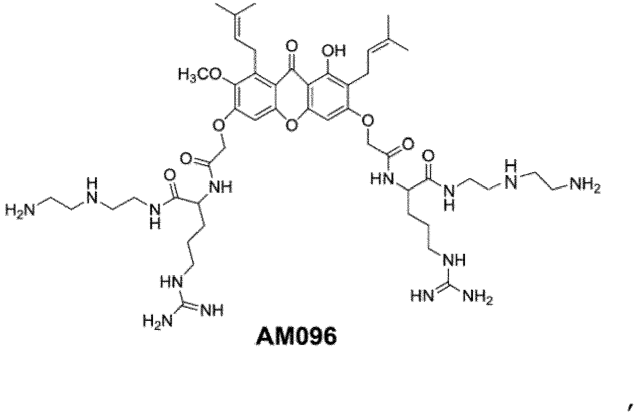 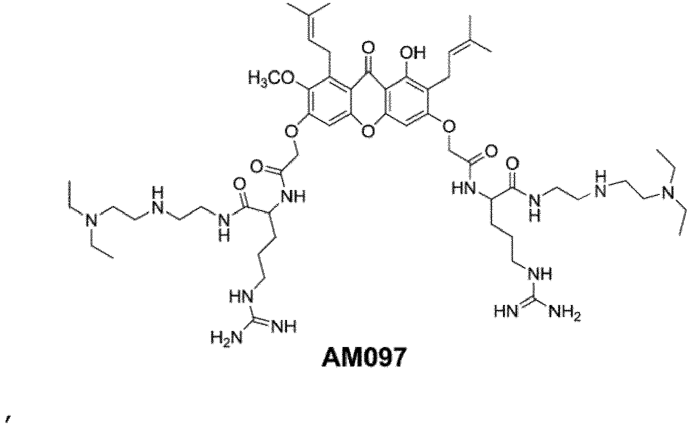 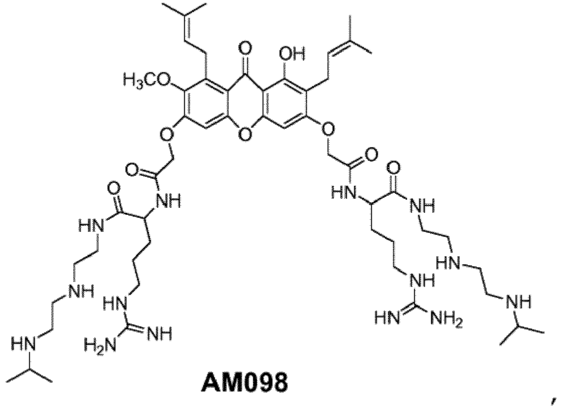 und

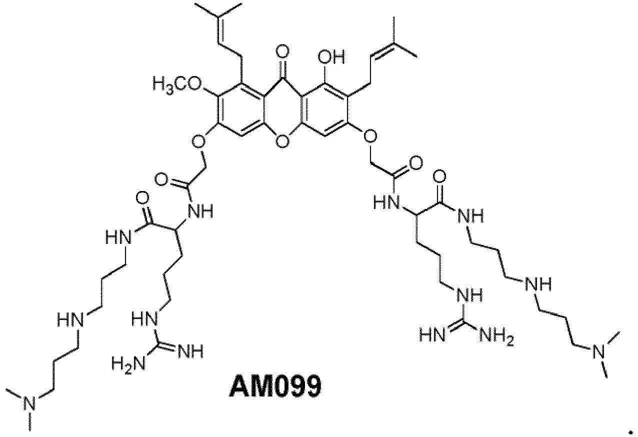

4. Pharmazeutische Zusammensetzung, umfassend eine Verbindung nach einem der Ansprüche 1 bis 3 zusammen mit einem pharmazeutisch verträglichen Träger.

5. Verbindung nach einem der Ansprüche 1 bis 3 zur Verwendung als ein Medikament.

6. Verbindung zur Verwendung nach Anspruch 5, wobei die Verwendung als ein Medikament zur Behandlung einer mikrobiellen Infektion erfolgt.

7. Verbindung zur Verwendung nach Anspruch 6, wobei die mikrobielle Infektion eine bakterielle Infektion ist, die durch Gram-positive oder Gram-negative Bakterien, Mykobakterien oder einen Pilz verursacht wird, wobei die Gram-positiven Bakterien ausgewählt sind aus der Gruppe bestehend aus *Streptococcus spp., Staphylococcus spp., Bacillus spp., Carynebacterium spp., Clostridium spp., Listeria spp.* und *Enterococcus spp.,* wobei vorzugsweise die Gram-positiven Bakterien *Staphylococcus aureus* sind und wobei vorzugsweise der *Staphylococcus aureus* Methicillin-resistenter *Staphylococcus aureus* ist.

8. Verbindung zur Verwendung nach einem der Ansprüche 6 bis 7, wobei das Medikament mit einem oder mehreren weiteren Wirkstoffen verabreicht werden soll, optional wobei der eine oder die mehreren weiteren Wirkstoffe gleichzeitig, getrennt oder aufeinanderfolgend mit dem Medikament verabreicht werden sollen.

9. Pharmazeutische Dosierungsform, umfassend eine Verbindung nach einem der Ansprüche 1 bis 3 oder eine Zusammensetzung nach Anspruch 4.

10. Kit, umfassend eine Verbindung nach einem der Ansprüche 1 bis 3, eine Zusammensetzung nach Anspruch 4 oder eine pharmazeutische Dosierungsform nach Anspruch 9 und Gebrauchsanweisungen.

## Revendications

1. Composé de formule II ou sel de celui-ci : 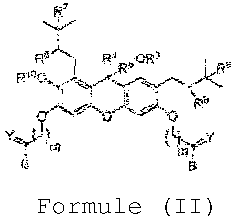 dans laquelle
m est 1 ;
Y est O ;
B est NHR²⁴ ;
chacun parmi R³ et R¹⁰, indépendamment pour chaque occurrence, est un atome d'hydrogène, un groupe alkyle, alcényle, alcynyle, cycloalkyle, cycloalcényle, aralkyle, hétérocycloalkyle, hétérocycloalcényle, hétéroaryle, hétéroaralkyle, -O(C=O)R¹¹,-O(C=O)OR¹¹, -O(C=O)NR¹²R¹³, -OSO₃R¹¹ ou -OPO₂OR¹¹ ;
R⁴ et R⁵ pris conjointement avec l'atome de carbone auquel ils sont liés forment (C=O);
R⁶ et R⁷ pris conjointement forment une liaison ; R⁸ et R⁹ pris conjointement forment une liaison ;
R¹¹ est, indépendamment pour chaque occurrence, un atome d'hydrogène, un groupe alkyle, alcényle, alcynyle, cycloalkyle, cycloalcényle, aralkyle, hétérocycloalkyle, hétérocycloalcényle, hétéroaryle ou hétéroaralkyle ;
chacun parmi R¹² et R¹³, indépendamment pour chaque occurrence, est un atome d'hydrogène, un groupe alkyle, alcényle, alcynyle, cycloalkyle, cycloalcényle, aralkyle, hétérocycloalkyle, hétérocycloalcényle, hétéroaryle ou hétéroaralkyle ; ou R¹² ou R¹³ pris conjointement forment un noyau hétérocyclique de 3 à 8 chaînons ;
R²⁴ est

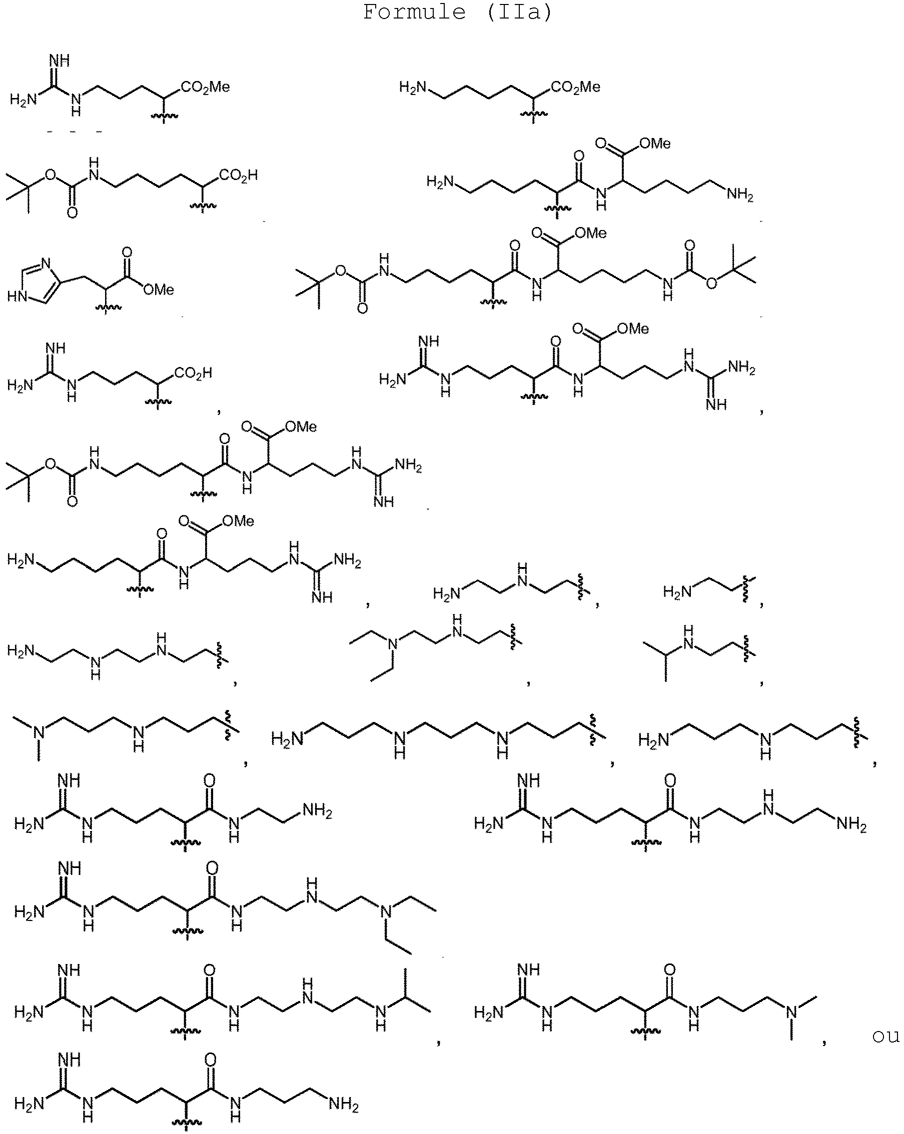

2. Composé selon la revendication 1, dans lequel R¹⁰ est un groupe alkyle.

3. Composé selon l'une quelconque des revendications 1 et 2, dans lequel le composé est choisi dans le groupe constitué de : 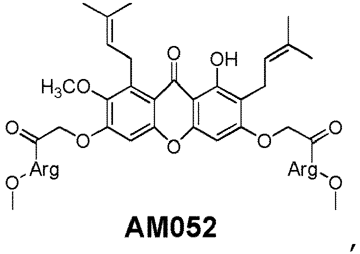 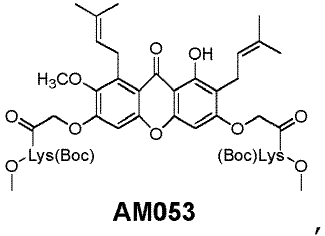 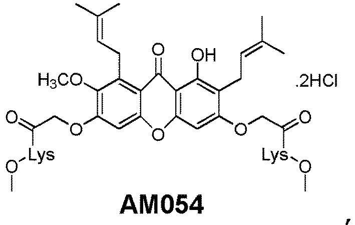 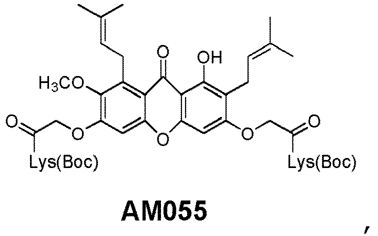 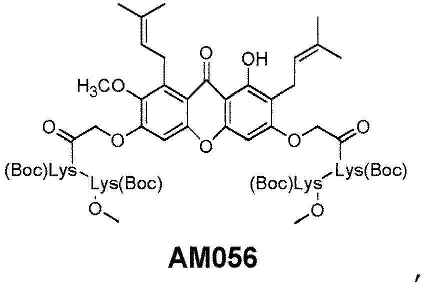 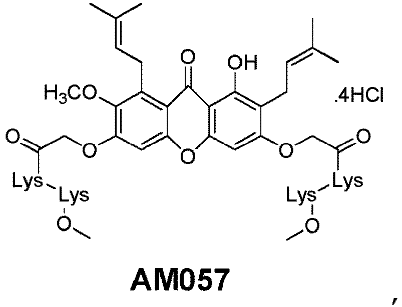 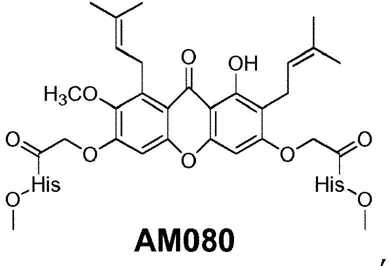 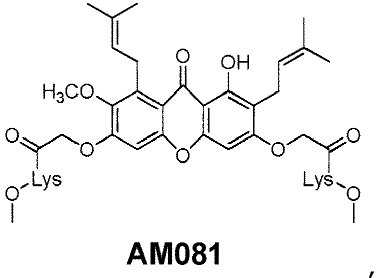 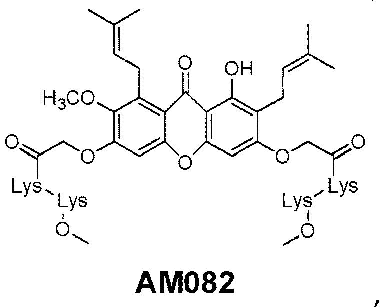 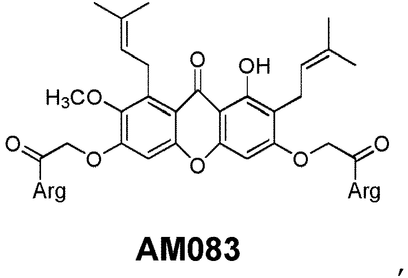 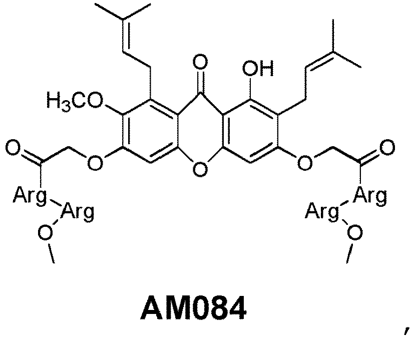 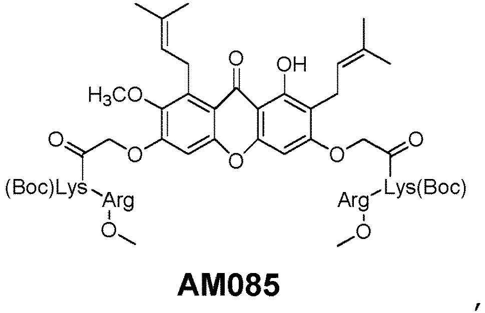 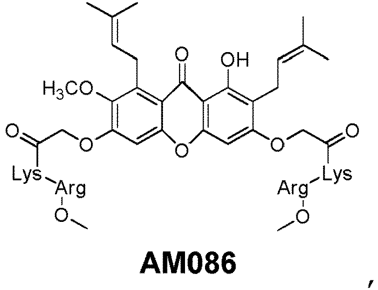 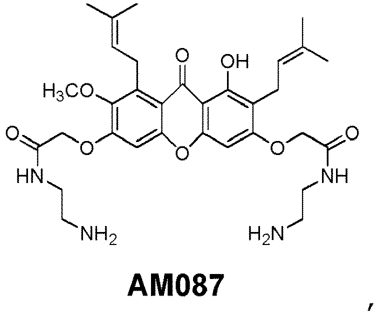 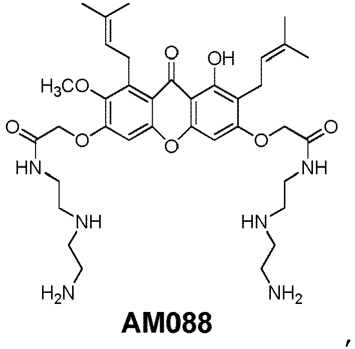 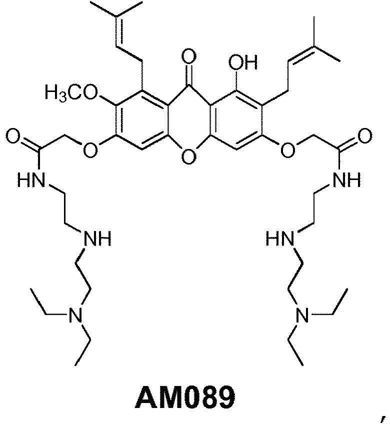 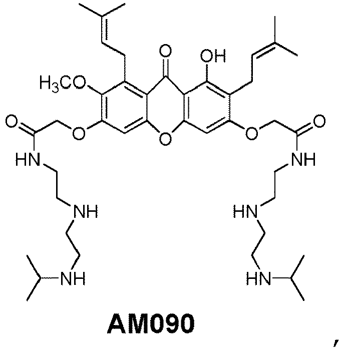 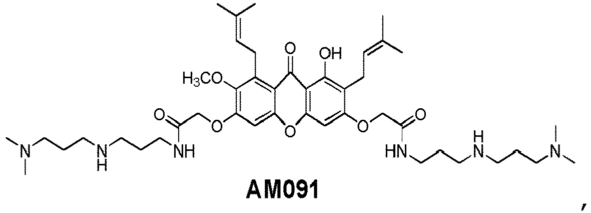 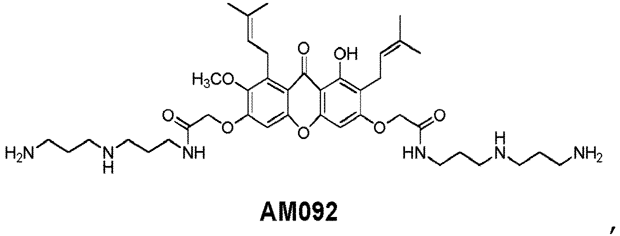 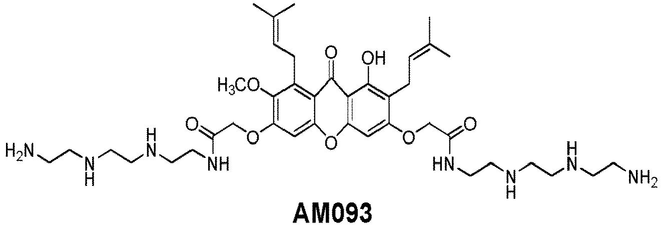 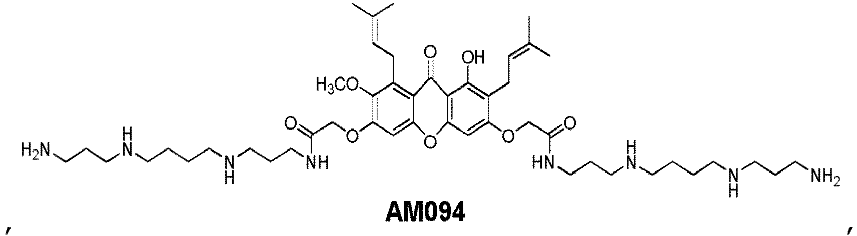 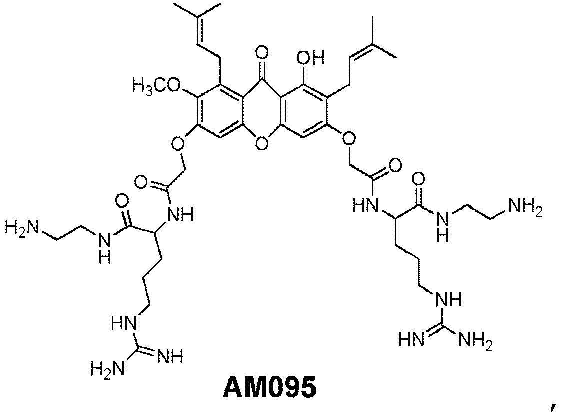 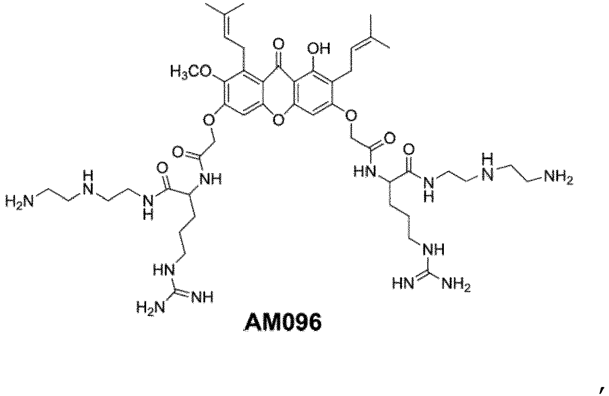 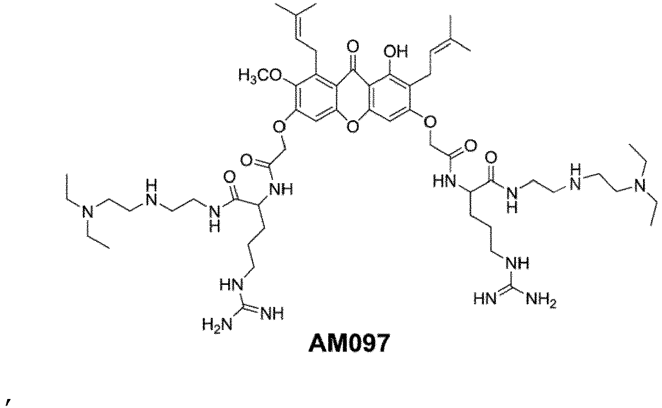 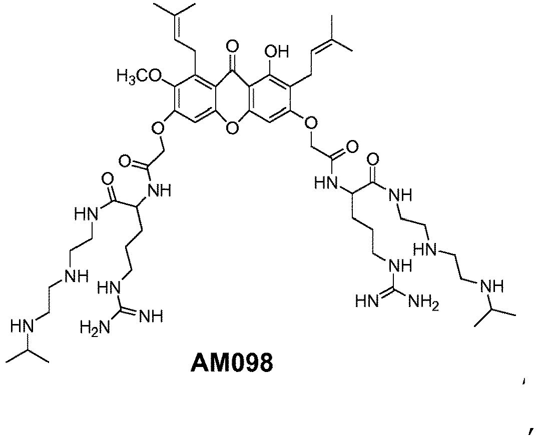 et

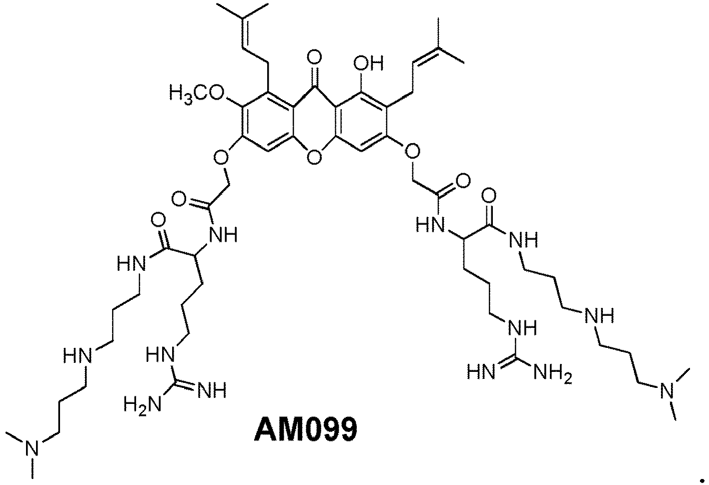

4. Composition pharmaceutique comprenant un composé selon l'une quelconque des revendications 1 à 3, conjointement avec un support pharmaceutiquement acceptable.

5. Composé selon l'une quelconque des revendications 1 à 3, destiné à être utilisé en tant que médicament.

6. Composé destiné à être utilisé selon la revendication 5, dans lequel ladite utilisation convient en tant que médicament pour le traitement d'une infection microbienne.

7. Composé destiné à être utilisé selon la revendication 6, dans lequel l'infection microbienne est une infection bactérienne provoquée par une bactérie Gram positive ou Gram négative, une mycobactérie ou un champignon, dans lequel la bactérie Gram positive est choisie dans le groupe constitué de *Streptococcus spp., Staphylococcus spp., Bacillus spp.*, *Carynebacterium spp., Clostridium spp., Listeria spp.,* et *Enterococcus spp.,* de préférence dans lequel ladite bactérie Gram positive est *Staphylococcus aureus* et de préférence dans lequel ledit *Staphylococcus aureus est* un *Staphylococcus aureus* résistant à la méthicilline.

8. Composé destiné à être utilisé selon l'une quelconque des revendications 6 à 7, dans lequel ledit médicament doit être administré avec un ou plusieurs autres agents actifs, éventuellement dans lequel les un ou plusieurs autres agents actifs doivent être administrés simultanément, séparément ou séquentiellement avec ledit médicament.

9. Forme posologique pharmaceutique comprenant un composé selon l'une quelconque des revendications 1 à 3 ou une composition selon la revendication 4.

10. Kit comprenant un composé selon l'une quelconque des revendications 1 à 3, une composition selon la revendication 4 ou une forme posologique pharmaceutique selon la revendication 9 et un mode d'emploi.
